(19) Europäisches Patentamt
European Patent Office
Office européen des brevets

(11) EP 1 020 179 B1

(12) **EUROPEAN PATENT SPECIFICATION**

(45) Date of publication and mention of the grant of the patent:
**09.12.2009 Bulletin 2009/50**

(21) Application number: **98935447.7**

(22) Date of filing: **03.07.1998**

(51) Int Cl.:
***A61K 9/00*** *(2006.01)* ***C07D 233/24*** *(2006.01)*
***C07D 233/54*** *(2006.01)* ***C07D 209/16*** *(2006.01)*
***C07D 211/88*** *(2006.01)* ***C07D 401/06*** *(2006.01)*
***C07D 209/26*** *(2006.01)* ***C07D 209/18*** *(2006.01)*
***C07D 403/06*** *(2006.01)* ***A61P 29/00*** *(2006.01)*
***A61P 31/00*** *(2006.01)* ***A61K 38/05*** *(2006.01)*

(86) International application number:
**PCT/RU1998/000215**

(87) International publication number:
**WO 1999/001103 (14.01.1999 Gazette 1999/02)**

(54) **PEPTIDE DERIVATIVES OR PHARMACEUTICALLY ACCEPTABLE SALTS THEREOF, METHOD FOR PRODUCING THE SAME, USE OF SAID DERIVATIVES AND PHARMACEUTICAL COMPOSITION**

PEPTIDDERIVATE ODER DEREN PHARMAZEUTISCH ANNEHMBARE SALZE, VERFAHREN ZU DEREN HERSTELLUNG, VERWENDUNG DIESER DERIVATE UND ARZNEIMITTEL

DERIVÉS DE PEPTIDES, SELS DE CES DERIVÉS ACCEPTABLES SUR LE PLAN PHARMACEUTIQUE, PROCÉDÉ DE PRODUCTION DE CES DERIVÉS, UTILISATION DE CES DERNIERS ET COMPOSITION PHARMACEUTIQUE

(84) Designated Contracting States:
**AT BE CH CY DE DK ES FI FR GB GR IE IT LI LU MC NL PT SE**

(30) Priority: **04.07.1997 RU 97111091**

(43) Date of publication of application:
**19.07.2000 Bulletin 2000/29**

(60) Divisional application:
**08006502.2**

(73) Proprietors:
- **Otkrytoe Aktsionernoe Obschestvo "Otechestvennye Lekarstva" Moscow 129090 (RU)**
- **Nebolsin, Vladimir Evgenievich Moscow, 113648 (RU)**

(72) Inventors:
- **NEBOLSIN, Vladimir Evgenievich Moscow, 113648 (RU)**
- **ZHELTUKHINA, Galina Alexandrovna Moscow, 129344 (RU)**
- **EVSTIGNEEVA, Rima Porfirievna Moscow, 119211 (RU)**

(74) Representative: **Keen, Celia Mary J.A. Kemp & Co. 14 South Square Gray's Inn London WC1R 5JJ (GB)**

(56) References cited:
**EP-A- 0 412 595** **EP-A1- 0 584 694**
**WO-A1-94/19325** **WO-A1-95/10294**
**WO-A1-96/36348** **CH-A5- 631 446**
**GB-A- 2 090 831** **RU-C1- 2 080 120**
**RU-C1- 2 091 389** **SU-A1- 1 807 353**
**US-A- 4 297 346**

- **CHEMICAL ABSTRACTS, vol. 70, no. 9, 3 March 1969 (1969-03-03) Columbus, Ohio, US; abstract no. 37347q, SCIOTINO,T. ET AL.: "Antiviral activity. IX. Higher acyl derivatives of sympathomimetic amines of biological significance." XP002225153 -& DATABASE CAPLUS [Online] CHEMICAL ABSTRACTS SERVICE, COLUMBUS, OHIO, US; CA 70:37347, XP002225158 & BOLL. CHIM. FARM., vol. 107, no. 8, - 1968 pages 506-511,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 020 179 B1

- BENG T. HO ET AL.: "Hydroxyindole-o-methyltransferase IV: Inhibitory activities of some N-acryltryptamines and N-crotonyltryptamines" JOURNAL OF PHARMACEUTICAL SCIENCES., vol. 59, no. 4, - April 1970 (1970-04) pages 573-574, XP002225150 AMERICAN PHARMACEUTICAL ASSOCIATION. WASHINGTON., US ISSN: 0022-3549
- CHEMICAL ABSTRACTS, vol. 64, no. 3, 31 January 1966 (1966-01-31) Columbus, Ohio, US; abstract no. 3473 c, J. GOOTJES ET AL.: "Derivatives of benzo- and indoloquinolizines. I. Synthesis of 2-substituted hexahydro-2H-benzo (a)quinolizines and octahydroindolo(2,3-a) quinolizines from substituted glutaric acids." XP002225151 -& DATABASE CAPLUS [Online] CHEMICAL ABSTRACTS SERVICE, COLUMBUS, OHIO, US; CA 64:3473c, XP002225156 & REC. TRAV. CHIM., vol. 84, no. 9-10, - 1965 pages 1183-1199,
- CHEMICAL ABSTRACTS, vol. 101, no. 1, 2 July 1984 (1984-07-02) Columbus, Ohio, US; abstract no. 5266e, MITA, HARUHISA ET AL.: "An attempt to produce an antibody to histamine and histamine derivatives." XP002225152 -& DATABASE CAPLUS [Online] CHEMICAL ABSTRACTS SERVICE, COLUMBUS, OHIO, US; CA 101:5266e, XP002225157 & AGENTS ACTIONS, vol. 14, no. 5-6, - 1984 pages 574-579,
- CHEMICAL ABSTRACTS, vol. 106, no. 17, 27 April 1987 (1987-04-27) Columbus, Ohio, US; abstract no. 138681, BLASKO, GABOR ET AL.: "Indole alkaloid beta-carboline-1-propionic acid." XP002225154 -& DATABASE CAPLUS [Online] CHEMICAL ABSTRACTS SERVICE, COLUMBUS, OHIO, US; CA 106:138681, XP002225159 & PLANTA MED., vol. 1, - 1986 pages 41-43,
- CHEMICAL ABSTRACTS, vol. 95, no. 23, 7 December 1981 (1981-12-07) Columbus, Ohio, US; abstract no. 200006, DELAAGE, M. A. ET AL.: "Radioimmunoassays for serotonin and 5-hydroxyindole acetic acid." XP002225155 & J. PHYSIOL. (PARIS), vol. 77, no. 2-3, - 1981 pages 339-347, -& DATABASE CAPLUS [Online] CHEMICAL ABSTRACTS SERVICE, COLUMBUS, OHIO, US; CA 95:200006, XP002225160

## Description

The present invention relates to the field of bioorganic chemistry and, in particular, it concerns novel dipeptide and pseudopeptide compounds comprising a heterocyclic, for instance, imidazole or indole group, a method to produce these and the known compounds of similar structure, as well as their use in medicine as potential therapeutic agents.

Prior art

Substances of peptide nature are known to possess a high biological activity. Different action aspects of these compounds are disclosed in the literature: on the immune system, on histamine release from rat peritoneal mast cells and human basophils [Marone G., Columbo N., Soppeisa L. et al., J. Immunol. (1984) Vol. 133, No 3, pp: 1542-1546; Wise J., Wojtecka-Lukasik E., Maslinski S., Agents Actions (1986) Vol. 18, No 1-2, pp. 262-265], on production and catabolism of prostaglandin $E_2$ [Duchateau J., Bolla K., Med. Oncol, and Tumor Pharmacoter. (1989) Vol. 6, No 6, pp. 19-23], and on immediate reaction development [8. Muller E., Sonnenschein B., Naturamed. (1989), Vol. 4, No 4, pp. 34-38].

Hypoxia is a common link in the pathogenesis of a broad circle of pathological conditions (stress, physical load, radiation, allergic diseases, atherosclerosis and accompanying diseases, hepatic lesions of different etiology, diabetes mellitus and others). It is known that in hypoxia, lipid peroxidation (LP) rises and cytochrome P-450 content and the activity of P-450-dependent enzymes drop [Proulx M., Dusouich P., J. Pharm. Pharmacol. (1995), Vol. 47, Iss 5, pp. 392-397; Barakat M., du Souich P., J. Pharm. Pharmacol. (1996), Vol. 48, pp. 906-910]. At the same time, the activity of superoxide-desmutase rises and hepatic glutathione content and the activity of glutathione-peroxidase drop. These changes result in an increased amount of the reactive oxygen radicals which can cause damage of the membrane-bound enzymes, in particular, the P-450 cytochrome system enzymes [Proulx M., Dusouich P., J. Pharm. Pharmacol. (1995), Vol. 47, Iss 5, pp. 392-397]. LP is an important physiological process continuously occuring in the cell membrane and normally playing an important role in the life activity of cells, e.g. during the synthesis of prostaglandins as well as in phagocytosis [Sokolov E.I. Diabetes mellitus and atherosclerosis. Moscow, Nauka publishers (1996), 405 pp.]. However, as background to tissue hypoxia development, this process becomes poorly controllable and a great amount of free-radical compounds are formed, which have no time for neutralization. Acidosis development is accompanied by the induction of cytochrome P-450 2E1 which exhibits a pronounced activity when lipid peroxides are formed that promotes the rise in LP to a greater extent still [Bestervelt L.L., Vaz A.D.N., Coon M.J., Proc. Natl. Acad. Sci. U.S.A. (1995), Vol. 92, Iss. 9, pp. 3764-3768]. Normalization of LP can serve as an important criterion in the therapy of said diseases.

It is known that change in the hepatic cytochrome P-450 system condition is closely related to its antioxidant function. Study of this problem has an applied character since in many diseases (atherosclerosis, liver cirrhosis, hepatitis, alcohol abuse and others) disbalance of lipid metabolism occurs with a subsequent rise in lipid peroxidation (LP).

The development of experimental allergic reactions has been shown to occur against the background of decrease in terminal oxigenase content and destabilized condition of the hepatic monooxigenase system components assessed by the renewal ability of $B_5$ and P-450 cytochromes [Krzhechkovskaya V.V., Meltsev G.Yu., Marokko I.N., The 4th All-Union Symposium on Medical Enzymology (1983), p. 137]. Change in the content and ratio of hepatic P450B and P450L cytochrome groups, correlates with change in Hexenal sleep duration, in hormonal status and in the severety of anaphylactic shock in guinea-pigs [Marokko I.N., Krzhechkovskaya V.V., Malikova N.A., Izotov M.V., Benediktova S.A., Spiridonova S.M., Bulletin of Experimental Biology and Medicine (1991) No 8, pp. 200-202]. Activation of the P-450 cytochrome system enzymes with Phenobarbital type inductors, results in the severety alleviation of experimental allergic reaction signs [Marokko I.N., Krzhechkovskaya V.V., Malikova N.A., Izotov M.V., Benediktova S.A., Spiridonova S.M., Bulletin of Experimental Biology and Medicine (1991) No 8, pp. 200-202]. At the same time, when sensitized animals are administered Methyrapone, an inhibitor of the P-450 cytochrome system and cortisol synthesis, an increase in bronchospasm is observed [Fornhem C., Kumlin M., Lundberg J.M., Alving K., Eur. Resp. J. (1995), Vol. 8, Iss. 7, pp. 1100-1109; Fornhem C., Lundberg J.M., Alving K., Eur. Resp. J. (1995), Vol. 8, Iss. 6, pp. 928-937]. Sensitization state in humans and experimental animals is accompanied by a drop in the blood and urine levels of glucocorticoids. Normalization of adrenocortical hormone levels results in the improvement of clinical disease picture in humans and in decreased severety of allergic reaction signs in animals [Marokko I.N., Krzhechkovskaya V.V., Malikova N.A., Proceedings of the All-Union Conference "P-450 Cytochrome and Modification of Macromolecules", Yalta (1989) p. 339; Macharadze D.Sh, Marokko I.N., Balabolkin I.I., Yukhtina N.V., Malikova N.A., Pediatry (1994) No 3, pp. 9-12], and a decrease in their levels results in the aggravation of allergic reaction signs [Fornhem C., Kumlin M., Lundberg J.M., Alving K., Eur. Resp. J. (1995) Vol. 8, Iss. 7, pp. 1100-1109; Fomhem C., Lundberg J.M., Alving K., Eur. Resp. J. (1995) Vol. 8, Iss. 6, pp. 928-937].

Arachidonic acid (AA) metabolites being formed in the P-450 cytochrome system, possess a number of important biological effects, in particular, vasodilating and bronchiolytic effects [Knickle L.C., Bend J.R, J. Moll. Pharmacol 91994),

Vol. 45, Iss. 6, pp. 1273-1280; Quiroga J., Prieto J., Phamacol. Therap. (1993), Vol. 58,Iss, 1, pp. 67-91; Ma Y.H., Gebremedich D., Schwartzman M.L., et al., Circulation Research (1993), Vol. 72, Iss. 1, pp. 126-136], cytoprotective action [Quiroga J., Prieto J., Pharmacol. Therap. (1993), Vol. 58, Iss. 1, pp. 67-91], they potentiate prostaglandin $E_2$ synthesis [Caroll M.A., Balazy M., Margiotta P., Falck J.R., Mcgiff J.C., J. Biol. Chem. (1993), Vol. 268, Iss. 17, pp. 12260-12266; Sakairi Y., Jacobson H.R., Noland T.D., Capdevilla J.H., Falck J.R., Breyer M.D., Amer. J. Physiol-Renal. F1. Elect. (1995), Vol. 37, Iss. 5, pp. F931-F939] and other effects, the vasodilating effect of AA being potentiated by administration of phenobarbital, which is a known hepatic monoamine oxygenase system inductor [Oyekan A.O., Eur. J. Pharmacol. (1995), Vol. 277, Iss. 2-3, pp. 123-132]. At the same time, the P-450-dependent AA metabolite 5,6-epoxyeucozatriene acid (5,6-EET) potentiates $PGE_2$ synthesis and secretion [Sakairi Y., Jacobson H.R., Noland T.D., Capdevila J.H.; Falck J.R., Breyer M.D., Amer. J. Physiol-Renal. F1. Elect. (1995), Vol. 37, Iss. 5. pp. F931-F939]. On one hand, $PGE_2$ inhibits the anaphylactogenic surge of histamine and the other allergy and inflammation mediators from the mast cells. On the other hand, histamine potentiates $PGE_2$ synthesis that is considered as one of the feedback mechanisms in allergic and inflammatory reactions.

Of special importance in the development of allergic diseases is a change in the pathochemical stage course of allergic reactions, which to a great extent is determined by the condition of the first order target cells of allergy (basophils and mast cells), the important feature of which is their ability to accumulate and to synthesize biologically active compounds, in particular, histamine. In IgE and/or IgG-mediated response to antigen, just these cells participate in the secretion of active substances and determine the pathochemical phase course and manifestation degree of allergy clinical picture [Parker Ch.V. Mediators: Release and Functions. In: Immunology ed. by U. Pole, Moscow, Mir publishers (1989) Vol. 3, pp. 170-247; Chakravarty N.K. In: The mast cell: Its role in health and disease. Ed. J Pepys (1979) pp. 38-46]. The importance of examining histamine secretion by mast cells is also determined by the fact that in mast cells transglutaminase . (EC 2.3.2.13) is present, i.e. the enzyme synthesizing protein-bound gamma-glutamyl histamine, which is a close analogue of the suggested compounds. Stimulation of mast cells was shown to rase the activity of transglutaminase and the content of protein-bound gamma-glutamyl histamine in mast cells [Fesus L., Szucs E., Barrett K. et al., J. Biol. C. Chem. (1995),No 25, pp. 13771-13778].

It has been demonstrated that in hepatic diseases, e.g. hepatitis and cirrhosis, the hepatic P-450 cytochrome system functional activity decreases and LP rises [Imaoka S., Sugiyama T., Taniguchi N., Funae Y., Carcinogenesis (1993), Vol. 14, Iss. 1, pp. 117-121; Debinski H.S., Lee C.S., Danks J.A., Mackenzie P.I., Desmond P.V., Gastroenterology (1995), Vol. 108, Iss. 5, pp. 1464-1469]. Changes in hepatic structure and function which are characteristic of a given pathology, are noted in experimental animals administered carbon quadrichloride ($CCl_4$). The effect of this substance is connected with the damage of the cellular membrane structures caused by elevated LP that is one of the hepatic P450 cytochrome system enzyme inactivation [Kapil A., Koul I.B., Suri O.P. Phytother. Res. (1995), Vol. 9, Iss. 3, pp. 189-1993].

Change in lipid metabolism in atherosclerosis is interconnected with change in the P450 cytochrome system condition [Wolfgang GHI, Robertson DG, Welty DF, Metz AL. Fund. Appl. Toxicol. (1995), Vol. 26, Iss. 2, pp. 272-281; Remaley A.T., Schumacher U.K., Amouzadeh H.R., Brever H.B., Hoeg J.M. J. Lipid Res. (1995), Vol. 36, Iss. 2, pp. 308-314; Stegeman J.J., Hahn M.E., Weisbrod R., Woodin B.R., Joy J.S., Najibi S., Cohen R.A. Mol. Pharmacol. Vol. 47, Iss. 2, pp. 296-306]. It is known that ischemic heart disease, which occupies first place among the planet adult population mortality rate, is the most often occuring atherosclerosis manifestation. One of the leading disorders in this disease is lipid metabolism disorder manifested by elevated levels of plasma, low density lipoprotein (LDL) and very low density lipoprotein (VLDL) cholesterol which are called "atherogenic" lipoproteins, with simultaneous decrease in "antiatherogenic" high density lipoproteins (HDL).

Change in plasma lipid levels and ratio has been shown to reflect changes in the membrane structure of parenchymatous organs. The membrane composition of cells, e.g. microsomal cells, is directly dependent on the ratio in experimental animals [Wade A., Harred W. Feder. Proct. (1976), Vol: 55, pp. 2475-2479]. Cholesterol administration to animals causes its accumulation in cellular memranes decreasing their fluidity which, in turn, results in a change in the functional state of enzymes [Buters J.T.M., Zysset T., Reichen J. Biochem. Pharmacol. (1993), Vol. 46, Iss. 6, pp. 983-991; Reichen J., Buters J.T.M., Sojcic Z., Roos F.J., Experentia (1992), Vol. 48, Iss. 5, pp. 482-486; Tatony Ya.N. Obesety. Pathophysiology, diagnostics, therapy., Warsaw, the Polish Medical publishers (1981) p. 364].

In addition to carbohydrate metabolism disorders in insulin-dependent diabetes mellitus, changes in ketone and fatty acid metabolism have been demonstrated, which result in disbalance of the hepatic microsomal monooxygenase system and further promotes disorder of the body hormonal status [Shimojo N., Ishizaki T., Imaoka S., Funae Y., Fujii S., Okuda K., Biochem. Pharmacol. (1993), Vol. 46, Iss. 4, pp. 621-627]. In this case, a change in lipid metabolism is observed, which is manifested by cholesterol level elevation in the LDL and VLDL fractions and cholesterol decrease in the HDL fraction, as well as by a significant rise in lipid peroxidation [Sokolov E.I. Diabetes mellitus and atherosclerosis, Moscow, Nauka publishers (1996), 450 pp.]. Changes in lipid metabolism in diabetes mellitus practically completely coincide with changes in atherosclerosis. Blood glucose level is one of the most important parameters of the pathological process stabilization in diabetes mellitus. Practically all antidiabetic drugs are known to lower blood glucose level not

only in diabetes patients but in healthy humans as well.

Phagocytosis is an important link in maintaining the body's internal medium. Phagocyting cells are represented in a large amount in the body. To them in particular belong peripheral blood neutrophils and macrophags (MP). Activation of MP is one of the homeostasis adaptive mechanisms promoting elimination of pathogenes, and antitumor resistance [Immunology. Edited by Pole U., Moscow, Mir publishers (1989), Vol. 3, pp. 202-228; Paltsev M.A., Ivanov A.A. Intercellular ineractions. Moscow, Meditsina publishes (1995), 224 pp.]. These cells are known to carry glucocorticoid receptors on the external membrane [Mayansky A.N., Pikuza O.I. Clinical aspects of phagocytosis. Kazan, MAGARIF publishers (1993), 192 pp.]. Elevation of glucocorticoid hormone levels, is one of neutrophil and MP activation mechanisms.

The surging of the adrenocortical hormones into the blood serves as a signal for neutrophil mobilization from the marrow, which is the mechanism providing concordance between the marrow and stress-limiting reactions [Mayansky A.N., Pikuza O.I. Clinical aspects of phagocytosis. Kazan, MAGARIF publishers (1993), 192 pp.].

Active forms of neutrophils and basophils have been shown to synthesize and release into extracellular medium biologically active substances such as hydrogen peroxide, peroxidase and others, which inactivate leukotrienes that cause bronchospasm in allergic diseases and play an important role in defence against infection and maintaining body homeostasis [Immunology: Edited by Pole U., Moscow, Mir publishers (1989), Vol. 3, pp. 202-228; Henderson W.R, Jorg A., Klebanoff S.J., J. Immunol. (1982), Viol. 128, No 6, pp. 2609-2613]. Moreover, tranformation of macrophags in consumption of increased amounts of cholesterol by animals, which results in functional activity disorder of macrophags, in particular Kupfer cells [Remaley A.T., Schumacher U.K., Amouzadeh H.R., Brever H.B., Hoeg J.M., J. Lipid Res. (1995), Vol. 36, Iss. 2, pp. 308-314], is considered to be one of the key links in atherosclerotic plaque formation [Sokolov E.I. Diabetes mellitus and atherosclerosis, Moscow, Nauka publishers (1996), 405 pp.].

It is known that the formation and development process of metastases is to a great extent determined by intravascular blood coagulation state, and as a result of tumor disease development and the effect of a number of accompanying factors, the blood coagulation system is activated. It has been shown that administration of anticoagulants can restore the disbalance of the blood coagulation and anticoagulation systems [Burov Yu.V., Syrkin A.B., Kinzirsky A.S., Koroleva A.M., Chemical-pharmaceutical production. Review information (1992), issue -11,42 pp.].

Blood neutrophil activity drops sharply in severe infections, which can cause generalization of infection, and a rise in the number of activated phagocytes including perypheral blood neutrophils and peritoneal macrophages, and increases the probability of an uncomplicated course of bacterial infection [Mayansky A.N., Pikuza O.I. Clinical aspects of phagocytosis. Kazan, MAGARIF publishers (1993), 192 pp.]. Antimicrobic, antiviral and antitumor defence of the body is directly related to neutrophil and macrophage activation effects.

β-alanylhistamine and γ-aminobutyrylhistamine - which correspond to general formula (I)- known compounds producable using the classical peptide chemistry methods and possess in vitro antioxidant activity as well as wound healing and anticataract activity [Evstigneeva R.P., Zheltukhina G.A., Ogrel' S.A., Nebol'sin V.E. Synthesis of pseudopeptides based on biogenic amines. Reports of the Russian Sciences Academy (1995), Vol. 345, No 4, pp. 493-495; Mc Caman M.W., Stetzler J., Clark B. Synthesis of γ-Glutamyl dopamine and Other Peptidoamines in the Nervous System of Aplysia californica. J. Neurochem. (1985), Vol. 45, No 6, pp. 1828-1835; Evstigneeva R.P., Zheltukhina G.A., Ageeva E.A., Babizhaev M.A. Lipoperoxidase activity of carnozine and carcinine. Reports of the Academy of Sciences of the USSR (1993), Vol. 333, No 1, pp: 104-106], and are also producable using an enzymatic method - by combining amino acid and histamine in the presence of an enzyme of hydrolase type [Patent Er.-2,701,947 - September 2nd, 1994].

The esters of N-acyl derivatives of γ-Glutamyl dipeptides produced in US 4,568,489 [of February 4th, 1986] using a DCC-method, are the closest by structure to the claimed compounds of dipeptide nature; however, it is known that the DCC-method used to produce said compounds, can result in side reactions involving the imidazole and indole groups [Schreder E., Lubke K. Peptides. Moscow, Mir publishers (1967), p. 249]. In WO 95/12581 [May 11th, 1995. - C1 CO7D 233/64.-A61K 31/415. - 7/42] pseudopeptide products are disclosed having an imidazole group and possessing antioxidant properties. However, none of the above indicated works discloses the claimed novel imidazole dipeptide derivatives, the processes of their synthesis and their broad spectrum of action.

Description of the invention

The present invention provides a compound which is cyclohexylcarbonylhistamine or N-(imidazolyl-4-acetyl)-heptylamine, or a peptide of general formula I:

$$R_1\text{-}(CH_2)_n\text{-}CONH\text{-}\underset{\displaystyle R_2}{\underset{|}{CH}}\text{-}(CH_2)_m\text{-}R_3 \quad (I)$$

either of structure

| $R_1$ | n | $R_2$ | m | $R_3$ |
|---|---|---|---|---|
| 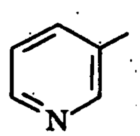 | 0 | H | 1 | 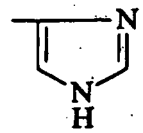 (-4-imidazolyl) |
| 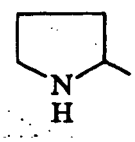 | 0 | H | 1 | 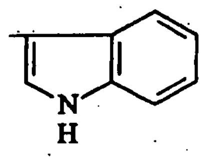 (-3-indolyl) |
| 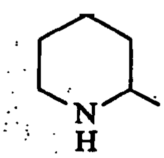 | 0 | H | 1 | 4 imidazolyl |
| 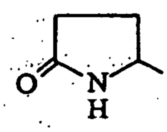 | 0 | H | 1 | -3-indolyl |

or wherein

$R_1$ is a $C_1$-$C_3$-hydrocarbon radical substituted by amino or carboxy, the carboxy group being optionally esterified and the amino group being optionally substituted by acyl or the ester of carbonic acid; or
$R_1$ is a $C_1$-$C_3$ hydrocarbon radical simultaneously substituted by amino and carboxy, the carboxy group being optionally esterified and the amino group being optionally substituted by acyl or the ester of carbonic acid; or
$R_1$ is a $C_1$-$C_3$ hydrocarbon radical substituted by an indole residue or a 5-6 membered unsaturated heterocyclic group the hydrocarbon radical optionally simultaneously comprising an amino group which is unsubstituted or substituted by acyl or the ester of carbonic acid;
$R_2$ is hydrogen or carboxy which is optionally esterified;
$R_3$ is an indole group, which group is unsubstituted or substituted by methyl or hydroxy, the hydroxy group in turn being unsubstituted or substituted by acyl, alkyl or aralkyl; or
$R_3$ is a 5-6 membered saturated or unsaturated heterocyclic group comprising oxygen, sulfur and/or 1-3 nitrogen atoms, or a 5-6-membered unsaturated carbocyclic group, or a methyl derivative thereof; or
$R_3$ is a $C_1$-$C_3$ hydrocarbon radical substituted by amino or carboxy, the carboxy group being optionally esterified; or
$R_3$ is a $C_1$-$C_3$ hydrocarbon radical simultenaously substituted by amino and carboxy;
n is from 0-4 and m is from 1-5;

or a pharmaceutically acceptable salt thereof, for use as an antibacterial, antitumour, antiviral or antimetastatic agent, or to alleviate stress conditions.

Preferably the compound of the invention is for use in combination with a cytostatic agent or radiotherapy. In

another preferred embodiment, when a compound of the invention is for antiviral use, it is for use in treating influenza or HIV.

The present invention also provides novel compounds (defined below). These novel compounds possess (i) antioxidant, antiradical, lipid regulating, hypoglycemic, antiinflammatory, antiaggregate, immunomodulating, antiallergic, antihypoxic, and antiatherosclerotic effects, (ii) the ability to induce the P-450 cytochrome system, to modulate the metabolism of arachidonic acid and adrenocortical hormones, to lower the level of antigen-dependent histamine secretion by peritoneal mast cells, to modulate the activity of macrophages and natural killers, and the interferon system (of cytokines), (iii) activity related to controlling the signs of and preventing asthma and pulmonary emphysema, (iv) wound healing properties, (v) activity related to controlling the signs of skin lesion, e.g. psoriasis, exzema, varicose veins, (vi) activity related to preventing dysfunctional disorders including imminent abortion, dysfunctional uterine bleeding, and amenorrhea, (vii) activity related to controlling the signs of ischemic disease, obesety, and diabetes mellitus, (viii) hepatoprotective properties, (ix) activity related to controlling radiation damage, hepatic lesions including toxic lesions, hepatitis, cirrhosis, and alcohol abuse, (x) the capability to prevent the development of and to eliminate the signs of herontological changes including cataracts, changes in cutaneous teguments, senile psychoses, Alzheimer and Parkinson diseases, (xi) antibacterial and antivirus activity including activity against HIV infection, (xii) antitumor and antimetastatic activity including when administered in combination with cytostatic agents and radiotherapy, and (xiii) adaptogen properties useful for overcoming stress conditions including a hard physical load.

In a preferred embodiment the present invention provides a compound which is a peptide of general formula (I) of claim 1, wherein:

$R_1$ is $NH_2$-$CH_2$-, n is 1-4 or
$R_1$ is HOOC-$CH_2$-, n is 0-3; or
$R_1$ is $R_2$-OCO-$CH_2$-, n is 0-3; Rz is -H or a $C_1$-$C_3$ hydrocarbon radical; or
$R_1$ is HOOC-$CH_2$-($CH_3$)C(Rv)-, Rv is H or $CH_3$; or
$R_1$ is $C_6H_5CH_2$-OCO-NH-$CH_2$-, n is 1-2; or
$R_1$ is Rx-CONH-$CH_2$-, n is 1-4, Rx is a $C_1$-$C_3$-hydrocarbon radical; or
$R_1$ is $CH_3$CONH-CH(CCOOM-, n is 1-2; or
$R_1$ is $CH_3$CONH-CH($[CH_2]_k$COOH)-, n is 0, k is 1-2; or
$R_1$ is $NH_2$-CH($[CH_2]_k$COOH)-, n is 0, k is 1-2; or
$R_1$ is HOOC-CH($NH_2$)-, n is 0; or
$R_1$ is HOOC-CH($NH_2$), n is 1-2; or
$R_1$ is $CH_3$OOC-CH($NH_2$)-, n is 1-2; or
$R_1$ is($CH_3$)$_3$C-OCONH-CH($COOCH_2$-$C_6H_5$)-, nis 1-2; or
$R_1$ is -$CH_2$-4-imidazolyl, -$CH_2$-3-indolyl, n is 0-1; or
$R_1$ is Rb-$CH_2$-CH(NHRy)-, Rb is -4-imidazolyl, -3-indolyl, Ry is tert-butyloxycarbonyl, benzyloxycarbonyl, H-, n is 0;
$R_2$ is -H, -COOH, -$COOR_2$ wherein Rz is -H or a $C_1$-$C_3$-hydrocarbon radical; and

$R_3$ =

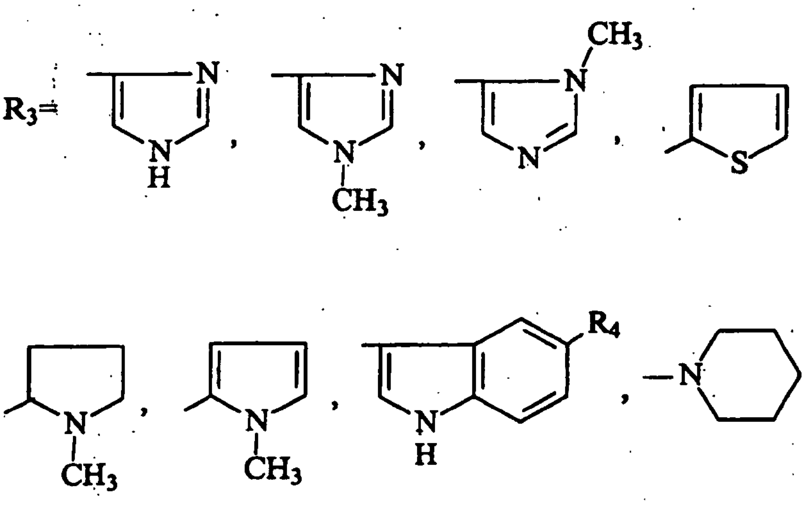

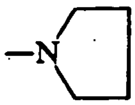 , 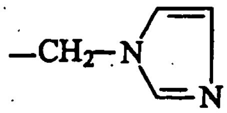 , 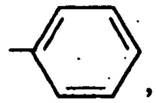 , 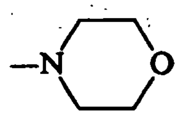 , 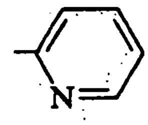

m = 1; or
$R_3$ is $-CH_2-NH_2$, m= 1-2; or
$R_3$ is $-CH_2-COOH$, m =1-2; or
$R_3$ is $-CH(NH_2)-COOH$, m = 1-2,

where $R_4$ = -H, -OH, $-OCH_3$ or $-OCH_2C_6H_5$, for use as an antibacterial, antitumour, antiviral or antimetastatic agent, or to alleviate stress conditions.

Characteristic representatives of the novel dipeptides and pseudopeptides corresponding to general formula (I) are those presented below:

Glutaryl histamine (n=3), Succinyl histamine (n=2)

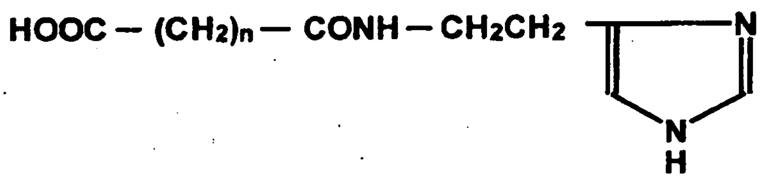

$N^{\alpha}$ -acetyl-L-γ-glutamyl histamine

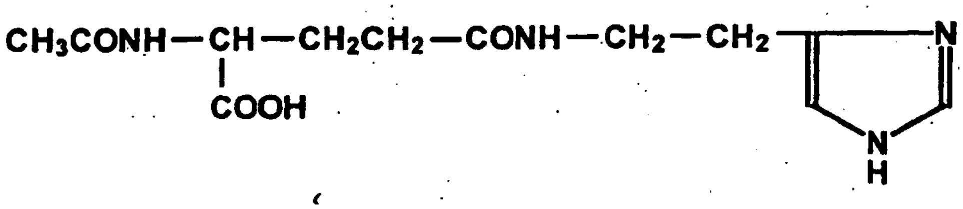

Methyl ester of glutaryl-L-histidine

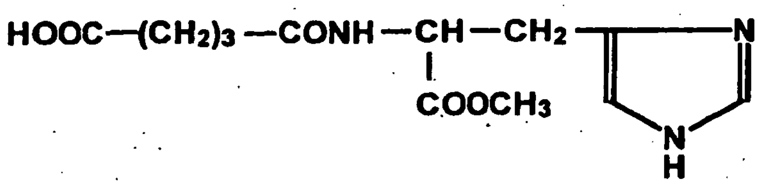

Methyl ester γ-aminobutyryl-L-histidine

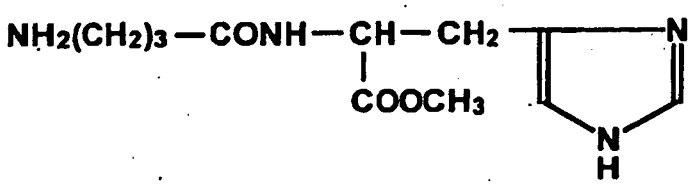

γ-Aminobutyryl tryptamine

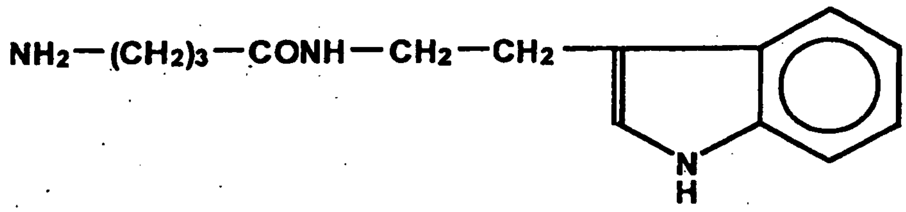

Glutaryl tryptamine

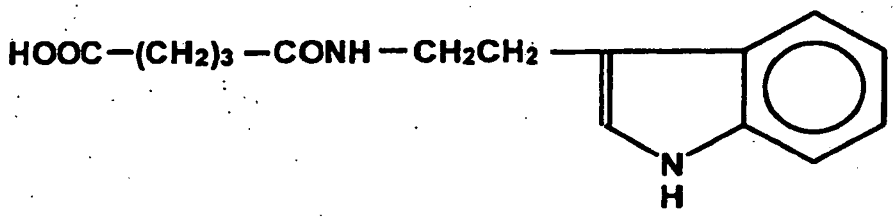

Glutaryl-4-(2-aminoethyl) morpholine

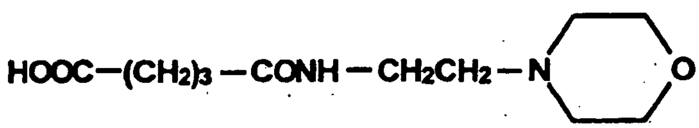

In a preferred embodiment the present invention provides a compound of general Formula (I)

$$R_1\text{-}(CH_2)_n\text{-}CONH\text{-}\underset{\displaystyle R_2}{\underset{|}{CH}}\text{-}(CH_2)_m\text{-}R_3 \quad (I)$$

which is selected from the following compounds

Table 1.

| N° comp. | $R_1$ | n | $R_2$ | m | $R_3$ |
|---|---|---|---|---|---|
| II | $HOOC-CH_2-$ | 1 | H | 1 | 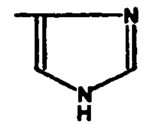 (-4-Im*) |
| III | $HOOC-CH_2-$ | 2 | H | 1 | -4-Im |
| IV | $HOOC-CH_2-$ | 3 | H | 1 | -4-Im |
| V | $CH_3CO-NH-CH(COOH)-$ | 2 | H | 1 | -4-Im |
| VI[a] | $(CH_3)_3COCONH-CH-$<br>\|<br>$COOCH_2C_6H_5$ | 2 | -COOCH3 | 1 | -4-Im |
| VI | $NH_2$ -CH-<br>\|<br>COOH | 2 | -COOCH3 | 1 | -4-Im |
| VII | $HOOC-CH_2-$ | 2 | -COOCH3 | 1 | -4-Im. |
| VIII | $NH_2-CH_2-CH_2-$ | 1 | -COOCH3 | 1 | -4-Im |
| IX | $HOOC-CH(NH_2)-$ . | 2 | -COOCH3 | 1 | -4-Im |
| X[a] | $C_6H_5CH_2OCONH-CH_2-$ | 2 | H | 1 | 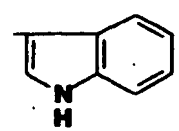 (-3-Ind**) |
| x | $NH_2-CH_2-$ | 2 | H | 1 | -3-Ind |

(continued)

| N° comp. | $R_1$ | n | $R_2$ | m | $R_3$ |
|---|---|---|---|---|---|
| XI | **$HOOC-CH_2-$** | **2** | **H** | **1** | 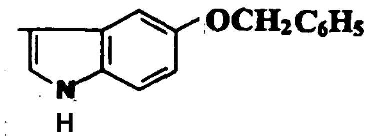 **(-3-Ind-$OCH_2C_6H_5$)** |
| XII | **$HOOC-CH_2-$** | **2** | **H** | **1** | 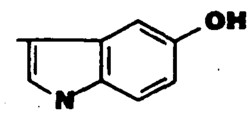 (-3-indolyl-OH) |
| XIII | **$HOOC-CH_2-$** | **2** | **H** | **1** | 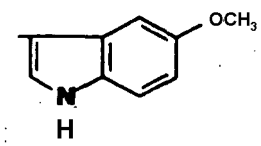 **(-3-Ind-OMe)** |
| XIV | **$HOOC-CH_2-$** | **2** | **H** | **1** | 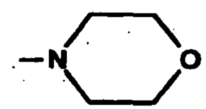 |
| XV | **$HOOC-CH_2-$** | **2** | **H** | **1** | 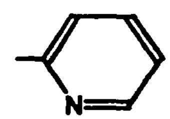 |
| XVI | **$HOOC-CH_2-$** | **2** | **H** | **1** | 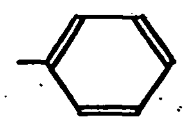 |

(continued)

| N° comp. | $R_1$ | n | $R_2$ | m | $R_3$ |
|---|---|---|---|---|---|
| XVII | $HOOC-CH_2-$ | 2 | H | 1 | -3-Ind |
| XVIII | $HOOC-CH_2-$ | 2 | H | 1 | 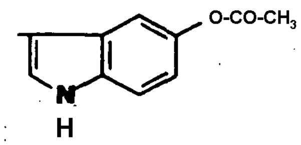 (-3-Ind-O-C(O)-$CH_3$) |
| XXII | $HOOCCH_2-$ | 1 | H | 1 | 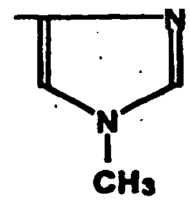 |
| XXIII | $HOOCCH_2-$ | 2 | $COOCH_3$ | 1 | 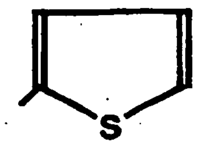 |
| XXIV | $HOOC-CH_2-$ | 2 | H | 1 | 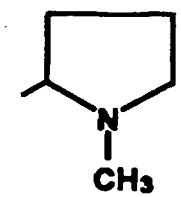 |
| XXV | $HOOC-CH_2-$ | 2 | H | 1 | 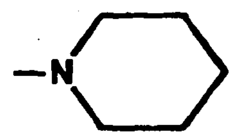 |

(continued)

| N° comp. | $R_1$ | n | $R_2$ | m | $R_3$ |
|---|---|---|---|---|---|
| XXVI | $HOOC-CH_2-$ | 2 | H | 1 | 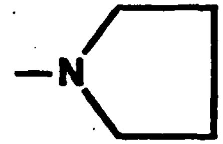 |
| XXVII | $HOOC-CH_2-$ | 1 | H | 2 | 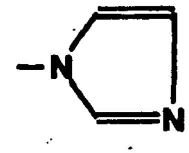 |
| XXVIII | $C_2H_5OCO-CH_2-$ | 0 | H | 1 | -4-Im |
| XXIX | 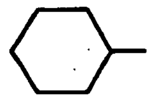 | 0 | H | 1 | -4-Im |
| XXX | 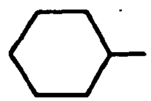 | 0 | H | 1 | -3-Ind |
| XXXIII | -CH2- 4- Im | 0 | H | 4 | $CH_3-CH_2-$ |
| XXXIV | -CH2- 3- Ind | 0 | | 2 | -CH2-(NH2) CH- COOH |
| XXXV | -CH2- 3- Ind | 1 | H | 1 | -CH2-NH2 |
| XXXVI | -CH (NH2)-CH2- 4- Im | 0 | H | 1 | -COOH |
| XXXVII | -CH2-NHCO- CH3 | 2 | H | 1 | -4-Im |
| XXXVIII | $NH_2-CH(COOH)-$ | 1 | H | 1 | -4-Im |
| XXXIX | $NH_2-CH(CH_2COOH)-$ | 0 | H | 1 | -4-Im |

(continued)

| N° comp. | $R_1$ | n | $R_2$ | m | $R_3$ |
|---|---|---|---|---|---|
| XL [a,b,c] | 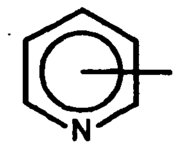 | **0** | **H** | **1** | **-4-Im** |
| XLI | | **0** | **H** | **1** | **-3-Ind** |
| | 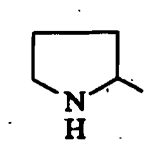 | | | | |
| **XLII** | 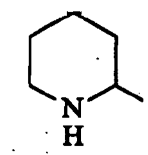 | 0 | H | 1 | -4-imidazolyl |
| **XLIII** | 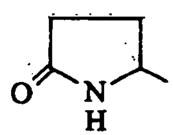 | 0 | H | 1 | -3-indolyl |
| **Q** | HOOC-$CH_2$-C($CH_3$)H- | 1 | H | 1 | -4-Im |
| (*Im=imidazolyl, **Ind=indolyl) | | | | | |

for use as an antibacterial, antitumour, antiviral or antimetastatic agent, or to alleviate stress conditions.

In a particularly preferred embodiment the present invention provides a compound of structure

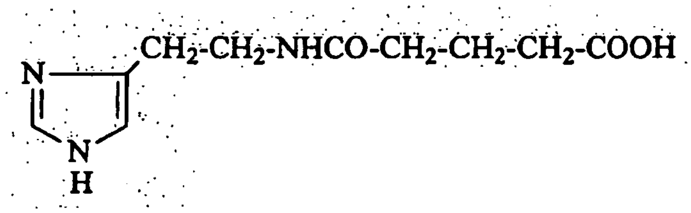

or a pharmaceutically acceptable salt thereof for use as an antibacterial, antitumour, antiviral or antimetastatic agent or to alleviate stress conditions.

In another preferred embodiment of the present invention, the compound which is for use as an antibacterial, antiviral or antimetastaric agent, or to alleviate stress conditions is a known compound of formula (I) wherein (i)

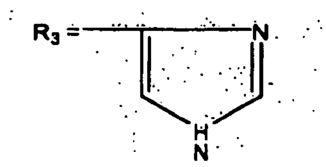

, m=1,

and either (a) $R_1$ = $NH_2CH_2$-, n=1-2, $R_1$ = $CH_3CONH$=CH(COOH)=, n=1; $R_1$ = $CH_3CONH$-CH($CH_2$COOH)-, n=0, or $R_1$-CO = pyroglutamyl, n=0, and $R_2$ = -H or (b) $R_1$ =$NH_2$-CH(COOH)-, n=2, and $R_2$ = -COOH,
or (ii) $R_1$ = $NH_2CH_2$-, n=1-2, or $R_1$ = $NH_2$-CH(COOH)-, n=2, and $R_2$=H,

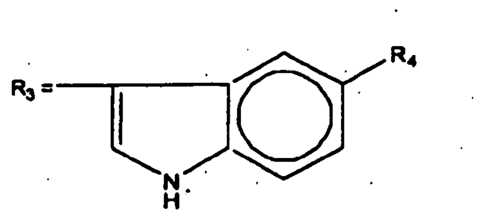

wherein $R_4$ = -H, -OH, -$OCH_3$, or -$CH_2C_6H_5$, and m=1.

In a more preferred aspect of this embodiment of the present invention the compound which is for use as an antibacterial, antitumour, antiviral or antimetastatic agent, or to alleviate stress conditions corresponds to general formula (I), wherein (i)

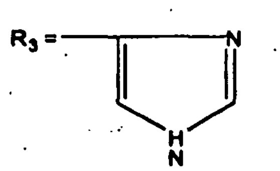

,

m=1,
and either (a) $R_1$ = $NH_2CH_2$-, n=1-2, $R_1$ = $NH_2$CH(COOH)-, n=2, $R_1$ = $CH_3CONH$-CH(COOH)-, n=1, $R_1$= $CH_3CONH$-CH($CH_2$COOH)-, n=0, or $R_1$-CO- = pyroglutamyl, n=0, and $R_2$ = -H, or (b) $R_1$ = $NH_2$-CH(COOH)-, n=1-2, and $R_2$=-COOH,
or (ii) $R_1$ = $NH_2CH_2$-, n=1-2, or $R_1$ = $NH_2$-CH(COOH)-, n=2, and $R_2$=H,

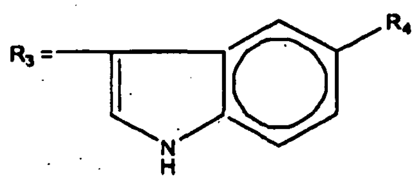

and m=1, wherein $R_4$ = -H or -OH.

Characteristic examples of the preferred known pseudopeptides corresponding to general formula (I), are presented below:

γ-L-, D-glutamylhistamine (XLIV, XLV)

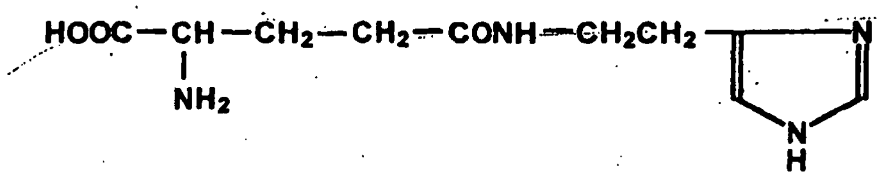

β-Alanylhistamine, n=2; γ-Aminobutyrylhistamine, n=3 (XLVI, XLVII)

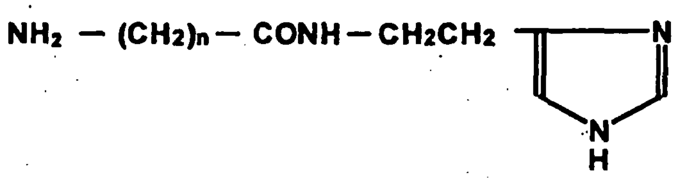

γ-L-glutamyltryptamine (XLVIII)

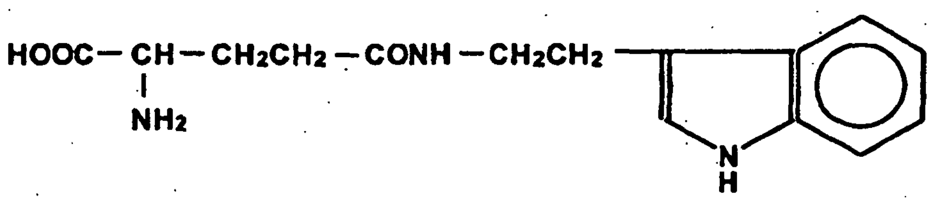

γ-L-glutamylserotonine (XLIX)

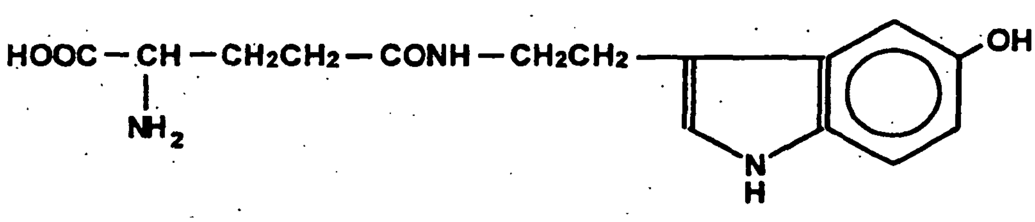

N-Acetyl-β-Aspartylhistamine (L)

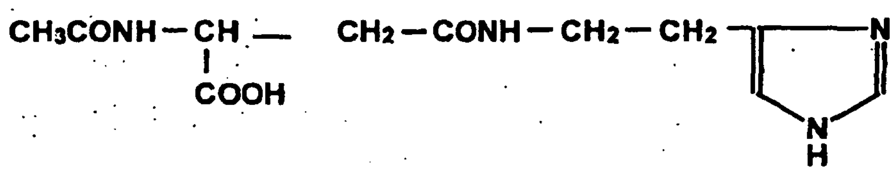

N-Acetyl-$\alpha$-Aspartylhistamine (LI)

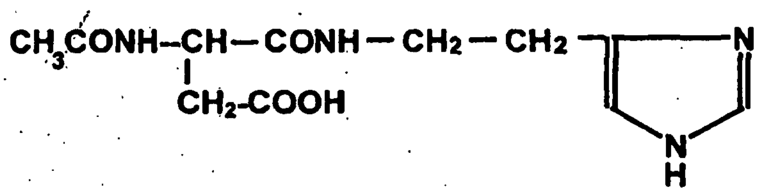

Pyroglutamylhistamine (LII)

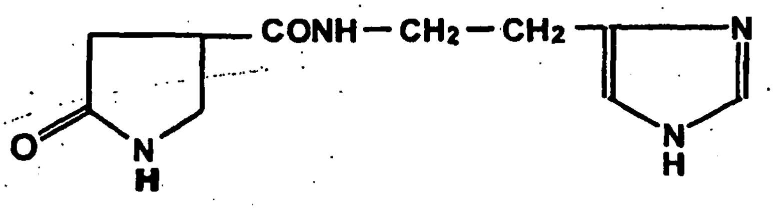

The present invention also provides novel compounds. The novel compounds are of structure (II), (III), (IV), (V), ($VI^a$), (VI), (VII), (VIII), (IX), ($X^a$), (X), (XI), (XII), (XIII), (XIV), (XV), (XVI), (XVIII). (XXII), (XXIII), (XXIV), (XXV), (XXVI), (XXVII), (XXVIII), (XXIX), (XXXIII), (XXXIV), (XXXV), (XXXVII), (XXXVIII), (XXXIX), ($XL^{a,b,c}$) (XLI), (XLII), (XLIII) or (Q) as defined above or a pharmaceutically acceptable salt thereof. Preferred is glutarylhistamine of formula

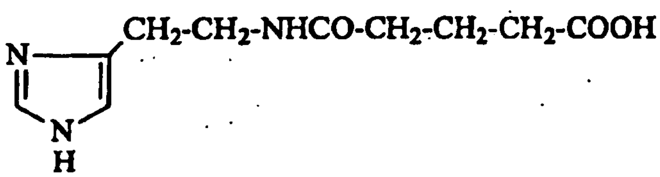

or a pharmaceutically acceptable salt thereof.

The present invention also provides a novel compound of the present invention for use in a method of treatment of the human or animal body by therapy.

In one embodiment the novel compounds of the present invention are for use as an antibacterial, antitumour, antiviral or antimetastatic agent, or to alleviate stress conditions.

In another embodiment the novel compounds of the present invention are for use as an antihypoxic, a lipid regulator, or a hypoglycemic agent, for controlling symptoms of or preventing asthma or pulmonary emphysemia, for controlling symptoms of cutaneous lesions, psoriasis or eczema, for preventing abortion, dysfunctional uterine bleeding or amenorrhea, for controlling the symptoms of ischemic disease or obesity, for use as a hepatoprotective agent, or for controlling or preventing the symptoms of gerontological disease.

Preferably in this embodiment the novel compound of the invention is for use in controlling hepatic lesions including toxic lesions, hepatitis, cirrhosis or alcohol abuse.

The present invention also provides a process for producing a novel compound of the present invention, which process comprises the amino group acylation of an amino compound of general formula $NH_2-CH(R_2)(CH_2)_m-R_3$ wherein $R_2$, $R_3$ and m are as defined above, with, as acylating agent, an activated carboxy compound of general formula $R_1-(CH_2)_n-COX$, wherein X is an activating group.

In a preferred aspect of this process of the present invention, $R_1$ is HOOC-$CH_2$-, n is 0-3; or $R_1$ = HOOC-$CH_2$-($CH_3$)C(Rv)-, n = 1 and Rv = H or $CH_3$; and $R_2$ is H or -$COOCH_3$, the activated carboxy compound is an anhydride of a dicarbonic acid and the process is carried out in an organic solvent.

In another preferred aspect of this process of the present invention, when $R_1$ = -$CH_2$-$NH_2$, n =1-2; $R_2$ = -H,

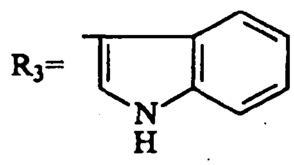
, m=1

,

or

when $R_1$ = HOOC-CH($NH_2$)-, n = 1-2; and

$R_2$ = -$COOCH_3$ or H; and

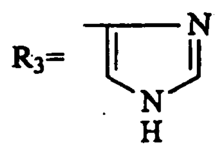
, m=1.

the acylating agent is a pentafluorophenyl ester of N-benzyloxycarbonyl-γ-aminobutyric acid or N-benzyloxycarbonyl-j3-alanine or an α-benzyl ester of N-tert-butyl oxycarbonyl-L-glutamic or aspartic acid and wherein the process is carried out in an organic solvent with subsequent cleavage of the N-benzyloxycarbonyl protecting group and α-benzyl ester or pentafluoro-phenyl ester from the protected derivative of a dipeptide using catalytic hydrogenolysis, with subsequent cleaving of the N-tert-butyl oxycarbonyl group in an anhydrous acidic medium.

In another preferred aspect of this process of the present invention, when $R_1$ = $CH_3$CONH-CH(COOH)- and n = 1-2; or $R_1$ = $CH_3$CONH-CH($[CH_2]_k$COOH)-, n = 0, k = 1-2; or $R_1$ = $CH_3$CONH-$CH_2$-, n = 2-4; and $R_2$ = H,

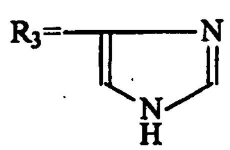
or
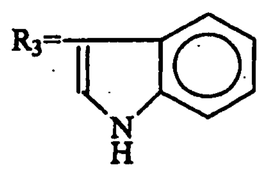

and m = 1,

the acylating agent is p-nitrophenyl acetate.

The compounds of the present invention are producable by acylation of an amine or amino acid amino group with a carbonic group, a dicarbonic group or an N-protected amino acid derivative with an activated carboxy group.

Synthesis of dipeptides and pseudopeptides comprising N-amino acylic substituent, can be performed according to classical methods of peptide chemistry, advantageously using activated species, for instance, N-oxysuccinimide esters. Preferred embodiments include the use of pentafluorphenyl esters, which are the most active known esters. As the activated derivatives of dicarbonic acids, cyclic internal anhydride thereof are preferably used.

The α-amino group of the carboxylic or amino component can be protected by different conventionally used groups, preferably a tert.-butyloxycarbonyl (Boc-) or benzyloxycarbonyl (Z-) protective group.

The α-carboxy groups of glutamic and asparagic acid are preferably protected by a benzyl (Bzl-) group and ornithine by a tert.-butyl group.

In the compounds of general formula (I), the carboxy group of the amino component histidine can be in the form of methyl esther or can remain unprotected.

The synthesis of compounds comprising an N-amino acyl substituent is shown in the following scheme 1:

Stage 1. **$R_y$-NHA-COOX+$NH_2$B→$R_y$-NH-A-CONHB**
Stage 2. **$R_y$-NH-A-CONH-B→$NH_2$-A-CONH-B**

**Stage 3.**

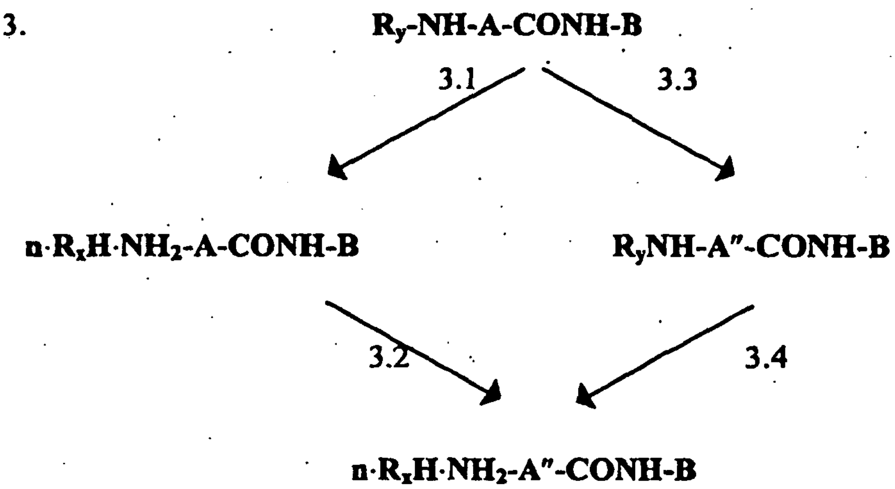


Stage 4. n·$R_x$H·$NH_2$-A"-CONH-B→$NH_2$-A"-CO-NH-B,

where $R_y$ = Boc-, Z-; $NH_2$-A-CO- = β-Ala-, γ-Abu-, H-L-Glu-OBzl, H-L-Asp-OBzl; $NH_2$-B = HA, H-His-OH, H-His-OMe, TrpA, H-Trp-OMe, 5-(OMe)TrpA, 5-(OBzl)TrpA; $NH_2$-A"-CO = H-L-Glu-, H-L-Glu-OMe, n•$R_x$H = HHal (HCl), $CF_3COOH$, n =1, 2.

Stage 1 is generally carried out in the medium of a dehydrated aprotonic solvent, preferrably dimethyl formamide (DMF), for 18-48 hours at room temperature, except for when producing dipeptide Boc-L-Glu(L-His)-OBzl. The latter is typically produced by the action of a three-fold excess of Boc-L-Glu(ONSu)-OBzl on unprotected L-histidine in anhydrous-dioxanic medium (1:1). The advantage of this method is in the simplification of the process due to the reduced number of stages (absence of the need to introduce and remove histidine Cprotection) and the possibility of producing the dipeptide wherein one of two carboxy groups is selectively protected.

If necessary, the protective groups of the intermediate compound $R_y$ -NH-A-CONH-B can be cleaved off in accordance with stages 2 and 3.

Stage 2 can be carried out by way of catalytic hydrogenolysis only in the case where $R_y$ =Z-, and $NH_2$-A-CO- =β-Ala-γ-Abu-.

Stage 3 ,if necessary, can be accomplished in two different modifications when groups -Boc- and Bzl are present in the intermediate compound $R_y$-NH-A-CONH-B, namely for the derivatives of glutamic and aspartic acids. Method 3.1 consists in acidolytic cleaving off of the $N^{\alpha}$-Boc-protecting group, for instance by the effect of hydrochloride in an organic solvent which is advantageously dioxan, methanol or a mixture thereof; or trifluoroacetic acid. Catalytic hydrogenolysis can be used to subsequently remove the Bzl-group (3.2). According to method 3.3, hydrogenolysis is first carried out and then, acidolytic cleaving off of the $N^{\alpha}$-protection (3.4). As a result of stage 3, products in the form of respective salts are produced.

If it is necessary to produce target compounds in the form of free bases, stage 4 is carried out.

If a compound does not comprise unprotected carboxy groups, it can be produced in the form of a free base by adding organic ($Et_3N$) or inorganic (NaOH) base, with subsequent isolation of the base salt from the target product. In addition, ion-exchange chromatography can be used to achieve this goal, in accordance with the technique described in Evstigneeva R.P., Zheltukhina G.A., Ogrel' S.A., Nebol'sin V.E. Synthesis of pseudopeptides based on biogenic amines., Reports of the Academy of Sciences of the USSR (1995), Vol. 345, No 4, pp. 493-495.

In addition, if necessary, the compound can be produced in the form of a base, in the form of a transition metal salt with formation of a chelate.

The known compound γ-L-Glu-HA [Konishi H., Kakimoto Y. Formation of γ-Glutamylhistamine from histamine in rat brain, J.Neurochem. (1976), Vol. 27, pp. 1461-1463], from which the nivel compound of structure (V) as defined above may be produced, can itself be produced using the method described in Mc Caman M.W., Stetzler J., Clark B. Synthesis of γ-Glutamyldopamine and Other Peptidoamines in the Nervous System of Aplysia californica, J. Neurochem. (1985), Vol. 45, No 6, pp. 1828-1835. The N-acetyl derivative γ-L-Glu-HA-Ac-γ-L-Glu-HA (V), can be produced in accordance with scheme 2.

Scheme 2

$$AcONp + H\text{-}\gamma\text{-}L\text{-}Glu\text{-}HA \xrightarrow{DIOXAN/H_2O} Ac\text{-}\gamma\text{-}L\text{-}Glu\text{-}HA$$

Scheme 2, which introduces an acetyl group into a dipeptide, is advantageous over using an N-acetyl derivative of glutamic acid as an initial product for creating a peptide bond, since it reduces the possibilty of racemization. The use of p-nitrophenylacetate is preferrable as compared with acetic anhydride, since its use is not accompanied by side reactions of the imidazole group, which consist of the formation of an acetyl derivative by imidazole and the respective salt of the acetil anhydride, with the release of a molecule of acetic acid.

Synthesis of compounds of the present invention which comprise a dicarbonic acid residue can be accomplished using different methods, preferrably in accordance with scheme 3, where an internal cyclic anhydride is used as a C-activated carboxy compound.

Scheme 3

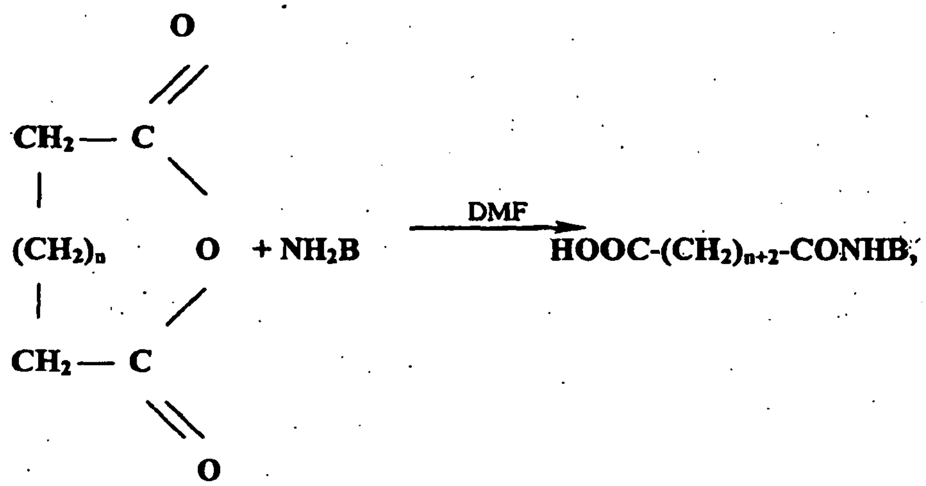


where n=0-1 and $NH_2B$ = HA, H-His-OMe,

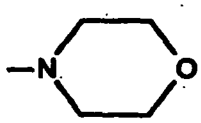 , 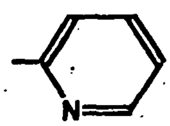 , 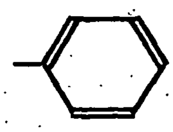 ,

or

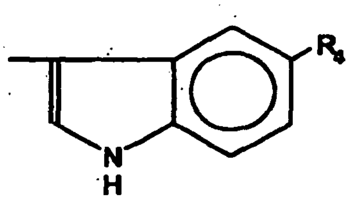

where **$R_4$=-H,-OH, -$OCH_3$, -$OCH_2C_6H_5$.**

In the case where an appropriate anhydride is not available, e.g. for adipinic acid, pseudopeptide synthesis can be accomplished using DCC. In this case, a reaction between dicarbonic acid and DCC is first carried out, the ratio being 2:1 M/M, and then the amino component is added (amine or an amino acid derivative).

Synthesis of compounds of the present invention which comprise an N-acyl residue of a fatty acid can be accomplished using chloroanhydride.

The present invention also provides a pharmaceutical or cosmetic composition comprising a pharmaceutically acceptable carrier or diluent and, as an active compound, a novel compound of the present invention as defined above. Such compositions possess antihypoxic; antioxidant, antiradical, lipid-regulating, hypoglycemic, antiaggregate, immunomodulating, wound healing, antiallergic, antiasthmatic, antiviral, antibacterial, antitumor, antimetastatic and adaptogenic effects and the ability to induce the hepatic P-450 cytochrome system, to modulate the metabolism of arachidonic acid and adrenocortical hormones, to lower the level of antigen-dependent histamine secretion by mast cells, to modulate the activity of macrophages, natural killers, and the interferon system (of cytokines), and to prevent abortions and dysfunctional uterine bleedings, and the signs of diabetes mellitus, obesity, ischemic heart disease, stress conditions, hepatitis, cirrhosis, toxic liver lesions, alcohol abuse, radiation injuries, and herontological changes.

Pharmaceutical compositions of the present invention can be used in the form of pharmaceutical preparations (for instance, in solid, semisolid and liquid forms), comprising a novel compound of the present invention as an active ingredient in a mixture with an organic or inorganic carrier or excipient, suitable for intramuscular, intravenous, intranasal, oral, sublingual inhalation or intrarectal administration. The active ingredient can be included into the pharmaceutical composition together with conventionally used non-toxic, pharmaceutically acceptable carriers, suitable for producing solutions, tablets, pellets, capsules, suppositoria; emulsions, suspensions, ointments or any other dosage forms. Water, glucose, lactose, gum arabic, gelatin, starch, magnesium trixylitol, maize starch, urea and other carriers suitable for preparing solid, soft or liquid preparations, can be used as carriers. In this case, stabilizers, thickeners, stains and flavours can be used as supplements.

The active target compound is introduces into a pharmaceutical composition in an amount sufficient to achieve the necessary effect depending on nosology, course and severety of disease.

In producing a single dosage form, the amount of active ingredient used in combination with a carrier, can vary depending on the recipient subjected to therapy and on the particular administration method.

Thus, for instance, in using a compound of present invention in the form of a solution for injection, the active ingredient content is 0.01-0.03%. As a diluent, 0.9% sodium chloride solution, distilled water, novocaine solution for injections, Ringer solution or glucose solution can be used. If using a compound of the present invention in the form of a tablet in suppository, the amount of active ingredient is 3.0-30.0 mg per single dosage form. For tablets and suppositoria, any pharmaceutically suitable base can be used as a pharmaceutical excipient.

To treat and prevent diseases and conditions connected with allergy, inflammation, bronchial asthma, pulmonary emphysema, psoriasis, eczema, varicose veins, dysfunctional disorders, imminent abortion, uterine bleedings, atherosclerosis, ischemic disease, obesity, diabetes mellitus, infections (viral and bacterial ones), oncology, hepatic lesions (hepatitis, cirrhosis), alcohol abuse, and toxic, radiation and herontological lesions as well as, in cases of need, to speed up wound healing, to exert an adaptogenic effect, to induce the hepatic P-450 cytochrome system, to modulate the metabolism of arachidonic acid and adrenocortical hormones, to reduce the level of antigen-dependent histamine secretion by mast cells, and to modulate the activity of macrophages, natural killers, and the interferon systems (cytokines), the compounds of the present invention as defined above can be administered orally, locally, parenterally, by inhalation or rectally in the form of single dosage forms comprising standard, non-toxic pharmaceutically acceptable carriers. The term "parenteral administration" as used herein, means subcutaneous, intravenous, intramuscular or intrathoracic injections and infusions.

The compounds of the present invention can be administered once daily at doses 0.03 to 0.5 mg per 1 kg of body weight per day for 5 to 10 days.

It should be noted that the particular dose for each individual patient will depend on many factors including the activity of the given compound being used, age, bodyweight, gender, health condition and nutrition regimen of the patient time and method of the therapeutic agent administration, the excretion rate of the agent from the body, the particular combination of therapeutic agents being used as well as the severity of disease being treated.

Dosage forms of the present invention can be produced using standard techniques.

It should be noted that the compounds of the present invention as defined above manifest biological activity at doses which are two to three orders lower than those of the known drugs used in comparative applications, in practically similar efficacy. Also, no side effects were detected for the compounds of the present invention as defined above and they have no contraindications for their use. At the same time, in toxicity testing, $LD_{50}$ was not found since upon administration to animals (e.g. one of the compounds was tested at an oral dose of 5000 $\mu$g/kg, and a parenteral dose of 10,000 $\mu$g/kg) death of experimental animals was not observed.

The antihypoxic activity of the claimed compounds allows one to use them as antiischemic agents, since it is known that lowered blood supply and hence, lower oxygen delivery to the specific cardiac region, is the most important factor in necrosis focus development.

The ability of the claimed compounds to modulate biochemical and physiological systems, i.e. the hepatic P-450 cytochrome system, hormonal background, and lipid peroxidation system (LP) and antiradical activity, arachidonic

acid metabolism, spontaneous and antigen-stimulated histamine secretion by peritoneal mast cells, explains their pronounced antiallergic and antiinflammatory, activity.

In addition, the endogenic rise in the level of precursors and glucocorticoid hormones as effected by the test compounds, can be used in the therapy of diseases associated with the reduced synthesis of these hormones in the body. Exogenic administration of hormonal drugs is known, as a rule, to result in systemic complications and it is a risk factor of the development of cancer diseases [Parker K.L., Schimmer B.P. The Role of Nuclear Receptors in Steroid-Hormones Production. Seminars in Cancer Biology (1994), Vol. 5, Iss. 5, pp. 317-325]. Also, it inhibits the synthesis of the respective hormones in the body. In particular, a positive effect of using the claimed compunds can be predicted in pathological conditions that are accompanied by a decrease in the level of corticosteroids: allergic and inflammatory diseases, hepatic lesions of different etiology, and gynecological diseases. The detected elevation in the amount of blood oxyprogesterone can be of importance in the therapy of a number of gynecological diseases, such as infertility, imminent abortion, dysfunctional uterine bleeding, etc. Since glucocorticoids and vasodilators are known for use in the therapy of cutaneous manifestations of allergic diseases, as well as a number of cutaneous diseases, including psoriasis, then the claimed compounds can be used in the therapy of these diseases.

Since the claimed compounds possess the ability to alleviate the signs of allergic and inflammatory reactions by local application, as well as to increase the synthesis of endogenous antiaggregate agents - prostaglandin $E_2$ and prostacycline (6-keto-$PGF_1$), then their use is possible in the form of an ointment in treating varicose veins development, wherein in the pathogenesis of the varicose veins development components which cause inflammation, a rise in blood coagulation, etc are present.

Aging processes are known to be associated with oxidative stress accompanied by elevated LP and as a sequence of this, MDA production, which, interacting with lysine of proteins, forms a lipofuscine-like pigment. This probably speeds up lipofuscinosis, which causes irreversible morphological shifts. Lowering of the MDA level by the claimed compounds decreases the probability of the given pigment being produced, so can result in decreased probability of age-dependent disease development. In addition, an increase in the average lifetime of experimental animals can serve as confirmation of the possibility to use the tested compounds in age-dependent diseases when low doses of Phenobarbital are used. [see Emanuel N.M., Obukhova L.K., Hajdich V.I., Murza L.I., Bunto T.V. Aging Inhibition through Activation of the System of Microsomal Oxidases with Phenobarbital. Reports of the USSR Academy of Sciences (1977 (, Vol. 235, No 4, pp. 957-960]. The claimed compounds cause changes in the hepatic P-450 cytochrome system similar to those caused by phenobarbital, and their effect on the body can result in a decrease in the rate of aging. Herontological disorders, include such pathological conditions as senile psychoses, cataract, senile changes in cutaneous teguments. The claimed compounds can also be used for therapy of Parkinson is disease, cataracts, and senile changes in cutaneous teguments, one of the pathogenesis links of which is oxidative stress in the nervous tissue (increase in LP).

The claimed compounds possess a pronounced hepatoprotective effect. This effect of the compounds is explained by the fact that upon their administration into the body, a functional reconstruction of many central body systems participating in maintaining homeostasis occurs. Thus allows their use in treating disorders caused by the effect on the body of factors such as stress, physical load, ionizing radiation, in preventing radiotherapy side effects, for activating reparation processes; in treating hepatic diseases (cirrhoses, hepatites of different etiology); in treating the conditions associated with intoxication by substances which activate lipid peroxidation, for instance, in alcohol abuse; and as prophylactic drugs in industries connected with the production of chemical reagents and exposure to toxic substances.

The claimed compounds can normalize the lipid metabolism parameters and they have antihypoxic and antiischemic efficacy. This allows one to propose to use them in the prophylaxis and therapy of diseases connected with lipid metabolism disorders characterized by an elevation in the level of triglycerides, total cholesterol (CS), low and very low density lipoptrotein cholesterol (LDL and VLDL) and by a decrease in the level of high density lipoprotein cholesterol, i.e, diseases such as atherosclerosis, obesety, ischemic heart and cerebral disease, myocardial infarction, stroke, risk factor for diabetes mellitus, manifestation and thrombogenesis.

The homeostasis stabilizing effect of the claimed compounds serves as the basis for recommending them for use in the therapy of diabetes mellitus.

The claimed compounds significantly inhibit the growth of a transplantable tumor and its metastasizing process, and they elevate the resistance of animals to microbial and viral infections.

The inhibition of tumor growth and metastasizing processes is connected with a number of bioigical effects of the claimed compounds : with change in the P-450 cytochrome system condition, with change in hormonal status, with the antioxidant and antiradical properties of the claimed compounds, and with an increase in the activity of phagocytes (neutrophils and peritoneal macrophages), natural killer cells and the production of interferons (cytokines) under stress effect. This allows the use of the claimed compounds in cancer therapy, since all the systems listed above are known to participate in maintaining homeostasis in the body and to undergo significant changes with cancer. Decreased 12-HETE production could be one more explanation for the antimetastatic effect of the claimed compounds, since this arachidonic acid metabolite is known to potentiate the process of metastasizing [Honn K.V., Tang D.G., Gao X., Butovich I.A., Liu B., Timar J., Hagmann W. Cancer and Metastasis Reviews (1994), Vol. 13, Iss. 3-4, pp. 265-396, Ref: 195]. An

increase in prostacycline/thromboxane ratio could also play a role in the antimetastatic effect of the claimed compounds. In addition, the compounds rase the antitumor activity of cyclophosphane, which is widely used in cancer therapy. An important characteristic of the claimed compounds is that they exhibit therapeutic properties upon oral administration. In administering a dosage form, therapeutic activity is comparable with the use of the substance. Decreasing the number of metastases is of great practical importance since the rate of this process course is extremely important and its inhibition results in a more favorable prognosis in cancerogenesis.

It has been established that normalization of the above mentioned parameters in animals with administration of the claimed compounds, especially the rise in the number of active peritoneal macrophages, the increase in the number of active. NK cells actively participating in the immune body state maintenance under stress effects and the rise in γ-interferon production which inhibits in vitro proliferation of the cells transformed by virus, can explain the increase in animals resistance to microbial and viral infections. The protective effect of the claimed compounds, via a cytodestructive effect on the human immunodefficiency virus, should be especially emphasized. The antiviral effect of the compounds can also be connected with lipid peroxidation normalization.

The ability of the claimed compounds to potentiate mitogen-simulated blasttransformation of human lymphocytes, can serve as the basis for using the tested compounds in lesions of cutaneous teguments, in particular, to speed up wound healing [Koyama, Masayoshi, Takahashi, Mikiko, Yanagawa, Masayoshi EP 95-107298 950513; JP 94-115161 940527. Production of L-lysyl-glycyl-L-histidine and therapeutic agents for wound healing containing the same. Eur. Pat. Appl., 5 pp.].

Thus, the tested compounds of the present invention as defined above can be recommended for use in the therapy of cancer diseases, bacterial and viral infections, and especially HIV infection.

The broad spectrum of action of the claimed compounds can be partially explained by the fact that these compounds act on the central body systems which stabilize homeostasis, for instance, the P-450 cytochrome system, LP and phagocytosis system. A change in the activity of one system in a live organism is known to be followed by changes in the other systems, and these changes attain a cascade character. With administration of the claimed compounds into the body, normalization of many bodily functions occurs. Thus, the claimed compounds act as adaptogens.

The present invention relates to methods of therapy and prevention of diseases of warm-blooded animals, the methods comprising administration of compounds of the present invention as defined above in an efficient amount. Diseases and uses for compounds of the present invention in this regard include: stress conditions, allergic diseases of immediate and delayed type - bronchial asthma, food allergy, anaphylactic shock, allergic eczemas as well as psoriasis, atherosclerosis, ischemic disease (of the heart and brain), obesety, type 1 and 2 diabetes mellitus, alcohol abuse, alcohol intoxication, varicose veins, prevention of thrombogenesis; hepatitis, hepatic cirrhosis; toxic hepatic lesions; nervous system diseases, Parkinson’s disease; herontological diseases: cataract, senile change in cutaneous teguments; radiation injury, elimination of radiotherapy consequences; viral and bacterial infections, in particular, SHIV infection; cancer diseases; hard physical load; to speed up wound healing. The compounds of the present invention as defined above can be used in medicine and veterinary practice for therapy and prevention of the above listed diseases.

Specific examples are given to illustrate the present invention which should not be considered as any limitation of the scope of the given invention.

The following abbreviations are used in the examples presented below:

AAS - active anaphylactic shock
AC - arachidonic acid
ALT - alanine aminotransferase
AN - activated neutrophils
AsA - ascorbic acid
AST - aspartate aminotransferase
AOF - active oxygen forms
HPLC - high performance liquid chromatography
DTH - delayed type hypersensitivity
ITH - immediate type hypersensitivit
HS - hexenal sleep
DMF - dimethyl formamide
MII - metastasizing inhibiton index
LD - lethal dose
LA - linolic acid
LO - lipoxygenase route
HDL - high density lipoproteins
LDL - low density lipoproteins
VLDL - very low density lipoproteins

LT - leukotrienes
MDA - malonic dialdehyde
MP - macrophages
NR - neutral red
NK - natural killer cells
NBT - nitroblue tetrazolium (formazan)
OVA - chicken ovalbumin
PG - prostaglandins
PCA - passive cutaneous anaphylaxis
PM- peritoneal macrophages
LP - lipid peroxidation
MLT - mean lifetime
FC - free cholesterol
TBA - thiobarbituric acid
TG-triglycerides
TGlu - transglutaminase
MC - mast cells
TMS - tetramethylsilane
TCA- trichloracetic acid
TX - thromboxane
TLC - thin layer chromatography
TCD - tissue cytopathic dose
P - phospholipids
PB - phenobarbital
PG - phagocytes
NS - normal saline
L - chemiluminescence
CS -cholesterol
CNS - central nervous system
CO - cyclooxygenase route
EDTA - ethylenediamide-tetraacetate
NMR - nuclear magnetic resonance
$Bu^tOH$ - tert-butanol
But - tert-butyl
Bzl - benzyl
Boc - tert-butyloxycarbonyl
DCC - N,N'- dicyclohexylcarbodiimide
DCU - N, N'- dicyclohexylurea
DMF - N, N - dimethylformamide
$Et_3N$ - triethylamine
EtOAc - ethylacetate
EtOH - ethanol
EET - epoxyeucozatrienic acid
GA - glutaric acid
Glu - glutamic acid
F - cortisol
HA - histamine
HETE - (S)-hydroxy-6,8,11,14-eucozatrienic acid
5-HT - 5-hydroxytryptamine (serotonin)
Ind - indol
Im - imidazol
MeCN - acetonitryl
MeOH - methanol
$OBu^t$ - tert-butylic ether
ONSu - N-oxysuccinimidyl
OPfp - pentafluorophenyl
Py - pyridine
P-4508 - a group of P-450 cytochromes with active center, oriented into cytosol

P-450L - a group of P-450 cytochromes with active center, oriented into phospholipid membrane
TrpA - tryptamine
TFA - trifluoracetic acid
Z - benzyloxycarbonyl

The glutamic acid, aspartic acid, histidine, tryptophan and ornithine derivatives used in synthesis are the L-row derivatives unless D-derivative is especially indicated. Individuality of the compounds produced is checked using TLC method on Silufol plates of the Kavalier company, UV-254 (Czechoslovakia) in the solvent systems chorophorm-methanot 9:1 (1), chloroform-methanol 8:2 (2), n-butanol-acetic acid-water 4:1:5 (3); on Kieselgel plates of the Merck company in the solvent systems chloroform-methanol-25% hydrous ammonia 5:3:1 (4); on Silufol plates: chloroform-methanol 9,3:0,7 (5); chloroform-methanol-25% hydrous ammonia 5:3:0,5 (6), chloroform-methanol 8,5:1,5 (7); isopropanol-water-25% hydrous ammonia 6:3:1 (8), chloroform (9), on Kieselgel plates of the Merck company in the solvent system isopropanol-water-25% hydrous ammonia 6:1:3 (10).

Chromatograms are developed with chlorotolidine solution, Pauly and Erlich reagents, ninhydrine and in UV light.

The melting temperature of compounds is determined using the Boetius instrument (Germany).

The $^1$H-NMR spectra are taken on the Brucker WM-250 apparatus (Germany) and the Varian XL-400 (Japan) with TMS as an internal standard.

Mass-spectrometry is carried out on the MSBCh instrument (Sumy, Ukraine) using the plasma-desorption ionization method with nuclear fragments of californium 252.

Analytical reversed phase HPLC is carried out under the conditions (1): the MPS-270 C-18 column (4,0 x 250 mm), 10 μm elution with 4% acetonitryl in water comprising 0,1% TFA; (2): the same column, elution with gradient from -10% to 50% of B phase in A phase for 20 minutes; A phase - 0,1 % TFA in water, B phase - 0,09% TFA in the mixture of acetonitryl and water 60:40; (3): the MPS-300 C-18T column (4,0 x 250 mm), 10 μm, elution with gradient from 0% to 40% of B phase in A phase for 20 minutes. A phase - 0,1% TFA in water, B phase - 0,09% TFA in the mixture of acetonitryl and water 60:40; (4) the MPS-270 C-18 column (4,0 x 250 mm), 10 μm, elution with 0,1M $Na_2HPO_4$, pH 2,3; (5) : the same column, elution with 0,1M $Na_2HPO_4$, pH 2,7; (6) the Diasorb 130C18T column (4,0 x 150 mm), 7 μm, elution with gradient from 0% to 42% acetonitryl in 0,1% TFA; (7) : the MPS-300 C-18T column (4,0 x 250 mm), 10 μm, elution with gradient from 0% to 18% acetonitryl in 0,1% TFA for 20 minutes; (8) : the Lichrosorb RP-18 column (4,6 x 250 mm), 5 μm, elution with gradient from 6% to 24% acetonitryl in 0,1% TFA for.20 minutes; (9) : the Diasorb 130 C 16T column (4,0 x 150 mm), elution 0,1% TFA; (10) : the Diasorb 130 C 16T column (4,0 x 150 mm), 7 μm, elution with gradient from 0% to 24% acetonitryl in 0,1% TFA for 30 minutes; (11) : the same column, elution with gradient from 3% to 54% acetonitryl in 0,1% TFA for 30 minutes; (12) : the same column, elution with gradient from 0% to 30% acetonitryl in 0,1% TFA for 30 minutes; (13) : the MPS C18T column (4,6 x 250 mm), elution with gradient from 12% to 60% acetonitryl in 0,1% TFA for 20 minutes; (14) : the MPS-270 column (4,0 -x 250 mm), 10 μm, elution with gradient from 0% to 60% acetonitryl in 0,1 % TFA for 20 minutes; (15) : the Diasorb 130 C 16T column (4,0 x 150 mm), 7 μm elution with gradient from 0% to 60% acetonitryl in 0,1% TFA for 30 minutes; (16) : the Diasorb 130 C 16T column (4,0 x 150 mm), 7 μm, elution with gradient from 60% to 100% acetonitryl in 0,1% TFA for 15 minutes.

In all cases, HPLC is carried out at elution rate 1 ml/min with detection at 214 nm.

EXAMPLE 1

N-SUCCINYLHISTAMINE (II)

To 0,080 g (0,72 mM) histamine solution in 4 ml DMF, 0,072 g (0,72 mM) succinic anhydride is added while stirring. The reaction mixture is stirred for 1 hour and left for 20 hours at 20˚C. Solvent is removed under vacuum. Oily residue is dissolved in 1,5 ml ethanol, 4 ml dry ether is added, ground and kept for 30 minutes at 4˚ C. The residue is separated and recrystallized three times from methanol. Yield is 0,075 g (49,2%). $R_f$ 0,41 (4). Melting temperature 153-155˚C. HPLC under the conditions (6): one peak, retention time 7,77 minutes. $^1$H-NMR spectrum, ($CD_3OD$), δ, ppm : 2,42 (t, 2H, -$CH_2$CONH), 2.55 (t, 2H, HOOC-$CH_2$), 2.8 (t, 2H, β-$CH_2$-HA), 3.4 (t, 2H, α-$CH_2$-HA), 7.0 (s, 1H, CH-5-Im), 8.0 (s, 1H, CH-Im). Mass-spectr; m/z: $[M+1H]^+$ 212.2.

EXAMPLE 2

N-GLUTARYLHISTAMINE

To 0,366 g (3.3 mM) histamine solution in 5 ml DMF, 0,376 g (3.3 mM) glutaric anhydride is added. The reaction mixture is stirred for 3 hours and left for 20 hours at 20˚C. White sediment is separated, dried under vacuum and

recrystallized. Yield is 0,510 g (70,0%). $R_r$ 0.36 (6), 0.34 (4). Melting temperature 187-189°C. HPLC under the conditions . (7): one peak, retention time 14.36 minutes. $^1$H-NMR spectrum, ($D_2O$), δ, ppm : 1,72 (m; 2H, β-$CH_2$), 2.18 (m, 4H, α,γ-$CH_2$), 2.85 (t, 2H, β-$CH_2$-HA), 3.5 (t, 2H, α-$CH_2$HA), 7.25 (s, 1H, CH-5-Im), 8.5 (s, 1H, CH-2-Im). Mass-spectr.; m/z: $[M+1H]^+$ 226.1.

EXAMPLE 3

N-ADIPINYLHISTAMINE CHLOROHYDRATE (IV).

To a solution of 0,197 g (1.35 mM) adipinic acid in 2.5 ml DMF at 0°C, 0.278 g (1.35 mM) DCC is added. The reaction mixture is stirred at 0°C for 30 minutes and a solution of 0.150 g (1.35 mM) histamine in 1 ml DMF is added and then the solution is left at 20°C for 20 hours. DCU sediment is separated by filtration. 10 ml dry ether is added to the reaction mixture and it is then left for 1 hour at 0°C. Oily residue is dissolved in ethanol, and 0.2 ml 4M HCl in dioxane is added. Solvent is removed under vacuum. The residue is washed with ether, dissolved in a 1.5:1 chloroform-ethanol mixture and purified on a column with silicagel 40/100 (22x175 mm). Elution is done with a chloroform-ethanol mixture from 7:3 to 2:8, ethanol, methanol and a 8:1:0,5 methanol-AcOH-$H_2O$ mixture. The fractions containing the target substance with $R_f$ 0,25 (6) are united and the solvent is removed under vacuum and dried over $P_2O_5$. Yield is 0,129 g (40%). $R_f$ 0,25 (6). HPLC under the conditions (11): one peak, retention time 3.6 minutes. $^1$H-NMR spectrum, ($CD_3OD$), δ, ppm : 1.6 (m, 4H, β,γ-$CH_2$), 2.3 (m, 4H, α,γ-$CH_2$), 3.0 (t, 2H, β-$CH_2$-HA), 3.55 (t, 2H, α-$CH_2$-HA), 7.25 (s, 1H, CH-5-Im), 8.65 (s, 1H, CH-Im).

EXAMPLE 4

N-ACETHYL-γ-L-GLUTAMYLHISTAMINE (V)

To 0.10 (0.405 mM) γ-L-glutamylhistamine, 5 ml water is added and stirred until dissolution of the basic substance mass has occured. 2.5 ml dioxane and 0.073 g (0.405 mM) p-nitrophenyl acetate are added to the reaction mixture, which is then stirred for 2 hours and left for 18 hours at 20°C. The solvent is removed under vacuum at 40°C. The residue is dissolved in the minimum amount of methanol and purified using column chromatography on Kieselgel 60, eluted with methanol. The fractions containing the target substance with $R_f$ 0.3 (4) are united and the solvent is removed under vacuum. A colorless glassy substance is produced. Yield is 0.046 g (40.0%). $R_f$ 0.3 (4). HPLC under the conditions (3): one peak, retention time 10.77[1]minutes. $^1$H-NMR spectrum, ($CD_3OD$), δ, ppm : 2.0-2.3 (m, 2H, β,-$CH_2$), 2.19 (s, 3H, $CH_3$CO), 2.45 (t, 2H, γ-$CH_2$), 3.07 (t, 2H, β-$CH_2$-HA), 3.64 (t, 1H, α-$CH_AH_B$-HA), 3.65 (t, 1 H, α-$CH_AH_B$-HA), 4.42 (t, 1H, α-CH), 7.42 (d, 1H, CH-5-Im), 8.69 (d, 1H, CH-2-Im).

EXAMPLE 5

5.1. $N^{\alpha}$-TERT-BUTYLOXYCARBONYL-α-BENZYL-L-GLUTAMYL-L-HISTIDINE METHYL ESTER ($VI^a$)

To a solution of 0,30 g (1.16 mM) L-histidine methyl ester dihydrochloride in 4 ml anhydrous methanol, which has been produced by heating to 40°C with subsequent cooling to 0°C, cold sodium methylate solution produced from 0.053 g metallic sodium (2.32 mM) and 1 ml methanol is added and then the reaction mixture is left for 20 minutes at 0°C. Then an equal volume of dry ether is added and left for 20 minutes at 0°C. Sodium chloride sediment is separated and the solvent is removed from the filtrate under vacuum. The residue is dissolved in 3.5 ml DMF and 0.604 g (1.16 mM) Boc-L-Glu (OPfp) - OBzl. The reaction mixture is stirred for 2 hour and left four 20 hours. The DMF is removed under vacuum. The oily residue is purified on a column 30x1.6 cm with silicagel 100/160; eluted with chloroform:methanol mixture (9:1). The fractions containing the target substance are united and the solvent is removed under vacuum. The target substance is dried over $P_2O_5$. Yield is 0.334 g (55.0%). $R_f$ 0.35 (1). $^1$H-NMR spectrum, ($CD_3OD$) : 1.45 (s, 9H, $(CH_3)_3C$), 2.0 (m, 2H, β-$CH_2$), 2.3 (t, 2H, γ-$CH_2$), 3.0 (d; 2H, β-$CH_2$His), 3.7 (s, 3H, $CH_3O$), 4.1 (t, 1H, α-CH), 4:55 (t, 1H, α-CH-His), 5.15 (m, 2H; $CH_2$-Bzl), 6.85 (s, 1H, CH-5-Im), 7.35 (m, 5H, $C_6H_5$-Bzl), 7.6 (s, 1H, CH-2-Im).

5.2. γ-L-GLUTAMYL-HISTIDINE METHYL ESTER DIHYDROCHLORIDE (VI)

To a solution of 0,30 g Boc-L-Glu (HisOMe) - OBzl ($Vi^a$) in 1 ml MeOH, 3 ml 4M N hydrochloride in dioxane is added. In 30 minutes 5 ml dry ether is added. The solvents are removed under vacuum, then dry ether is added and also removed under vacuum. The residue is ground with dry ether, then the ether is separated by decantation. The resulting white solid substance is dried over $P_2O_5$ under vacuum. 0.25 g 2HCl•L-Glu (HisOMe) - OBzl is produced.

To a solution of 0.140 g of the produced substance in 4.5 ml anhydrous methanol, 0.10 g 10% palladium on

activated carbon is added and the solution is stirred for 2.5 hours with an air flow periodically passed through it. The catalyst is separated by using a filter and washing with MeOH. The solvent is removed from the united filtrate under vacuum. Dry ether is added to the residue and also removed under vacuum. The substance is dried under vacuum over $P_2O_5$- Yield is 0,103 g . (90,3%). $R_f$ 0.35 (6). HPLC under the conditions (9): one peak, retention time 6.16 minutes. $^1$H-NMR spectrum, ($D_2O$), δ, ppm : 2.15 (m, 4H, β-$CH_2$), 2.55 (t, 2H, γ-$CH_2$), 3.15 (t, 1H, α-CH), 3.75 (s, 3H, $CH_3O$), 4.0 (t, 1H, α-CH-His), 4.8 (d, 2H, β-$CH_2$-His), 7.4 (s, 1H, CH-5-Im), 8.81 (s, 1H, CH-2-Im). Mass-spectr; m/z: 299.1.

EXAMPLE 6

N-GLUTARYL--L-HISTIDINE METHYL ESTER (VII)

To a solution of 0,30 g (1.16 mM) L-histidine methyl ester dihydrochloride in 4 ml anhydrous, methanol which has been produced by heating to 40˚C with subsequent cooling to 0˚C, cold sodium methylate solution produced from 0.053 g (2.32 mM) metallic sodium and 1 ml methanol is added. The reaction mixture is left for 30 minutes at 0˚C, then an equal volume of dry ether is added and the reaction mixture is left for 20 hours at 20˚C. Sodium chloride sediment is separated under vacuum. The residue is dissolved in 3.5 ml DMF and 0.132 g (1.16 mM) glutaraldehyde is added. The reaction mixture is stirred for 2 hours and left for 20 minutes at 20˚C. The solvent is removed under vacuum. The oily residue is purified on a column (30x.1.6 cm) with silicagel 100/160, eluted with methanol. The fractions containing the target substance are united. The solvent is removed under vacuum and dried over $P_2O_5$ under vacuum. Yield is 0,095 g (28%). $R_f$ 0.43 (10). HPLC under the conditions (8): one peak, retention time 9.95 minutes. $^1$H-NMR spectrum, ($D_2O$), δ, ppm : 1.85 (m, 2H, β-$CH_2$), 2.25 (t, 4H, α, γ-$CH_2$), 3.05 (d, 2H, β-$CH_2$-His), 3.7 (s, 3H, $CH_3O$), 4.67 (t,1H, α-CH-His), 6.92 (s, 1H, CH-5-Im), 7.72 (s, 1H, CH-2-Im). Mass-spectr; m/z: 284.4.

EXAMPLE 7

7.1. N-BENZYLOXYCARBONYL-γ-AMINOBUTRIC ACID PENTAFLUOROPHENYL ESTER ($VIII^a$)

To 0.60 g (2.53 mM) N-benzyloxycarbonyl-γ-aminobutyric acid, 9 ml anhydrous ethylacetate is added, and the reaction mixture is stirred and cooled to 0˚C. 0.52 g (2.53 mM) DCC and 0.465 (2.53 mM) pentafluorophenol are added. The reaction mixture is stirred for 2 hours, then left for 20 hours at 20˚C. The DCU residue is separated and washed with anhydrous ethylacetate. The filtrates are united and solvent is removed under vacuum. White, slightly yellowish Z-γ-Abu-OPfp crystals are produced, which are dried under vacuum. Yield is 1.05 g (98.0%). $R_f$ 0.85 (1).

7.2. N-(BENZYLOXYCARBONYL-γ-AMINOBUTYRYL)-L-HISTIDINE METHYL ESTER ($VIII^b$)

To 0.30 g (1.16 mM) L-histidine methyl ester dichlorohydrate, 4 ml anhydrous histidine is added and the reaction mixture is heated until dissolution occurs. The solution is cooled to 0˚C and cooled sodium methylate solution, produced from 0.053 g (2.32 mM) metallic sodium and 1 ml anhydrous methanol, is added. The reaction mixture is left at 0˚C for 20 minutes, an equal volume of dry ether is added, and then the mixture is left for 20 minutes at 20˚C. The sodium chloride residue is separated. The solvent is removed from the filtrate under vacuum. To the oily residue, 5 ml anhydrous DMF and 0.50 g (1.24 mM) Z-γ-Abu-OPfp are added, and the mixture is left for 20 hours at 20˚C. The solvent is removed under vacuum. The sodium chloride residue is separated. The solvent is removed from the filtrate under vacuum. To the oily residue, 5 ml DMF and 0.50 g (1.24 mM) Z-γ-Abu-OPfp are added and the mixture is left to 20 hours at 20˚C. The solvent is removed under vacuum. The residue is purified using column chromatography on silicagel L 40/100, eluted with chloroform, wherein there is a methanol gradient in the chloroform to the ratio of 2:8. The fractions containing the target substance with $R_f$ 0,46 (2), are united and the solvent is removed under vacuum. Excess dry ether with 1 drop of triethylamine is added to the produced residue and it is ground. A white solid substance is separated, washed with dry ether and dried under vacuum. Yield is 0,243 g (65.0%). $R_f$ 0,46 (2). Mass-spectrum, m/z: 375.

7.3. γ-AMINOBUTYRYL-L-HISTIDINE METHYL ESTER (VIII).

To 0.04 g (0.124 mM) Z-γ-Abu-L-His-OMe 7 ml anhydrous methanol, 0.04 g 10% palladium on activated carbon is added and the reaction mixture is hydrated for 1 hour while stirring. Following complete conversion of the original substance with $R_f$ 0.46 (2), into the target product with $R_f$ 0 (2), the catalyst is separated washed with methanol. The filtrates are united and the solvent is removed under vacuum. A colorless viscous oil is produced. Yield is 0,030 g (90.0%). $R_f$ 0.05 (3), $R_f$ 0.1 (4). HPLC under the conditions (1): one peak, retention time 4.8 minutes. $^1$H-NMR spectrum, ($D_2O$), δ, ppm : 1.85 (m, 2H, β-$CH_2$), 2.23 (t, 2H, γ-$CH_2$), 2.95 (d, 2H, β-$CH_2$-His), 3.1 (t, 2H, α-$CH_2$), 3.7 (s, 3H, $CH_3O$), 4.65 (t, 1H, α-CH-His), 6.85 (s, 1H, CH-5-Im), 7.6 (s, 1H, CH-2-Im).

EXAMPLE 8

8.1. $N^{\alpha}$-TERT-BUTYLOXYCARBONYL-$\alpha$-BENZYL-L-GLUTAMYL HISTIDINE ($IX^a$)

To a solution of 0.100 g (0.645 mM) L-histidine in 3 ml water, 0.75 ml dioxane is added while stirring and then 0.560 g (1.29 mM) Boc-L-Glu (ONSu) OBzl and 2.25 ml dioxane are added in portions for 2 hours (until the dioxane to water ratio is 1:1). The resulting suspension is stirred for 2 hours and left for 20 hours at 20˚C. Then, the suspension is transformed into solution. The solvent is removed under vacuum. The oily residue is purified on a column 30 x 1.6 cm with silicagel 100/160, eluted with a 5:1 chloroform-methanol mixture, gradually increasing the MeOH content up to 50%. The fractions containing the target substance are united. The solvent is removed under vacuum. A white solid substance is produced. Yield is 0,150 g (45.7%). $R_f$ 0.48 (3). $^1$H-NMR spectrum, ($CD_3OD$), $\delta$, ppm : 1.4 (s, 9H, $(CH_3)_3C$), 1.98 (m, 2H, $\beta$-$CH_2$), 2.3 (t, 2H, $\gamma$-$CH_2$-Glu), 3.1 (m, 2H, $\beta$-$CH_2$-His), 4.07 (t, 1H, $\alpha$-CH), 4.5 (t, 1H, $\alpha$-CH-His), 5.18 (m, 2H, $CH_2$ -Bzl), 7.17 (s, 1H, CH-5-Im), 7.35 (m, 5H, $C_6H_5$), 8.4 (s, 1H, CH-2-Im).

8.2. $\gamma$-L-GLUTAMYL HISTIDINE $\alpha$-METHYL ESTER DICHLOROHYDRATE (IX)

To a solution of 0.140 g Boc-L-Glu (His)-OBzl ($IX^a$) in 1 ml anhydrous methanol, 3 ml 4 N hydrochloride in dioxane is added and the mixture is kept at 20˚C for 40 minutes. Ether is added to the reaction mixture and solvents are removed under vacuum. The sediment is ground with ether until a white solid substance is formed. The ether is decanted and the residue is dried under vacuum and purified with preparative paper chromatography in the butanol-acetic acid-water system 4:1:5, upper phase. 0.12 g (95.8%) dichlorohydrate dipeptide are produced, $R_f$ 0.26 (3). To a solution of 0.117 g of the obtained product in 4.5 ml anhydrous methanol, 0.096 g 10% palladium on activated coal is added. The reaction mixture is stirred for 2.5 hours, with hydrogen flow being periodically passed through it. The catalyst is separated by using a filter and washing with 5 ml MeOH. The filtrates are united and the solvent is removed under vacuum. The residue is dried under vacuum over $P_2O_5$. Yield is 0,091 g (96,1%). $R_f$ 0,3 (6). HPLC under the conditions (10): one peak, retention time 5,40 minutes. Mass-spectr; m/z: 299.2. $^1$H-NMR spectrum, ($CD_3OD$), $\delta$, ppm : 2,15 (m, 2H, $\beta$-$CH_2$), 2.55 (t, 2H, $\gamma$-$CH_2$), 3.16 (t, 1H, $\alpha$-CH), 3.75 (s, 3H, $CH_3O$), 3.99 (t, 1H, $\alpha$-CH-His), 4.8 (dd, 2H, $\beta$-$CH_2$-His), 7.38 (s, H, CH-5-Im), 8. 80 (s, H, CH-2-Im).

EXAMPLE 9

9.1. N-(BENZYLOXYCARBONYL-$\gamma$-AMINOBUTYRYL)TRYPTAMINE ($X^a$)

To a solution of 0.252 g (0.625 mM) Z-$\gamma$-Abu-OPfp in 5 ml DMF, 0.10 g (0.625 mM) tryptamine is added and the reaction mixture is stirred at 25˚C for 2 hours. The reaction mixture is than left for 16 hours at the same temperature, after which a 7-fold excess (by volume) of water is added. A white coagulated sediment is filterred off, washed on a filter with water and dried. Yield is 0.22 g (93.0%). $R_f$ 0.6 (5), $R_f$ 0.54 (3). Mass-spectr, m/z: 380.6

9.2. $\gamma$-AMINOBUTYRYLTRYPTAMINE (X)

To 0.133 g (0.35 mM) Z-$\gamma$-Abu-TrpA, 7 ml anhydrous methanol is added and the mixture is stirred until dissolution of the basic substance mass occurs. To the resulting product, 0.133 g 10% palladium on activated coal is added and the reaction mixture is hydrated for 1 hour. Following complete conversion of the initial substance with $R_f$ 0.6 (5) into the target product with $R_f$ 0 (5), the catalyst is separated and the product is washed with methanol. The filtrates are united and the solvent is removed under vacuum. A colorless viscous oil is produced. Yield is 0,086 g (99.0%). $R_f$ 0,43 (3). HPLC under the conditions (2): one peak, retention time 18.5 minutes. $^1$H-NMR spectrum, ($CD_3OD$), $\delta$, ppm : 1,65 (m, 2H, $\beta$-$CH_2$), 2,05 (t, 2H, $\gamma$-$CH_2$), 2.85 (t, 2H, $\beta$-$CH_2$TrpA), 3.0 (t, 2H, $\alpha$-$CH_2$), 3.45 (m, 2H, $\alpha$-$CH_2$-TrpA), 6,95 (s, 1H, CH-2-Ind), 6.99 (m, 1H, CH-5-Ind), 7.05 (m, 1H, CH-6-Ind), 7.28 (d, 1H, 1H, CH-7-Ind), 7.48 (d, 1H, 1H, CH-4-Ind).

EXAMPLE 10

N-GLUTARYL-O-BENZYLSEROTONIN (XI)

To a suspension of 0.20 g (0.66 mM)O-benzyl serotonin chlorohydrate in 3 ml DMF, 0.09 ml (0.66 mM) triethylamine is added while stirring and then 0.075 g (0.66 mM) glutaranhydride. The reaction mixture is stirred for 1 hour and left for 20 hours at 20˚C. The sediment of triethylamine chlorohydrate is separated and the solvent is removed under vacuum. The oily residue is purified on a column 31 x 1.5 cm with silicagel Silica gel 60, 0.063-0.2 mm (Merck). Elution with a 9:1 chloroform-methanol mixture is done. Fractions containing the target product are united and the solvent is

removed under vacuum. A colorless glassy product is obtained. Yield is 50 mg (20%). $R_f$ 0,48 (1). HPLC under the conditions (14): one peak, retention time 23.8 minutes. $^1$H-NMR spectrum, ($CD_3OD$) δ, ppm : 2.05 (m, 2H, β-$CH_2$-GA), 2,45 (m, 4H, α,γ-$CH_2$-GA), 3.1 (t, 2H, β-$CH_2$), 3.67 (t, 2H, α-$CH_2$), 5.3 (s, 2H, $CH_2$-Bzl), 7.0 (dd, 1H, CH-6-Ind), 7.1-7.7 (m, 8H, CH-7-Ind, CH-2-Ind, CH-4-Ind, $C_6H_5$).

EXAMPLE 11

N-GLUTARYLSEROTONIN (XII)

To 25 mg (0.065 mM) glutaryl-O-benzyl serotonin, 4 ml anhydrous methanol is added. To the resulting solution, 30 mg of a catalyst - palladium on activated coal is added and the reaction mixture is hydrated for 1 hour while stirring. The catalyst is filterred out. The solvent is removed from the filtrate under vacuum. Yield is 17 mg (90%). $R_f$ 0,31 (1). HPLC under the conditions (14): one peak, retention time 16.27 minutes. $^1$H-NMR spectrum, ($CD_3OD$), δ, ppm : 2.0 (m, 2H, β-$CH_2$-GA), 2.5 (m, 4H, α,γ-$CH_2$-GA), 3.0 (t, 2H, β-$CH_2$), 3.65 (t, 2H, α-$CH_2$), 6.85 (dd, 1H, CH-6-Ind), 7.15 (d, 1H, CH-4-Ind), 7.25 (s, 1H, CH-2-Ind) 7.4 (d, 1H, CH-7-Ind).

EXAMPLE 12

N-GLUTARYL-5-O-METHYLSEROTONIN (XIII)

To a solution of 0.20 g (1.05 mM) O-methyl serotonin in 3 ml DMF, 0.12 g (1.05 mM) glutaricanhydride is added while stirring. The reaction mixture is stirred for 1 hour and left for 20 hours at 20˚C. The solvent is removed under vacuum. The oily residue is purified on a column 31 x 1.5 cm with silicagel Silica gel 60, 0.063-0.2 mm (Merck). Elution with a 9:1 chloroform-methanol mixture is done. Fractions containing the target product are united and the solvent is removed under vacuum. A colorless glassy product is obtained. Yield is 0.095 g (29.8%). $R_f$ 0.51 (1). HPLC under the conditions (14): one peak, retention time 19.0 minutes. $^1$H-NMR spectrum, ($CDCl_3$+$CD_3OD$), δ, ppm : 1.95 (m, 2H, β-$CH_2$-GA), 2,37 (m, 4H, α,γ-$CH_2$-GA), 3.0 (t, 2H, β-$CH_2$), 3.56 (t, 2H, α-$CH_2$), 3.92 (s, 3H, $CH_3O$), 6:83 (dd, 1H, CH-6-Ind), 7.1 (d, 2H, CH-2,4-Ind), 7.31 (d, 1H, CH-7-Ind).

Example 13

N-GLUTARYLTRYPTAMINE (XVII)

To a solution of 0.15 g (0.94 mM) tryptamine in 3 ml DMF, 0.107g (0.94 mM) glutaricanhydride is added while stirring. The reaction mixture is stirred for 1 hour and left for 20 hours at 20˚C. The solvent is removed under vacuum. The oily residue is purified on a column 31 x 1.5 cm with silicagel Silica gel 60, 0.063-0.2 mm (Merck). Elution with a 9: 1 chloroform-methanol mixture is done. Fractions containing the target product are united and the solvent is removed under vacuum. A colorless glassy product is obtained. Yield is 0.2 g (78%). $R_f$ 0.41 **(I).** HPLC under the conditions (14): one peak, retention time 18.95 minutes. $^1$H-NMR spectrum, ($CDCl_3$+$CD_3OD$), δ, ppm : 1.90 (m, 2H, β-$CH_2$), 2,27 (dt, 4H, α,γ-$CH_2$), 2.95 (t, 2H, β-$CH_2$-TrPA), 3.50 (t, 2H, α-$CH_2$-TrpA), 7.0 (d, 1H, CH-7-Ind), 7.55 (d, 1H, CH-4-Ind).

EXAMPLE 14

N-GLUTARYL-4-(2-AMINOETHYL)MORPHOLINE (XIV)

To a solution of 0.5 g (4.39 mM) glutaricanhydride in 1 ml DMF, 0.57 ml (4.39 mM) 4-(2-aminoethyl) morpholine is added while stirring and cooling. The reaction mixture is stirred for 30 minutes and left for 20 hours at 20˚C. The solvent is removed under vacuum. The oily residue is washed with ether, the solvent is removed under vacuum, then the residue is dried and left at +4˚C for 20 hours. The formed crystallize substance is washed and ground with ether (3 x 1.5 ml) and then acetone (4x1.5 ml), then separated from the solvents and dried under vacuum. Yield is 0.265 g (24.8%). $R_f$ 0.41 (6). HPLC under the conditions (11): one peak, retention time 3.93 minutes. $^1$H-NMR spectrum, ($CD_3OD$), δ, ppm : 0.5 (m, 2H, β-$CH_2$-GA), 0.9 (m, 4H, α,γ-$CH_2$-GA), 1.3 (m, 8H, $CH_2$-2,3,5,6-morpholinyl), 2.0 (t, 2H, β-$CH_2$), 2.38 (t, 2H, α-$CH_2$).

EXAMPLE 15

N-GLUTARYL-2-(2-AMINOETHYL)PYRIDINE (XV)

To a solution of 0.3 g (2.63 mM) glutaricanhydride in 1 ml DMF, 0.315 ml (2.63 mM) 2-(2-aminoethyl) pyridine is added while stirring and cooling with water. The reaction mixture is stirred for 1 hour and left for 20 hours at 20˚C. The white sediment is filterred off, washed many times with ether and acetone, and dried. Yield is 0.29 g (46.7%). $R_f$ 0.27 (1). HPLC under the conditions (12): one peak, retention time 14.33 minutes. $^1$H-NMR spectrum, ($CD_3OD$), δ, ppm: 0.41 (m, 2H, β-$CH_2$-GA), 0.81 (m, 4H, α,γ-$cH_2$-GA), 1.6 (t, 2H, β-$CH_2$), 2.15 (t, 2H, α-$CH_2$), 5.9 (m, 2H, CH-4,5-pyridinyl), 6.4 (m, 1H, CH-3-pyridinyl), 7.05 (m, 1H, CH-6-Py).

EXAMPLE 16

N-GLUTARYLPHENYLETHYLAMINE (XVI)

To a solution of 0.5 g (4.39 mM) glutaricanhydride in 1 ml DMF, 0.55 ml (4.39 mM) phenylethylamine is added while stirring and cooling with water. The reaction mixture is stirred for 30 minutes, then left for 20 hours at 20˚C. The solvent is removed under vacuum and the residue is purified on a column 25 x 230 mm with Kieselgel 40 for column chromatography ("Fluka"). Elution with a chloroform: -chloroform: MeOH (9:1) mixture is done. Fractions containing the target product with $R_f$ 0.57 (1) are united, and dried following solvent removal under vacuum. Yield is 1.02 g (99%). HPLC under the conditions (13): one peak, retention time 15.12 minutes. $^1$H-NMR spectrum, ($CD_3OD$), δ, ppm: 1.9(m, 2H, β-$CH_2$-GA), 2.15 (m, 4H, α,γ-$CH_2$-GA), 2.8 (t, 4H, α,γ-$CH_2$-GA), 7.2 (m, 5H, $C_6H_5$).

EXAMPLE 17

CYCLOHEXYLCARBONYLHISTAMINE (XXIX)

To 700 mg (3.85 mM) histamine dichlorohydrate, 9 ml anhydrous methanol is added and the mixture is heated up to 50˚C and stirred until dissolution occurs, then cooled to 0˚C. To the resulting solution 1.42 ml (7.7 mM) sodium methylate solution in methanol is added. The mixture is kept at 0˚C for 20 minutes and then an equal volume of anhydrous ether is added and the mixture kept for 20 minutes at 0˚C. The NaCl sediment is separated. The solvent is removed from the filtrate under vacuum. The oily residue is dissolved in 5 ml DMF and cooled to +5˚C 0.537 ml (3.85 mM) triethylamine is added to the solution while stirring and then 0.52 ml (3.85 mM) cyclohexane carbonic acid chloroanhydride is added. The reaction mixture is stirred for 1 hour and then the white sediment is separated. The solvent is removed from the filtrate under vacuum. The product is crystallized from 2 ml acetone with adding triethylamine. The sediment is separated, washed with 2 ml of ether and acetone (1:1) mixture and 2 ml methanol with acetone (1:1) with the addition of triethylamine. $R_f$ 0.57 (7). Yield is 184 mg (52%). HPLC under the conditions (1): one peak, retention time 18.05 minutes. $^1$H-NMR spectrum, ($CD_3OD$), δ, ppm: 1.36-1.78 (m, 10H, $(CH_2)_5$ - cyclohexane), 2.18 (m, 1H, >CH-CO-), 2.8 (t, 2H, β-$CH_2$-HA), 3.41 (t, 2H, α-$CH_2$-HA), 6.85 (s, 1H, 5-CH-Im), 7.6 (s, 1H, 2-CH-Im).

EXAMPLE 18

N-CYCLOHEXYLCARBONYLTRYPTAMINE (XXX)

To a solution of 0,20 g (1.25 mM) tryptamine in 2.5 ml dimethyl formamide, 0.17 ml (1.25 mM) triethylamine and 0.17 ml (1.25 mM) cyclohexanoyl chloride are added while vigorously stirring. The reaction mixture is stirred for 1.5 hours in darkness and then 10 ml 5% $Na_2CO_3$ solution is added and the mixture is stirred for an additional 30 minutes. The obtained emulsion is extracted with 2 x 15 ml ethylacetate. The ethylacetate extracts are united, washed with 30 ml water and dried over anhydrous $Na_2SO_4$. The solvent is removed under vacuum. The oily residue is purified on a column with silicagel Kieselgel 60 with particle size 0.035-0.07 mm (Fluka company) with elution by gradient of solvents chloroform-chloroform:methanol (9:1). The fractions containing the target product are united and the solvent is removed under vacuum. Yield is 0.068 g (20.3%). $R_f$ 0.63 (1). HPLC under the conditions (15): one peak, retention time 32.7 minutes. $^1$H-NMR spectrum, ($CDCl_3$), δ, ppm: 1.1-1.85 (m, 10H, $(CH_2)_5$-cyclohexane), 2.0 (m, 1H, CH-), 2.97 (t, 2H, β-$CH_2$-TA), 3.6 (t, 2H, α-$CH_2$-TA), 7.0 (s, 1H, 2-CH-Ind), 7.1 (t, 1H, 5-CH-Ind), 7.2 (t, 1H, 6-CH-Ind), 7.4 (d, 1H, 4-CH-Ind), 7.6 (d, 1H, 7-CH-Ind).

EXAMPLE 19 (COMPARATIVE EXAMPLE)

N-HEXANOYLTRYPTAMINE (XXXI)

To a solution of 0,20 g (1.25 mM) tryptamine in 2.5 ml dimethyl formamide, 0.18 ml (1.25 mM) triethylamine and 0.17 ml (1.25 mM) hexanoyl chloride are added while vigorously stirring. The reaction mixture is stirred for 1.5 hours in darkness and then 10 ml 5% $Na_2CO_3$ solution is added and the stirring continued for an additional 30 minutes. White sediment is separated and washed on a filter with 2 x 4 ml water, 2 x 4 ml hydrochloric acid and 3 x 4 ml water and then dried under vacuum over $P_2O_5$. The obtained product is then purified on a column with silicagel Kieselgel 60 with particle size 0.063-0.2 mm (Merck company) with elution by a gradient of solvents chloroform-chloroform:methanol (9:1). The fractions containing the target product are united and the solvent is removed under vacuum. Yield is 0.108 g (33.4%). $R_f$ 0.54 (1). HPLC under the conditions (15): one peak, retention time 33.13 minutes. $^1$H-NMR spectrum, ($CDCl_3$), δ, ppm: 1.9 (t, 3H, $CH_3$ -), 1.25 (m, 4H, $(CH_2)_2$-hexanoyl), 1.6 (m, 2H, β-$CH_2$- hexanoyl), 2.15 (t, 2H, α-$CH_2$-hexanoyl ), 3.0 (t, 2H, β-$CH_2$-hexanoyl ), 3.6 (t, 2H, α-$CH_2$-TA), 7.0 (s, 1H, 2-CH-Ind), 7.1 (t, 1H, 5-CH-Ind), 7.2 (t, 1H, 6-CH-Ind), 7.4 (d, 1H, 7-CH-Ind), 7.6 (d, 1H, 4-CH-Ind).

EXAMPLE 20

N-HEXANOYL-L-TRYPTOPHANE METHYL ESTER (XXXII)

To a solution of 0,40 g (1.57 mM) HCl•H-Trp-Ome in 2.5 ml dimethyl formamide, 0.44 ml (3.14 mM) triethylamine and 0.216 ml (1.57 mM) hexanoyl chloride are added while stirring. After cooling the reaction mixture with ice 2,5 hours 20 ml 5% $Na_2CO_3$ solution is added and the mixture is stirred for 10 minutes. The product is extracted with 2 x 25 ml ethyl acetate. The united ethyl acetate extracts are washed with an equal volume of water and dried under anhydrous $Na_2SO_4$. The solvent is removed under vacuum. The oily residue is purified on a column with silicagel Kieselgel 60 with particle size 0.063-0.2 mm (Fluka company), with elution by a gradient of solvents chloroform-chloroform:methanol (9,5: 0,5). The fractions containing the target product are united and the solvent is removed under vacuum. Yield is 0.138 g (27.7%). $R_f$ 0.79 (1). HPLC under the conditions (16): one peak, retention time 6.92 minutes. $^1$H-NMR spectrum, $CDCl_3$), δ, ppm: 0.9 (t, 3H, $CH_3$ -), 1.24 (m, 4H, γ,δ, -$CH_2$ -hexanoyl), 1.57 (m, 2H, β-$CH_2$-), 2.13 (t, 2H, α-$CH_2$-), 3.31 (d, 2H, β-$CH_2$-Trp), 3.7 (s, 3H, $CH_3$O-), 4.96 (t, 1H, α-CH-Trp), 7.0 (s, 1H,2-CH-Ind),7.12 (m, 2H, 5-CH,6-CH-Ind), 7.33-7.53 (dd, 1H, 4,7-CH-Ind).

EXAMPLE 21

N-(IMIDAZOLYL-4-ACETHYL)-HEPTHYLAMINE(XXXIII)

To a solution of 0.30 g (1.84 mM) imidazolacetic acid chlorohydrate in 2.5 ml DMF, 0.248 g (1.84 mM) 1-oxybenzotriazole and 0.38 g (1.84 mM) dicyclohexylcarbodiimide are added while stirring and cooling with ice. The reaction mixture is then stirred for 1 hour and left for 20 hours at +4˚C. The dicyclohexylurea sediment is separated and the solvent is removed from the filtrate under vacuum. The fractions containing the target product are united, and the solvent is removed under vacuum. Yield is 0.12 g (29%). $R_f$ 0.39 (1). HPLC under the conditions (15): retention time of individual substance is 28.01 minutes. $^1$H-NMR spectrum, (CDCl3), δ, ppm: 0.9 (t, 3H, $CH_3$-), 1.3 (m, 10H, -$(CH2)_5$-), 1.8 (t, 2H, α-$CH_2$-), 3.0 (s, 2H,-$CH_2$-), 7.6 (s, 1H, 5-CH-Im), 8.2 (s, 1H, 2-CH-Im). Mass-spectrum: $[M+1H]^+$, m/z:224.

EXAMPLE 22

22.1 $N^{\alpha}$-TERT.-BUTYLOXYCARBONYL-β-ALANINE BENZYL ESTER

A solution of 0.8 g (5.29 mM) Boc-β-Ala in 4 ml ethanol is added to a solution of 1.38 g (5.29 mM) cesium carbonate in water. The solvent is removed under vacuum. The oily residue is dried under vacuum until 1.45 g glassy residue of cesium Boc-β-Ala salt is formed, which then is dissolved in 10 ml DMF. 0.538 ml (4.53 mM) benzyl bromide are to the solution added while stirring and cooling, and then the mixture is stirred for a further 1,5 hours. The cesium bromide sediment is separated and the solvent is removed from the filtrate under vacuum. The oily residue is purified with column chromatography on silicagel with elution by chloroform. 0.70 g (55.0%) Boc-β-Ala benzyl ester are produced. $R_f$ 0.27 (9).

22.2 N -BENZYLOXYCARBONYIL-HSTIDYL-β-ALANINE BENZYL ESTER

To 60 g (2.15 mM) Boc-β-Ala-OBzl, 3.5 ml 4N HCl solution in dixane is added and the mixture is for 40 minutes. Then 3x5 ml anhydrous ether is added and the solvent is removed under vacuum. The oily residue is dissolved in 3,5 ml DMF, and triethylamine is added up to pH 8. 0.622 g (2.15 mM) Z-L-His and 1.63 g (2.15 mM) "F complex" are added while cooling with ice and stirring. The reaction mixture is then stirred at 0˚C for a further 1 hour and then left for 20 hours at +4˚C. The dicyclohexylurea sediment is separated and the solvent is removed from the filtrate under vacuum. The oily residue is dissolved in acetone and triethylamine is added up to pH 9. The solvent is removed under vacuum and the residue is purified on a column with silicagel in a gradient of solvents chloroform-chloroform: methanol (9,5: 0<5). 0.170 g of white solid substance is produced. $R_f$ 0.44(1).

22.3 N-L-HISTIDYL-β-ALANINE (XXXVI)

To a solution of 0.14 g Z-L-His-β-Ala-OBzl in 3 ml methanol, 0.1 g 10% palladium on coal is added and the reaction mixture is stirred for 1,5 hours, with hydrogen flow being periodically passed through it. The catalyst is separated, the solvent is removed under vacuum, and the residue is then dried. Yield is 0.085 g (98%). $R_f$ 0.69 (10). HPLC under the conditions (15): retention time of individual substance is 3.55 minutes.

EXAMPLE 23

23.1. ASPARTYL HISTAMINE $N^{\alpha}$ -TERT.-BUTYLOXYCARBONYL-β-BENZYL ESTER

To a solution of 1.7 g (3.48 mM) Boc-Asp(OBzl)-OPfp, 5 ml DMF is added while stirring. The reaction mixture is then stirred for a further 1,5 hours and then left for 20 hours at +4˚C. The solvent is removed under vacuum. The oily residue is dissolved in 2 ml chloroform, and triethylamine is added up to pH 8 and then the residue is purified on a column 30 x 1,5 cm with silicagel 40/100 in a gradient of solvents chloroform-chloroform:methanol (9:1). Fractions containing the target product are united and the solvent is evaporated. A colorless substance is produced. Yield is 800 mg (55%), $R_f$ 0.29(1).

23.2.$N^{\alpha}$-TERT.-BUTYLOXYCARBONYL- ASPARTYLIESTAMINE

85 mg Boc-Asp (OBzl)HA is dissolved in 2 ml methanol, then 80 mg Pd/C is added and the reaction mixture is stirred for 1,5 hours, with hydrogen flow being periodically passed through it. The catalyst is filtered off, and the solvent in the filtrate is evaporated. 65 mg (95%) clear oil is produced. $R_f$ 0.35 (3).

23:3. α-L-ASPARTYLHISTAMINE (XXXVIII)

A solution of 65 mg Boc-Asp-HA in 2 ml 4N HCl in dioxane is kept for 40 minutes. The solvent is removed under vacuum. The residue is washed with dry ether, then the solvent is removed under vacuum. Yield is 50 mg (83%) 2HCl Asp-HA. $R_f$ 0.57 (10). $^1$H-NMR spectrum, ($CDCl_3$+$CD_3OD$), δ, ppm: 2.0 (d, 2H, β-$CH_2$-Asp), 2.9 (t, 2H, β-$CH_2$-HA), 3.5 (t, 2H, α-$CH_2$-HA), 4.2 (t, 1H, α-$CH_2$-Asp), 7.0 (s, 1H, 5-CH-Im), 8.15 (s, 1H, 2-CH-Im). Mass-spectrum:, m/z: $[M+1H]^+$ 227.2.

According to typical techniques, novel compounds of general formula (I) shown in table 2, are also produced.

Table 2

| Structure and characteristics of compounds of general formula (I) | | | | | | |
|---|---|---|---|---|---|---|
| N | $R_1$ | n | $R_2$ | m | $R_3$ | Physicochemical characteristics |
| Q | 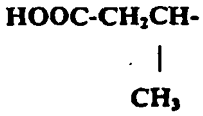 | 1 | H | 1 | -4-Im | $^1$H-NMR spectrum: 1.5 (d, 3H, $CH_3$), 1.75 (m, 1H, β-H), 2.2 (m, 4H, α, γ-$CH_2$), 2.8 (t, 2H, β-$CH_2$-HA), 3.4 (T, 2H, α-$CH_2$-HA), 7.3 (s, 1H, CH-5-Im), 8.6 (s, 1H, CH-2-Im). Mass-spectrum, m/z: $[M+1H]^+$ 240.2. |
| | 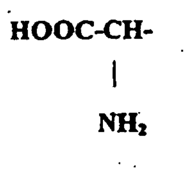 | 2 | $CH_3$ OOC | 1 | -3-Ind | $^1$H-NMR spectrum: 2.12 (m, 2H, β-$CH_2$), 2.43 (t, 2H, γ-$CH_2$), 3.12 (t, 2H, β-$CH_2$ Trp), 3.7 (s, 3H, $CH_3O$), 3.85 (t, 1H, α-CH), 3.92 (t, 1H, α-CH-Trp), 6.99 (t, 1H, 5-CH-Ind), 7.05 (s, 1H, 2-CH-Ind), 7.06 (t, 1H, 6-CH-Ind), 7.55 (d, 2H, 4,7-CH-Ind). Mass-spectrum, m/z: $[M+1H]^+$ 348.3. |
| | **$NH_2$-$CH_2$-** | 1 | H | 1 | -3-Ind | $^1$H-NMR spectrum: 2.35 (dt, 4H, α,β-$CH_2$), 3.05 (t, 2H, β-$CH_2$-TrpA), 3.63 (t, 2H, α-$CH_2$-TrpA), 7.07 (m, 3H, 2-CH-2,5,6-Ind), 7.43 (d, 1H, CH-7-Ind), 7.43 (d, 1H, CH-7-Ind), 7.65 (d, 1H, CH-4-Ind). Mass-spectrum, m/z: $[M+1H]^+$ 232.5. |
| XVIII | **HOOC- $CH_2$-** | 2 | H | 1 | 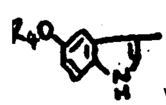 where $R_4$ =$CH_3CO$- | $^1$H-NMR spectrum,: 1.98 (m, 2H, β-$CH_2$ -GA), 2.25 (s, 3H, $CH_3CO$), 2.45 (m, 4H, α, γ-$CH_2$-GA), 3.13 (t, 2H, β-$CH_2$), 3.60 (t, 2H, α-$CH_2$), 6.91 (dd, 1H, CH-6-Ind), 7.15 (d, 2H, CH-2,4-Ind), 7.36 (d, 1H, CH-7-Ind). Mass-spectrum, m/z: $[M+1H]^+$ 333.3 |

(continued)

| Structure and characteristics of compounds of general formula (I) | | | | | | |
|---|---|---|---|---|---|---|
| N | $R_1$ | n | $R_2$ | m | $R_3$ | Physicochemical characteristics |
| XXII | **HOOC-CH$_2$-** | 1 | H | 1 | 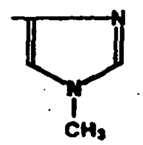 | HPLC under the conditions (6): one peak, retention time 8,9 minutes. Mass-spectrum, m/z: $[M+IH]^+$ 226.2 |
| XXIII | **HOOC-CH$_2$-** | 2 | $CH_3OOC$- | 1 | 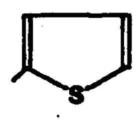 | Mass-spectnum:, M/z: $[M]^+$ 299.2 Calculated %: S 10.69. Found %: 10.85. |
| XXIV | **HOOC-CH$_2$-** | 2 | H | 1 | 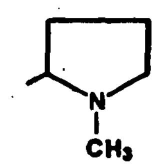 | $^1$H-NMR spectrum: 0.62 (m, 2H, β-$CH_2$-GA), 0.95 (m, 4H, α,γ-$CH_2$-GA), 1.15 (d, 3H, $CH_3$), 1.75 (m, 4H, $CH_2$-3,4-pyrrolidinyl), 2.5 (s, 3H, $CH_3$-), 2.74 (t, 2H, β-$CH_2$), 3.05 (t, 2H,α-$CH_2$), 3.21 (t, 2H, $CH_2$-5-pyrrolidinyl), 3.34 (m, 1H, CH-2-pyaolidinyl) Mass-spectrum, m/z: $[M]^+$ 242.3. |
| XXV | **HOOC-CH$_2$-** | 2 | H | 1 | 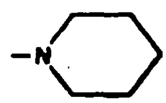 | $^1$H-NMR spectrum: 0.5-65 (m, 2H, β-$CH_2$-GA; $CH_2$-3,4,5-piperazinyl), 0.9 (m, 4H, α,γ-$CH_2$-GA), 1.25 (m, 4H, $CH_2$-2,6-piperazinyl); 2.42 (t, 2H, α-$CH_2$). Mass-spectrum, m/z: $[M]^+$ 242.3. |
| XXVI | **HOOC-CH$_2$-** | 2 | H | 1 | 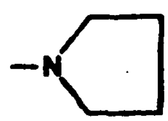 | $^1$H-NMR spectrum: 0.6 (m, 2H,β-$CH_2$-GA), 0.8-0.9 (m, 4H, $CH_2$-3,4-pyrrolidinyl), 1.05 (m, 4H, α,γ- $CH_2$-GA), 1.35 (m, 4H, CH,2,6-pyrrolidinyl), 2.05 (t, 2H, β-$CH_2$),2.4(t,2H, α-$CH_2$). Mass-spectrum, m/z: $[M]^+$ 228.2. |
| XXVII | **HOOC-CH$_2$-** | 1 | H | 2 | 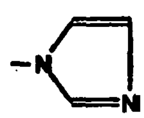 | HPLC under the conditions (7): one peak, retention, time 13,8 minutes. Mass-spectrum, m/z: $[M+1H]^+$ . 240.2 |

(continued)

| Structure and characteristics of compounds of general formula (I) | | | | | | |
|---|---|---|---|---|---|---|
| N | $R_1$ | n | $R_2$ | m | $R_3$ | Physicochemical characteristics |
| XXVIII | **$C_2H_5OCO-CH_2-$** | 0 | H | 1 | -4-Im | $^1$H-NMR spectrum,: 1.77 (m, 2H, β-$CH_2$), 2.22(m, 4H, α,γ-$CH_2$), 2.9 (t, 2H, β-$CH_2$-HA), 3.49 (t, 2H, α-$CH_2$ HA), 3:6 (dd, 2H, -$CH_2$-O-CO), 3.75 (t, 3H, $CH_3$-$CH_2$-), 7.26 (s, 1H, CH-5-Im), 8.6 (s; 1H, CH-2-Im). Mass-spectrum, m/z: $[M+1]^+$ 226.2. |
| XXXVII | **$CH_3$-CONH-$CH_2$-** | 2 | H | 1. | -4-Im | $^1$H-NMR spectrum,: 1.91 (m, 2H, β-CH2), 2.18 (s, 3H, $CH_3$CO), 2,36 (t, 2H, γ-$CH_2$), 2.97 (t, 2H, β-$CH_2$-HA), 3.32 (t, 2H, α-$CH_2$-HA), 3.53 (t, 2H, α-$CH_2$), 7.32 (s, 1H, CH-5-Im), 8.66 (s, 1H, CH-2-Im). Mass-spectrum, m/z: $[M+1H]^+$ 239.1. |
| XXXVIII | 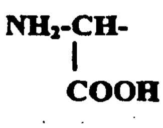 | 1 | H | 1 | -4-Im | $^1$H-NMR spectrum: 1.8 (d, 2H, β-$CH_2$-Asp), 2.72 (t, 2H, β-$CH_2$-RA), 3.31 (t, 2H, α-$CH_2$-HA), Asp), -6.9 (s, 1H, CH-5-Im), 7.95 (s, 1H, CH-2-Im). Mass-spectrum, m/z: $[M+1H]^+$ 227.2. |
| XXXIV | **-$CH_2$-3-Ind** | 0 | H | 2 | -($NH_2$)CH-COOH | $^1$H-NMR spectrum: 1.85 (m, 4H, β,γ-$CH_2$), 2.1 (t, 2H, δ-$CH_2$), 3.1 (s, 2H, -$CH_2$-), 3.45 (t, 2H, α-CH), 6.95 (s, 1H, CH-2-Ind), 7.0-7.05 (m, 2H, CH-5,6-Ind), 7.4-7.45 (dd, 2H, CH-4,7-Ind). Mass-spectrum, m/z: $[M+1H]^+$ 290.2. |

(continued)

| Structure and characteristics of compounds of general formula (I) | | | | | | |
|---|---|---|---|---|---|---|
| N | $R_1$ | n | $R_2$ | m | $R_3$ | Physicochemical characteristics |
| XXXV | **$-CH_2$-3-Ind** | 1 | H | 1 | $-CH_2-NH_2$ | $^1$H-NMR spectrum: 1.7 (m, 2H, β-$CH_2$), 2.1 (t, 2H, γ-$CH_2$), 2,9 (t, 2H, β-$CH_2$-indolyl-pmpion.), 3.05 (t, 2H, α-$CH_2$), 3.5 (m, 2H, α-$CH_2$-indolyl-propion.), 7.0 (s, 1H, CH-2-Ind), 7.05 (m, 1H, CH-5-Ind), 7.1 (m, 1H, CH-6-Ind), 7.32 (d, 1H, CH-7-Ind), 7.5 (d, 1H, CH-4-Ind). Mass-spectrum, m/z: $[M+IH]^+$ 245.2. |
| XL6 | 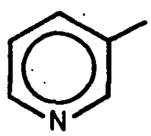 | 0 | H | 1 | -4-Im | $^1$H-NMR spectrum: 2.4 (t, 2H, β-$CH_2$HA), 3.3 (t, 2H, α-$CH_2$HA), 7.1-7.15 (m, 4H, 2,4,5,6-CH), 7.3 (s, 1H, CH-5-Im), 8.65 (s, 1H, CH-2-Im). Mass-spectrum, m/z: $[M+1H]^+$ 217.1. |
| XLI | 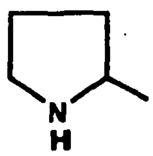 | 0 | H | 1 | -3-Ind | $^1$H-NMR spectrum: 2.01 (m, 2H, 4-$CH_2$), 2.3 (m, 2H, 3-$CH_2$-), 2.42 (t, 2H, β-$CH_2$TrpA), 3.4 (t, 2H, 5-$CH_2$-), 3,4 (t, 2H, 5-$CH_2$-), 3.46 (t, 2H, α-$CH_2$-TrpA), 4.1 (m, 1H, 2-CH), 6.95 (m, 3H, 2,5,6-CH-Ind), 3.5 (m, 2H, α-$CH_2$ -indolyl-propion.), 7.0 (s, 1H, CH-2-Ind), 7.05 (m, 1H, CH-5-Ind), 7.6 (dd, 2H, 4,7-CH-Ind). Mass-spectrum, m/z: $[M+1H]^+$ 257.2. |
| XLII | 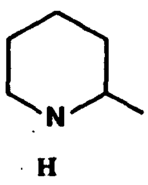 | 0 | H | 1 | -4-Im | $^1$H-NMR spectrum: 2.1-2.25 (m, 6H, 3,4,5-$CH_2$-), 2.4 (t, 2H, β-$CH_2$-), 2.4 (t, 2H, β-$CH_2$-HA), 3.45 (t, 2H, 6-$CH_2$-), 3,48 (t, 2H, α-$CH_2$-HA), 4.15 (t, 1H, 2-CH), 7.2 (s, 1H, CH-5-Im), 8.5 (s, 1H, CH-2-Im). Mass-spectrum, m/z: $[M+1H]^+$ 223.2.. |

(continued)

| Structure and characteristics of compounds of general formula (I) | | | | | | |
|---|---|---|---|---|---|---|
| N | $R_1$ | n | $R_2$ | m | $R_3$ | Physicochemical characteristics |
| XLIII | 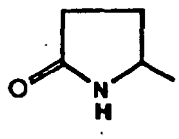 | 0 | H | 1 | -3-Ind | $^1$H-NMR spectrum: 2.1 (m, 2H, (β-$H_2$); 2.5 (t, 2H, β-$CH_2$ TrpA), 2.93 (t, 2H, γ-$CH_2$), 3.45 (t, 2H, α-$CH_2$-TrpA), 4.25 (t, 1H, α-CH), 3.46 (t, 2H, α-$CH_2$-TrpA), 7.03 (m, 1H, CH-5-Ind), 7.09 (s, 1H, CH-2-Ind), 7.1 (m, 1H, CH-6-Ind), 7.6 (d, 1H, CH-7-Ind), 7.65 (d, 1H CH-4-Ind). Mass-spectrum, m/z: $[M+IH]^+$ 271.2. |

Examples of biological trials with compounds of the present invention are presented, below.

All experimental results were statistically processed [Gubler E.V. Computer analysis methods and recognition of pathological processes, Moscow, Nauka publishers (1978), 365 pp.].

EXAMPLE 24

ANTIHYPOXIC ACTIVITY STUDY OF COMPOUNDS OF THE PRESENT INVENTION

Tests for determining the average lifetime of animals in a closed volume and the model of acute hypobaric hypoxia at the minimum atmospheric rarefaction (170-185 mm Hg) were used to study the antihypoxic activity of the compunds of general formula (I).

A. Determining average life-span of animals in a closed volume

Mice were placed one by one into sealed glass jars and their lifetime was recorded.

The experiment was carried out on 90 mongrel male mice having an initial body mass of 20 ± 0,5 g, housed in cages of 10 animals and given a standard vivarium ration. The average lifetime of a mouse in a closed vessel having a volume of 250 ml was preliminarily determined and it was 22 to 25 minutes. All the groups had 10 animals each.

Experimental protocol:

a)-preliminary hypoxia for 12 minutes;
b)- first administration of the compound immediately following preliminary hypoxia;
c)- second administration of the compound 24 hours after the first administration, then daily administration for three days;
d)- recording the lifetime of animals under hypoxic conditions in a sealed space, 2 hours after the last administration of the compound.

The test compounds were dissolved in distilled water and orally administered to animals for 5 days.

Experimental groups:

Group 1 -(control)- without preliminary hypoxia, with oral administration of distilled water for 5 days;

Groups 2 and 3 - administration of compound XLIV using doses of 50 and 500 μg, respectively;
Group 4 - oral administration of sodium oxybutyrate at a dose 300 mg/kg 2 hours before hypoxia;
Group 5 -(control)- with preliminary hypoxia for 12 minutes with subsequent administration of distilled water for 5 days;
Groups 6 and 7 - administration of compound XLV using doses of 50 and 500 μg, respectively;
Groups 8 and 9 - administration of compound III using doses of 50 and 500 μg, respectively.

The data presented in Table 3 give evidence of the fact that the compounds of the present invention possess a pronounced antihypoxic activity, significantly increasing the lifetime of Experimental animals by 20-50 percent It should be noted that the tested compounds rased the lifetime of mice in a way comparable with the effect of the reference drug (sodium oxybutyrate) when administered at doses that are lower by three-four orders.

Table 3

| Effect of test compounds on lifetime of mice under hypoxic conditions | | |
|---|---|---|
| Groups of animals | Drug dose (mg/kg) | Lifetime (seconds) |
| Group 1 - without preliminary hypoxia, | - | 1440,3±86,2 |
| Group 2 - compound XLN | 0,05 | 2110,6±123,6**[1] |
| Group 3 - compound XLN | 0,50 | 1742,8±68,1*[1] |
| Group 4 - sodium oxybutyrate | 300 | 2103,9±100,5**[1] |
| Group 5 - control with hypoxia | - | 1437,2±87,3 |
| Group 6 - compound XLIV | 0,05 | 1415,5±47,2 |
| Group 7-compound XLIV | 0,50 | 1844,8±93,2*[5] |
| Group 8 - compound III | 0,05 | 1670,6±25,3*[5] |
| Group 9 - compound III | 0,50 | 1532,6±96,0 |
| *- significant difference relative to the respective control group (figures 1 and 5); * = p<0,05; * = p<0,01. | | |

B. Acute hypobaric hypoxia model (AHBH)

Stimulation of AHBH was done in a flow-through chamber at a temperature of 22°C. Experiments were carried out on mongrel mice with an initial body mass of 20 g, with a minimum rarefaction of 170-185 mm Hg, which corresponds to an altitude of 10500-11000 m. The lift rate was 35 m/second. Each group included 15 animals. Compounds were administered orally and the lift to altitude was performed 2 hours after the last administration.

Groups of animals:

Group 1 - intact control -administration of normal saline four times.
Groups 2 and 3 - administration of compound III at doses of 50 and 500 μg/kg ,respectively, for 4 days.
Group 4 - single administration of sodium oxybutyrate at a dose 300 μg/kg.

Results are statistically processed.

The results presented in table 4 give evidence of the fact that administration of the compound at a dose 500 μg/kg increases the lifetime of animals under hypoxic conditions.

Table 4

| Effect of the compounds on lifetime of mice under hypoxic conditions | | | |
|---|---|---|---|
| Group of animals | Dose in μg/kg | Lifetime under hypoxic conditions (minutes) | Effect in percent |
| Group 1 (control) | - | 306,0 ± 9,8 | 100 ± 3,2 |
| Group 2 | 50 | 322,0 ± 10,9 | 105 ± 3,5 |

(continued)

| Effect of the compounds on lifetime of mice under hypoxic conditions | | | |
|---|---|---|---|
| Group of animals | Dose in μg/kg | Lifetime under hypoxic conditions (minutes) | Effect in percent |
| Group 3 | 500 | 345,3 ± 15,9 | 113 ± 5,2* |
| Group 4 | 300,000 | 364,3 ± 13,5 | 119 ± 4,4* |
| *= significant difference relative to the control group; *=p<0,05. | | | |

EXAMPLE 25

ANTIISCHEMIC ACTIVITY OF COMPOUNDS

The antiischemic activity of compounds was assessed by an integral test, i.e. the effect on the necrosis zonearea following the reproduction of myocardial infarction in mongrel rats weighing 250-300 g.

The effect of a compound on the necrosis zone area was studied 4 hours after arterial occlusion in rats, using a differential indicator method [Sernov L.N., Gatsura V.V., A differential indicator method for determinig ischemic and necrotic zone in experimental myocardial infarction in rats, Bulletin of Experimental Biology and Medicine (1989), No 5, pp. 534-535]. All the painful manipulations were performed on animals under Ethaminal anesthesia (Ethaminal - 40 mg/kg intraperitoneally). The experiments were carried out 15 hours after the last compound administration. Each group included 8 animals.

Groups of animals:

Group 1 - intact control -administration of normal saline four times.
Groups 2 and 3 - oral administration of compound III at dose of 50 and 500 μg/kg ,respectively, for 4 days.
Group 4 - single intraperitoneal administration of Nicorandil at a dose of 1 mg/kg immediately after occlusion.

The data presented in Table 5 give evidence of the fact that the compounds possess antiischemic activity.

Table 5

| The compound effect on the necrosis zone area 4 hours after coronary artery occlusion in rats | | | | |
|---|---|---|---|---|
| Group of animals | Dose, μg/kg | Ischemia zone, percent to the total myocardial mass | Necrosis zone, percent to the total myocardial mass | Necrosis zone percent to myocardial zone |
| Group 1 | - | 25,5 ± 1,7 | 17,1 ± 1,2 | 67,6 ± 4,0 |
| Group 2 | 50 | 27,2 ± 2,8 | 15,4 ± 1,7 | 56,8 ± 2,7* |
| Group 3 | 500 | 31,1 ± 3,1 | 17,8 ± 1,0 | 54,3 ± 2,1* |
| Group 4 | 1000 | 25,0 ± 1,9 | 11,3 ± 1,6 | 44,3 ± 5,0* |
| *= significant difference relative to the control group; *=p<0,05. | | | | |

EXAMPLE 26

CHANGE IN FOOD ANAPHYLAXIS SEVERETY AS AFFECTED BY THE COMPOUNDS OF THE PRESENT INVENTION

Tests were carried out on mongrel guinea-pigs with an initial body mass of 250-300 hg, which were given a general vivarium ration. Sensibilization of animals was performed according to the published method [Shaternikov V.A., Marokko I.N., Pyatnitsky N.N.; Shirina L.I., Gorgoshidze L.Sh., Kasyanenko V.V., Sugonyaeva N.P., Zhminchenko V.M.,

Vinokurova N.M., Experimental reproduction of food anaphylaxis, Voprosy pitaniya (Nutrition problems)(1982) No 2, pp. 27-31], with once recrystallized chicken ovalbumin (OVA) manufactured by the Olajne Scientific-Productive Amalgamation BIOCHEMREACTIV; at a dose 50 mg per an animal daily for 3 days. 14 days after completion of sensibilization, active anaphylactic shock (AAS) was induced in the animals with intravenous injection of an anaphylaxis-provoking homologous protein at a dose of 5 mg in 0,5 ml normal saline. AAS severety was assessed by the lethality level, the number of seizure signs and by the value of anaphylactic index [Weigle W., Cochrane C., Dixon F. Anaphylactogenic properties of soluble antigen-antibody complexes in the guinea pigs and rabbits. J. Immunology. (1969), Vol. 85, pp. 469-477].

Compounds III and XLV and Claritine (groups 2, 3 [30 guinea pigs] and 4 [24 animals], respectively) were orally administered for 3 days before provoking anaphylaxis , at a dose of 50 μg/kg for compounds III and XLV and of 1000 μg/kg for Claritine. Animals of the control group at respective terms were administered normal saline (group 1-30 guinea pigs).

The significance of differences between the groups was determined using the Fischer method of angular transformation [Gubler E.V. Computer methods of analysis and recognition of pathological processes. Moscow, Nauka publishers (1978) 365 pp.].

Table 6 shows a significant decrease in the severety of anaphylactic shock manifestations in guinea pigs which were given compounds III and XLV as compared with both the control group and the group of animals which were administered Claritine.

Table 6

Change in food anaphylaxis severety in guinea pigs as affected by one of the compounds of the present invention

| Food anaphylaxis severety / Group of animals | Lethality in percent | Seizures in percent | Anaphylactic index |
|---|---|---|---|
| Sensibilization with OVA | 20.0 | 40.0 | 2.13 |
| Sensibilization with OVA + compound III | 6.7** | 13,3* | 0.83* |
| Sensibilization with OVA + compound XLV | 6.7** | 15.5* | 0.90* |
| Sensibilization with OVA + Claritine | 25.0 | 53.3 | 2.03 |

* = significant difference in relation to groups 1 and 3.

** = p<0.01

EXAMPLE 27

STUDY OF THE EFFECT OF THE COMPOUNDS OF THE PRESENT INVENTION ON BRONCHOSPASM SEVERETY

The antiasthmatic effect of the test compounds was studied using an antigen-induced bronchospasm model in actively sensitized guinea pigs according to the Andersson method [Andersson P. Antigen induced bronchial anaphylaxis in actively sensitized guinea pig. Allergy (1980), Vol. 35, pp. 65-71].

Male guinea pigs were sensitized with an intramuscular injection of a 0.5 ml suspension comprising 20 micrograms ovalbumin (OVA, manufactured by the Sigma company [grade III]) and 100 mg $Al(OH)_3$ per one animal. An

anaphylaxis-provoking dose of 150-200 μg /kg OVA in 0.1 ml normal saline was intravenously (v. jugularis) injected on day 26 after sensibilization. Bronchospasm induction and the measurement of external respiration parameters were carried out according to the method described in Yu-Hong L., Barnes P., Rogers D., Inhibition of neurogenic plasma exudation and bronchoconstriction by a K+ channel activator, BRL 38227, in guinea pig airways in vivo. Europ. J. Pharmacol., Vol. 239, pp.257-259]. The measurement of respiration parameters was performed using a transducer (Ugo Basel, model 7020), connected with a cannula and a recorder (Millichrome), to record breathing amplitude. Bronchospasm dynamics were observed for 30-60 minutes. The efficacy of compounds was determined by the change in bronchospasm value.

A number of the compounds of the present invention were tested (III, VI, XLIV, XLVIII, XLIX). The compounds were dissolved in normal saline and intragastrally infused to animals at a dose 50 μg/kg 72, 48 and 18 hours prior to bronchospasm induction i.e. they were infused three times, and compound III intratracheally at the same dose 15 minutes prior to bronchospasm induction. Inthal was used as a reference drug and was administered to animals at a dose 5 μg/kg according to the same dosage regimen as the test substances were administered. The control group of animals received an equivalent amount of normal saline.

Compounds III and XLIV were studied in greater detail.

Groups of animals: group 1 - control; group 2 - animals given Inthal; group 3 - animals given oral compound XLIV; group 4 - animals given oral compound III; group 5 - animals given intratracheal compound III; group 6 - animals given compound XLIX, group 7 -(comparative example)- animals given compound XXXI, group 8 - animals given compound XLVI; group 9 - animals given compound VI, group 10 - animals given compound XLVIII.

All the tested compounds lowered the bronchospasm value by 20-70% as compared with the control values. Inthal decreased the bronchospasm value by almost 50%.

Table 7 summarizes the results, giving evidence of the fact that the compounds of the present invention decrease the bronchospasm value by over 50% as compared with the control values.

Table 7

| Change in bronchospasm value as affected by the test compounds | | |
|---|---|---|
| Group of animals | Number of animals in group | Bronchospasm (percent of the maximum value) |
| Group 1 - control | 21 | 87.5 10.7 |
| Group 2 - Inthal, 5 mg/kg | 14 | 40.7 3.4* |
| Group 3 - compound XLIV, 50 μg/kg per os | 12 | 15.6 3.2** |
| Group 4 - compound III, 50 μg/kg, per os | 12 | 12.0 4.0** |
| Group 5 - compound III, 50 μg/kg, intratracheally | 12 | 24.6 6.4* |
| Group 6 - compounds XLIX, 50 μg/kg, per os | 15 | 33.0 9.4* |
| Group 7 - compound XXXI, 50 μg/kg, per os | 12 | 39.3 7.6* |
| Group 8 - compound XLVI, 50 μg/kg,per os | 12 | 35.0 8.2* |
| Group 9 - compound VI, 50 μg/kg, per os | 12 | 35.0 10.2* |
| Group 10 - compound XLVIII, 50 μg/kg, per os | 12 | 22.0 3.4* |
| *= significant difference with the control group, *= p<0.05, **= p<0.01 | | |

Thus, the tested compounds of the present invention caused a similar decrease in bronchospasm value as the reference drug Inthal. The effect was manifested for both oral and intratracheal administration ration of the compounds. However, the effective dose of the tested substances was two orders lower than that of Inthal.

EXAMPLE 28

STUDY OF CHANGE IN PASSIVE CUTANEOUS ANAPHYLAXIS REACTION AS EFFECTED BY THE COMPOUNDS OF THE PRESENT INVENTION

Experiments were carried out on mongrel male mice with an initial body mass of 22-24 g. Each group included 10 animals. Sensibilization of animals was accomplished by intradermally injecting 20-30 μl of the serum of mice obtained from preliminarily immunized animals comprising reagent antibodies against chicken ovalbumin (OVA). The test compounds III and XLIV were orally administered to animals for 3 days starting with the dermal sensibilization day. Doses of 50, 150 and 500 μg/kg were used for compound III, and a dose of 50 μg/kg was used for compound XLIV. Reference drugs Suprastine at a dose 10 mg/kg and Ckaritine at a dose 2 mg/kg were administered in a similar way. 48 hours after sensibilzation, an anaphylaxis-provoking OVA dose (1 mg and 0,2 ml Evans blue in 0.2 ml normal saline) was intravenously injected into the mice. Passive cutaneous anaphylaxis reaction intensity (PCA) was determined in 30 minutes by the size (area) of a blue spot on the internal skin surface at the site of administration of the reagent anibodies. Spot size was measured in two mutually perpendicular directions and the result was expressed in $mm^2$.

The results presented in Table 8 give evidence of the fact that the administration of the test compound III at a dose 50 μg/kg to mice results in a significant decrease in PCA reaction intensity by 42%, and at doses 150 and 500 μg/kg, by 34.3% and 30.0% respectively. The administration of the compound XLIV results in decrease in PCA reaction intensity by 50%. The administration of Suprastine to experimental animals did not change PCA manifestations. When Claritine was used, a pronounced decrease in PCA reaction was observed.

Table 8

| Effect of the test compounds on passive cutaneous anaphylaxis reaction | | |
|---|---|---|
| Group of animals | Reaction intensity, $mm^2$ | Percent of the reaction inhibition |
| Control - 1 | 81.7 ± 14.5 | - |
| Compound III - 150 μg/kg | 53.7 ± 10.1$^{*1}$ | 34.3 |
| Compound III - 500 μg/kg | 57.2 ± 12.6$^{*1}$ | 30.0 |
| Control - 2 | 88.9 ± 34.6 | - |
| Compound III - 50 μg/kg | 51.6 ± 17.1$^{*2}$ | 42 |
| Compound XLIV - 50 μg/kg | 44.6 ± 11.6 | 50 |
| Suprastine - 10 μg/kg | 82.1 ± 18.1 | 7.6 |
| Claritine - 2 μg/kg | 6.4 ± 2.3$^{**2}$ | 92.8 |
| $^{*1}$ and $^{*2}$ = significant difference relative to the control groups 1 and 2, respectively; * = p0.05; ** = p<0.01. | | |

Thus, compounds III and XLIV possess the ability to lower PCA manifestation to a greater extent than Suprastine. PCA reaction intensity was inhibited to a greater extent with administration of Claritine than with administration of compounds III and XLIV, however, the effective dose of Claritine was an order higher than that of compounds III and XLIV.

EXAMPLE 29

STUDY OF THE EFFECT OF THE COMPOUNDS OF THE PRESENT INVENTION ON DELAYED HYPERSENSITIVITY

A. Effect of compound III on the delayed type hypersensitivity reaction development induced by sheep red blood cells

Studies were carried out on mongrel white male mice with an initial body mass of 22-24 g. Each group included 10 animals. Mice were sensitized by intravenous injection of the suspension of 2 x $10^5$ sheep red blood cells in 0.05 ml normal saline. On day 5, the suspension of $10^8$ sheep red blood cells in 0.05 ml normal saline was injected into the hind paw pad. As a control, the solvent, i.e. normal saline in equivalent amount, was injected. Reaction intensity was assessed after 24 hours by the difference in the mass of the paw of each animal.

$$\text{Reaction index} = \frac{M_t - M_c}{M_c} \times 100$$

The test compound III was orally administered for three days at doses 50 and 500 μg/kg according to the dosage regimen : days 3, 4 and 5. Administration of the anaphylaxis-provoking dose was on day 5.

The data presented in Table 9 show that administration of compound III at doses of 50 and 500 μg/kg significantly lowers delayed hypersensitivity intensity.

Table 9

| Effect of the compound III on the manifestation of delayed hypersensitivity induced by sheep red blood cells | | | |
|---|---|---|---|
| Group of animals | Difference in paw mass (g) | Reaction index, percent | Percent to the control |
| Control | 22.4 ± 3.7 | 13.7 ± 1.25 | 100 |
| 50 μg/kg | 16.7 ± 1.9* | 10.3 ± 1.1* | 75.5 |
| 500 μg/kg | 17.2 ± 1.3* | 10.8 ± 0.8* | 79.3 |
| * = significant difference in relation to the control group.<br>* = p<0.05 | | | |

B. Study of the local effect of the compounds of the present invention on delayed hypersensitivity induced in mice by picryl chloride

The study of the compounds effect on the delayed type hypersensitivity reaction induced in mice by 2,4,6-tichlorobenzol (TCB) was carried out according to the method of Tarayre et al. [Tarayre J.P., Barbara M., Aliaga M., Tisne-Versilles, Comparative actions of immunosuppressants, glucocorticoids and non-steroidal antiinflammatory drugs on various models of delayed hypersensitivity and on a non-immune inflammation in mice. Arzneim.-Forsch. Drug Res. (1990), Vol. 40, pp.1125-1131].

Studies were conducted on mongrel male mice with an initial body mass of 30-35 g. Each group included 10 animals. Mice were sensitized by the application of 0.1 ml 3% TCB solution in acetone on a shaved abdominal area. After 7 days an anaphylaxis-provoking TCB dose (0.025 ml of 3% solution) was applied on both surfaces of the right ear. After 30 minutes 0.05 ml of the test ointment was rubbed onto the same surfaces. 24 hours after edema induction the mice were sacrified and their ears were cut off and weighed. The allergic reaction intensity was assessed by measuring the difference in weight in grams of the right ear (the test one) in relation to the left ear (the control ear).

Allergic reaction inhibition expressed in percent was calculated according to the formula:

$$100 - \left[\frac{R\text{–}L(\text{exper.})}{L} : \frac{R\text{–}L(\text{control})}{L} \times 100\right] (\%)$$

where R = the right ear weight, and L = the left ear weight..
Control = intact animals, experiment = animals which were administered the compounds.

The test compounds were applied on the ear in the form of 1% and 5% ointments, the base of which was a 10% methylcellulose solution and propylene glycol at 1:1 ratio.

Groups of animals:

Group 1 - control, ointment base without active substance was applied on the ear of animals.

Group 2 - ointment base comprising 1% compound III was applied on the ear of animals.
Group 3 - ointment base comprising 1% compound XVII was applied on the ear of animals.
Group 4 - ointment base comprising 5% compound XLVIII was applied on the ear of animals.

The data presented in Table 10 give evidence of the fact that the test compounds possess the ability to inhibit delayed hypersensitivity induced by TCB.

Table 10

| Effect of the test compounds on delayed hypersensitivity induced in mice by TCB | | | | |
|---|---|---|---|---|
| Group of animals | Weight of ears (g) | | weight increment (g) | Reaction inhibition (%) |
| | Right | left | | |
| Group 1 | 22.9 ± 1.72 | 15.0 ± 141 | 7.90 ± 0.56 | - |
| Group 2 | 20.35 ± 1.21 | 15.4 ± 1.12 | 4.9 ± 0.5* | 38% |
| Group 3 | 21.6 ± 1.02 | 15.5 ± 1.02 | 6.1 ± 0.59* | 24% |
| Group 4 | 17.9 ± 0.94 | 14.2 ± 0.98 | 3.7 ± 0.81** | 54% |
| * = significance of differences in relation to the control group<br>*=p<0.05; ** = p<0.01. | | | | |

EXAMPLE 30

STUDY OF THE EFFECT OF THE COMPOUNDS OF THE PRESENT INVENTION ON ACUTE INFLAMMATION DEVELOPMENT

A. The model of edema induced by Freund's complete adjuvant.

The experiment was carried out on white mongrel male rats weighing 280-300 g. The model of paw edema induced by Freund's complete adjuvant (FCA), was reproduced according to the method of Ezamuzie and Umezurike [Ezamuzie Ch., Umezurike C.C., Effect of histamine H2-receptor antagonists on acute inflammatory of the rat paw oedema. J. Pharmacol. (1989), Vol. 41, pp. 261-265]. Paw volume measurement was done using a plethysmograph (Ugo Basile). The inhibition of inflammatory reaction, expressed in percent, was calculated according to the formula:

$$100 - \left[\frac{R-L(\text{exper.})}{L} : \frac{R-L(\text{control})}{L} \times 100\right] (\%)$$

where R = the right paw volume and L = the left paw volume.
Control - intact animals, experiment - animals which were administered the compounds.

The test compounds were intragastrally administered to animals through a probe four times, at a dose 50 μg/kg in 0.5 ml of 1% starch solution, 72, 48, 24 and 1 hour before FCA administration. The reference drug Naproxene was administered once, at a dose of 50 μg/kg, 1 hour before edema induction. Each group included 10 animals.

Group of animals:

Group 1 - control, animals were administered 0.5 ml of 1% starch solution.
Group 2 - animals were administered the compound XLVIII.
Group 3 - animals were administered the compound III.
Group 4 - animals were administered Naproxene.

The data presented in Table 11, give evidence of the fact that the tested compounds possess the ability to

inhibit the FCA induced inflammatory reaction. Antiinflammatory effect intensity for the compound III is comparable to the effect of Naproxene, but the total dose of Naproxene was 25 times higher than that of compound III.

Table 11

| Effect of the test compounds on the FCA-induced edema of paws in rats | | | | |
|---|---|---|---|---|
| Group of animals | Volume of paws (conditional units) | | Volume increment | Inflammation inhibition (%) |
| | Right | left | | |
| Group 1 | 2.22 ± 0.85 | 1.42 ± 0.45 | 0.80 ± 0.07 | - |
| Group 2 | 1.95 ± 0.48 | 1.30 ± 0.62 | 0.65 ± 0.04* | 19% |
| Group 3 | 1.85 ± 0.62 | 1.25 ± 0.34 | 0.54 ± 0.04* | 32.5% |
| Group 4 | 1.75 ± 0.54 | 1.30 ± 0.25 | 0.45 ± 0.02** | 44% |
| * = significance of differences in relation to the control group.<br>*= p<0.05; ** = p<0.01. | | | | |

B. Study of the effect of the compounds on the carrageenan edema model in rats

This experiment was carried out on white mongrel male rats weighing 280-300 g. Carrageenan-induced edema was caused according to the method of Winter et al. [Winter, De Rosa M., Giroud J.P., Willoughby D.A., Studies of the mediators of the acute inflammatory reponse induced in rats in different sites by carrageenan , and turpentine; J. Pharmacol. (1971), Vol. 104, pp. 15-29]. Paw volume was measured using a plethysmograph (Ugo Basile) 1, 3 and 4 hours after carrageenan administration. The inhibition of inflammatory reaction, expressed in percent, was calculated according to the formula:

$$100 - \left[\frac{R-L(\text{exper.})}{L} : \frac{R-L(\text{control})}{L} \times 100\right] (\%)$$

where R = the right paw volume and L = the left paw volume.
Control = intact animals; experiment = animals which were administered the compounds.

Series I of experiment. The test compounds were intragastrally administered to animals through a probe four times, at a dose of 50 μg/kg in 0.5 ml of 1% starch solution, 72, 48, 24 and 1 hour before FCA administration. The reference drug Naproxene was administered one at a dose of 20 μg/kg 1 hour before edema induction. Each group included 10 animals.

Groups of animals:

Group 1 - control, animals were administered 0.5 ml of 1% starch solution.
Group 2 - animals were administered the compound III.
Group 3 - animals were administered the compound II.
Group 4 - animals were administered Naproxene.

The data presented in Table 12 give evidence of the fact that the compounds, on oral administration, possess the ability to inhibit carrageenan induced inflammatory reaction. The antiinflammatory effect intensity of compound III is comparable to the effect of Naproxene, when the total dose of Naproxene was 10 times higher than that of the compound III.

Table 12

| Effect of the test compounds on the carrageenan-induced edema of paws in rats with oral administration | | | | |
|---|---|---|---|---|
| Group of animals | Volume of paws (conditional units) | | Volume increment | Inflammation inhibition (%) |
| | Right | left | | |
| Group 1 | 2.12 ± 0.63 | 1.35 ± 0.23 | 0.77 ± 0.04 | - |
| Group 2 | 1.72 ± 0.27 | 1.23 ± 0.41 | 0.49 ± 0.07** | 30%. |
| Group 3 | 1.92 ± 0.65 | 1.28 ± 0.28 | 0.64 ± 0.05*1 | 12% |
| Group 4 | 1.82 ± 0.54 | 1.34 ± 0.25 | 0.48 ± 0.02* | 37% |
| * = significance of difference in relation to the control group. **=p<0.01; *1=p<0.1. | | | | |

Series 2 of experiment. 2% carrageenan solution was injected sublaterally into the paws of rats. After 1 minute 0.1 ml of a gel comprising the test compounds was applied to the right paw. The gel base consisted of propylene glycol dissolved in a mixture of water and ethanol. The concentration of the compounds in the gel was 1%. The volume of paws was measured 4 hours after carrageenan administration.

Groups of animals:

Group 1 - control -1, gel without compound was applied on paw.
Group 2 - the applied gel comprised the compound XL.
Group 3 - the applied gel comprised the compound III.
Group 4 - the applied gel comprised the compound XXXVI.
Group 5 - the applied gel comprised the compound XIII.
Group 6 - control -2, gel without compound was applied on paw.
Group 7 - gel comprised 1% Voltaren.

The data presented in Table 13, give evidence of the fact that the tested compounds in cutaneous application possess the ability to inhibit the carrageenan-induced inflammatory reaction. The antiinflammatory effect intensity of the compounds is comparable with the effect of the reference drug Voltaren.

Table 13

| Effect of the test compounds on the carrageenan-induced edema of paws in rats, with cutaneous application | | | | |
|---|---|---|---|---|
| Group of animals | Volume of paws (conditional units) | | Volume increment | Inflammation inhibition (%) |
| | right | left | | |
| Group 1 control-1 | 2.56 ± 0.83 | 1.84 ± 0.13 | 0.72 ± 0.08 | - |
| Group 2 | 2.12 ± 0.36 | 1.87 ± 0.24 | 0.29 ± 0.08** | 63% |
| Group 3 | 2.13 ± 0.64 | 1.83 ± 0.21 | 0.30 ± 0.06*1 | 60% |
| Group 4 | 2.23 ± 0.42 | 1.86 ± 0.16 | 0.37 ± 0.07** | 50% |
| Group 5 | 2.29 ± 0.18 | 1.82 ± 0.34 | 0.47 ± 0.08** | 35% |
| Group 6 control-2 | 2.42 ± 0.25 | 1.56 ± 0.34 | 0.74 ± 0.06 | - |
| Group 7 | 1.99 ± 0.24 | 1.63 ± 0.26 | 0.40 ± 0.03 ** | 48% |
| * = significance pf differences in relation to the control group. **= p<0.01. | | | | |

EXAMPLE 31

ASSESSMENT OF INFLAMMATORY EFFECT OF THE COMPOUNDS OF THE PRESENT INVENTION ON THE NON-INFECTIOUS PULMONARY GRANULOMATOSIS MODEL

The antiinflammatory and immunomodulating effect of compound III upon intragastral administration was studied using the non-infectious pulmonary granulomatosis model.

20 female 4 month old Wistar rats weighing 200-220 g were used in the exepriment. Wistar rats were selected because with aerosol Sephadex A-25 administration granulomatous inflammatory process develops in lungs [Makarova O.V., Kovaleva V.L., Sladkopevtsev A.S., Mikhailova L.P., Noseikina E.M., Experimental model of non-infectious pulmonary granulomatosis (1996), No 1, pp. 76-79]. Data on the number of animals in groups are presented in Table 14.

Table 14

| Experimental groups and number of animals in them. | | |
|---|---|---|
| Order No. | Group of experiments | Number of animals |
| 1. | Control (intact animals) | 12 |
| 2. | Sephadex A-25 (day 7) | 12 |
| 3. | Sephadex A-25 + compound III | 16 |

Technique of aerosol Sephadex A-25 administration

Sephadex A-25, at a dose of 5 mg per 1 kg of body mass, was administered to rats under ether anesthesia using an original dosing device for inhalation administration of dry powders manufactured at the Scientific-Research Institute for Medical Instrument-Making. Wistar rats quickly recovered from anesthsia following Sephadex A-25 administration and they did not show any peculiarities in their behaviour and respiration character. Compound III was administered intragastrally using a gastric probe at a dose 50 mg per 1 kg of body mass and immediately after that, single aerosol administration of Sephadex A-25 was performed According to the technique already described. Then compound III was administered intragastrally once daily for 6 days at a dose 50 mg/kg of body mass, in the form of a water solution. Rats were examined at the 7th day after commencement of administering compound III and Sephadex A-25.

Bronchoalveolar lavage was obtained under Hexenal anesthesia Cytosis, i.e. the absolute number of cells in 1 ml, was determined in the bronchoalveolar lavage fluid. The endopulmonal cytogram (in percent) was calculated in the smears stained according to Romanovsky-Gimza. Following the bronchoalveolar lavage procedure, lungs with trachea were resected from the thoracic cavity and macropreparation was placed into a 2% acetic acid solution. After 18-24 hours, the trachea, main and lobar bronchi were dissected under a lense. The volume density of lymphoid follicles was estimated under the lense using the point count method. A histological examination of lungs stained with hematoxylin and eosine was performed. The volume density of alveolitis and emphysema was determined using the point count method.

The histological examination of the lungs of the control group rats (intact animals) did not reveal any pathological changes. The histological examination of the lungs of Wistar rats on day 7 after aerosol effect of Sephadex A-025 found a morpological picture of a granulomatous inflammatory process mature macrophage granulomas, acute bronchitis and alveolitis with neutrophilic component, focal emphysema. Granulomas were disposed along the pulmonary connective tissue and most of them were found in the connective tissue along blood vessels, pulmonary artery branches and pulmonary veins, as well as in the wall of bronchi. A little portion of small macrophage granulomas was found in the interstitium of interalveolar septa "angles", alveolar passages and respiratory and terminal bronchioles. The cellular composition of granulomas was predominantly represented by macrophages as well as by occasional neutrophils and lymphocytes. In the morphometric examination of Wistar rats' lungs, the parameters of alveolitis and emphysema volume density were 7.0 $\pm$ 3.0 and 15.1 $\pm$ 5.1%, respectively (Table 15).

Table 15

| Morphometric characteristics of Wistar rats' pulmonary tissue following the aerosol effect of Sephadex A-25 and showing the effect of the administration of the compound III | | | |
|---|---|---|---|
| Order No. | Group of Animals | Volume density in percent | |
| | | Alveolitis | Emphysema |
| 1. | Control (intact animals) | - | 1.8 $\pm$ 1.0 |

(continued)

| Morphometric characteristics of Wistar rats' pulmonary tissue following the aerosol effect of Sephadex A-25 and showing the effect of the administration of the compound III | | | | |
|---|---|---|---|---|
| Order No. | Group of Animals | | Volume density in percent | |
| | | | Alveolitis | Emphysema |
| 2. | Sephadex A-25 (day 7) | | 7.0 ± 3.0 | 15.1 ± 5.1 |
| 3. | Sephadex A-25 + compound III | | 3.8 ± 1.0 | 11.2 ± 3.2 |
| Significance of differences P | | 1 - 2 | - | <0.05 |
| | | 2 - 3 | >0.05 | >0.05 |
| | | 1 - 3 | - | <0.05 |

According to the data of bronchoalveolar lavage fluid cytological examination in Wistar rats at day 7 following aerosol effect of Sephadex A-25, the cytosis index rase as compared with the control (table 16). The increase in percentage content of neutrophils and lymphocytes in the endopulmonary cytogram was observed, but the differences were not statistically significant.

Table 16

| Bronchoalveolar lavage cytosis and cellular composition parameters in Wistar rats following exposure to aerosol Sephadex A-25 and treatment with compound III | | | | | | |
|---|---|---|---|---|---|---|
| No | Group of animals | | Cytosis (absolute number of cells in 1 ml) | Endopulmonary cytogram in percent | | |
| | | | | Macrophages | Lymphocytes | Neutrophils |
| 1. | Control (intact animals) | | 0.168 ± 0.050 | 95.5 ± 1.1 | 3.7 ± 1.1 | 0.8 ± 0.2 |
| 2. | Sephadex A-25 (day 7) | | 0.189 ±0.042 | 54.2 ±9.4 | 15.3 ±1.1 | 34.0 ±12.0 |
| 3. | Sephadex A-25 + compound III | | 0.169 ±0.054 | 75.2 ±4.5 | 7.6 ±1.5 | 16.8 ± 2.6 |
| Significance of differences P . | | 1 - 2 | >0.05 | <0.05 | <0.01 | <0.05 |
| | | 1 - 3 | >0.05 | <0.05 | <0.05 | <0.05 |
| | | 2 - 3 | >0.05 | >0.05 | <0.01 | >0.05 |

In the group of rats treated with compound III, the histological examination of lungs also revealed the picture of granulomatous inflammation. According to morphometric examination data, the prevalence of alveolitis and emphysema decreased and was 38±1.0 and 11.2±3.2%, respectively, but the differences were statistically insignificant (see Table 15). According to cytological examination data, cytosis index in the group of rats treated with compound III normalized (see table 16). A significant decrease in percent content of neutrophils and lymphocytes was found in endopulmonary cytogram, however, the normalization of parameters was not observed.

The effect of compound III on the pulmonary immune system was assessed by its effect on the lymphoid tissue associated with bronchi. The volume density morphometric parameters of the lymphoid tissue associated with bronchi are represented in Table 17.

Table 17

| Morphometric parameters of the lymphoid tissue associated with bronchi in Wistar rats following exposure to Sephadex A-25 aerosol and treatment with compound III | | |
|---|---|---|
| No | Group of animals | Volume density of lymphoid follicles in percent |
| 1 . | Control (intact animals) | 15.0 ±3.9 |
| 2. | Sephadex A-25 (day 7) | 44.8 ±3.7 |

(continued)

| Morphometric parameters of the lymphoid tissue associated with bronchi in Wistar rats following exposure to Sephadex A-25 aerosol and treatment with compound III | | | |
|---|---|---|---|
| No | Group of animals | | Volume density of lymphoid follicles in percent |
| 3. | Sephadex A-25 + compound III | | 34.7 ±10.3 |
| Significance of differences P | | 1 - 2<br>1 - 3<br>2 - 3 | <0.05<br>>0.05<br>>0.05 |

The volume density index of lymphoid follicles in Wistar rats was normally low making up 15.0 ± 3.9 %. A pronounced pulmonary lymphoid apparatus hyperplasia was noted by day 7 following exposure to Sephadex A-25 aerosol, the volume density index of lymphoid follicles being 44.8 ± 3.7 %. In the group of rats treated with the compound III, a the volume density of lymphoid follicles decreased to 34.7 ± 10.3%.

Thus, compound III was found to have antiinflammatory activity on the pulmonary non-infectious granulomatosis induced by Sephadex -A-25. According to the data of pathological, morphometric and cytological examinations, compound III induces a decrease in inflammation exudative component intensity and alveolitis prevalence, as well as the inhibition of lymphoid tissue hyperplasia.

EXAMPLE 32

ANTIOXIDANT ACTIVITY STUDY OF ONE OF THE COMPOUNDS OF THE PRESENT INVENTION IN AN IN VIVO EXPERIMENT

Antioxidant activity was studied in an in vivo experiment, using the model of acute toxic hepatic lesion with carbon quadrichloride ($CCl_4$).

The experiment was carried out on 40 mongrel male rats with an initial body mass of 190-200 g and fed a standard vivarium ration. Each group included 10 animals. Hepatic lesion (experimental hepatitis) was induced in the animals by intragastral administration of 0.25 ml per 100 g of body mass of $CCl_4$ in the form of a 50% solution in vaseline oil, for 3 days [Vengerovsky A.I., Chuchalin V.S., Pauls O.V., Saratikov A.S., Effect of hepatoprotectors on lipid metabolism in hepatitis induced by CCl4, Bulletin of Experimental Biology (1987), No 4, pp. 430-432]. The-test compound III was administered intragastrally to animals at oF doses of 50 and 500 μg/kg for three days when $CCl_4$ was administered (groups 3 and 4). Animals of the control group were administered $CCl_4$ as described above (group 2). Intact animals were orally given normal saline in an equivalent amount (group 1).

Blood and liver specimens were taken for analysis 18 hours after the last $CCl_4$ administration.

The content of primary lipid peroxidation (LP) products - diene conjugates, diene ketones and trienes - were determined using the published method [Volchegorsky I.A., Nalimov A.G., Yarovinsky B.G., Lifshits R.I., Comparison of different approaches to determining lipid peroxidation products in heptane-isopropanolic blood extracts, Voprosy Meditsinskoy Chimii (Problems of Medical Chemistry) (1989), No 1, pp. 127-131]. The content of LP products was calculated by correlating the values of respective extinctions with 1ml of test sample.

The amount of LP products - malonic dialdehyde (MDA) - was determined by the test with thiobarbituric acid (TBA) [Korobeinikova E.N., Modification of lipid peroxidation products in the reaction with thiobarbituric acid., Laboratornoye delo (Laboratory Matter) (1989), No 7, pp. 8-10]. The concentration of the TBA-active product was calculated using regression equation. The MDA content in the liver of experimental animals was assessed using the modified method [Stalnaya I.D., Garishvili T.G., The method of determining malonic dialdehyde using thiobarbituric acid., In: Modern methods in biochemistry, Moscow, Meditsina publishers. (1977), pp. 66-69], performing preliminary extraction of lipids according to Folch [Cates M. Technology of Lipidology, Moscow, Mir publishers (1975), pp. 74-76].

The results presented in Table 18 give evidence of the fact that $CCl_4$ administration to rats resulted in the accumulation of PL products in blood serum and liver, and that in animals of experimental groups which were administered the test compound, a significant decrease in the hepatic content of primary LP products and serum and hepatic MDA content was noted.

Table 18

**Effect of the test compounds on serum and hepatic content of lipid peroxidation products in rats with experimental hepatitis**

| Parameters / Group of animals | Blood serum, MDA, nM/ml | Liver, MDA, nM/mg of tissue | Liver, diene conjugates, $E_{232}$/mg of tissue | Liver, diene ketones, trienes $E_{278}$/mg of tissue |
|---|---|---|---|---|
| Group 1, intact rats | 3.59 ± 0.16 | 4.68 ± 0.21 | 15.26 ± 0.48 | 7.45 ± 0.34 |
| Control of Group 2 $CCl_4$ | 5.34± 0.054**[1] | 7.18± 0.43***[1] | 19.12 ± 1.07*[1] | 12.44 ± 0.98*[1] |
| Group 3 simultaneous administration of $CCl_4$ and the compound at a dose 50 µg/kg | 4.05 ± 0.198*[2] | 4.98 ± 0.13**[2] | 16.25 ± 0.61 | 8.42 ± 0.25**[2] |
| Group 3 simultaneous administration of $CCl_4$ and the compound at a dose 500 µg/kg | 3.89 ± 0.11**[2] | 5.01 ± 0.17**[2] | 15.89 ± 0.38 | 8.24 ± 0.19*[2] |

* = significant difference. * = $p<0.05$; ** = $p<0.01$; *** = $p<0.001$.

The superscript numbers 1 and 2 show the numbers of the group in relation to which differences are significant.

The results showed that compound III of the present invention possesses a pronounced antioxidant effect in the model of acute toxic hepatic lesion.

EXAMPLE 33

CHANGE IN HEPATIC P-450. CYTOCHROME SYSTEM AS EFFECTED BY THE COMPOUNDS OF THE PRESENT INVENTION

A. Effect of compounds of the present invention on Hexenal sleep duration in animals

Change in Hexenal sleep (HS) duration is the in vivo measurable index of the hepatic P-450 cytochrome system condition.

studies were carried out on male mice of the lines BALB/c*h75, C57B1/6, CBA, DBA/2 and first generation CBF1 and BDF1 hybrides, with an initial mass 18-20 g, each group including 10 mice; and on mongrel male guinea pigs with an initial mass of 250-300 g, each group including 24 animals. All the test compounds (from II to LII) were administered orally in drinking water, for three days at doses of 50 and 500 µg/kg. Hexenal hydrochloride was administered at a dose 36 µg/kg of animals mass 24 hours after the last drug administration, except for the BALB/c*h75 mice line, which were administered Hexenal at a dose of 60 µg/kg at the same time, and guinea pigs for which the Hexenal dose was 30 µg/kg. HS duration was determined in minutes.

Table 19 summarizes data on the change in Hexenal sleep (HS) duration in mice of different lines, CBF1 and BDF1 hybrides, as well as in guinea pigs as influenced by the compounds of the present invention. It was shown that

the compounds decrease HS duration in experimental animals at doses of both 50 and 500 μg/kg.

Table 19

| Change in Hexenal sleep duration in animals as influenced by the test compounds | | | | |
|---|---|---|---|---|
| Compound | Animals | Hexenal sleep duration (minutes) | | |
| | | Control | Doses of the compound | |
| | | | 50μg/kg | 500μg/kg |
| XLIV | BALB/c*h75 | 29.16 ± 5.68 | 26.50 ± 3.49 | 16.72 ± 1.27*** |
| XLV | CBA | 21.88 ± 1.34 | 17.98 ± 1.66* | 16.13 ± 1.86** |
| XLV | BDF1 | 23.78 ± 0.78 | - | 20.33 ± 0.59* |
| XLIV | guinea pigs | 24.76 ± 3.23 | 13.48 ± 1.02* | - |
| XXXIII | CBF1 | 25.91 ± 1.57 | - | 19.82 ± 2.12* |
| XLVIII | CBF1 | 25.91 ± 1.57 | 16.27 ± 1.26** | 20.91 ± 1.48* |
| XLIX | CBF1 | 25.91 ± 1.57 | 19.68 ± 2.04** | - |
| III | CBF1 | 25.91 ± 1.57 | 19.61 ± 2.69** | 22.02 ± 1.45* |
| II | the same | the same | - | 21.72 ± 1.24* |
| IV | the same | the same | 20.39 ± 1.59* | - |
| XI | the same | the same | - | 17.71 ± 0.66*** |
| VII | the same | the same | 17.62 ± 0.96* | 16.99 ± 1.33* |
| IX | the same | the same. | 16.71 ± 0.94** | 20.76 ± 1.32 |
| XLVI | the same | the same | 16.91 ± 1.28** | 13.21 ± 0.70*** |
| XXXI (Comparative Example) | the same | the same | 12.59 ± 0.88*** | 12.61 ± 1.45*** |
| X | the same | the same | 13.37 ± 1.78*** | 12.01 ± 1.23*** |
| XII | the same | the same | 19.74 ± 1.94* | 17.12 ± 1.90** |
| VI | the same | the same | 16.86 ± 1.38*** | 14.39 ± 1.73*** |
| LII | the same | the same | 21.14 ± 1.39 | - |
| * =Significance of differences in relation to the control group.<br>* = $p<0.05$; ** = $p<0.01$; *** = $p<0.001$ | | | | |

In examining changes in HS duration as influenced by the rest of the compounds of the present invention in CBF1 mice, it was shown that the compounds decrease HS duration by from 13% to 54% as compared with the control.

Thus, all the tested compounds decrease Hexenal sleep duration, which gives evidence that they induce the hepatic P-450 cytochrome system.

B. Change in terminal oxidase and cytochrome $B_5$ content as influenced by compounds of the present invention.

The change in hepatic monooxygenase system was studied using mongrel male guinea pigs with an initial mass of 250-300 g each group included 12 animals. The test compound III and Phenobarbital (PB) were administered three times 72, 48 and 24 hours before monooxygenase system examination. The content of P-450 and $B_6$ cytochromes was measured, according to the published method [Omura T., Scato R., The monooxide binding pigment of liver microsomes., 11. Solubilization, purification and properties. J. Chem. (1964), Vol. 239, pp. 2379-2385], in the microsomal liver fraction, and isolated using the differential centrifugation method [Ahokas J., Pelkonen O., Karkin N. Characterisation of BP-hydroxylase of Trout-liver. Cancer Res. (1977), Vol. 37, pp. 3737-3743]. The priority method [Izotov M.V., Scherbakov V.M., Devichensky V.M. et al., The method of determining the content of P-450 cytochrome isoenzymes in liver microsomes. The inventor's certificate No 1,488,738. B.I. (1989), No 3.-6.06.09, ICI, 01. - No 33/15, No 33/48]. Microsomal protein content was determined using the modified Lowry method [Hartree E. Determination of protein: a modification of the Lowry method, that gives a linear photometric responses., Ann. Biochem. (1972), Vol. 48, pp. 422-427].

The results presented in table 20, give evidence of the fact that in guinea pigs pretreated with the test compound and PB, the total hepatic content of P-450 cytochromes and $B_5$ rises significantly. At the same time, animals of groups 2 and 3 showed a significant increase in P-450B cytochromes and animals administered the test compound of the present invention also showed a significant increase in the ratio of P-450B to P450L cytochromes and in the ratio of $B_5$ to P-450 cytochromes.

The test compound III was found to change the hepatic P-450 cytochrome system in a way similar to PB, however, it has its own peculiarities, such as elevating the ratio of P-450B to P450L cytochromes and the ratio of $B_5$ to P-450 cytochromes.

Table 20

**Change in the hepatic P–450 cytochrome system as effected by the test compound**

| Group of animals / Parameters | Control 1 | Phenobarbital 2 | Compound 3 |
|---|---|---|---|
| $B_5$ cytochrome nM/mg of protein | 0.61 ± 0.02 | 1.21 ± 0.10* | 1.00 ± 0.03* |
| P–450 cytochrome nM/mg of protein | 0.82 ± 0.07 | 1.50 ± 0.09* | 1,03 ± 0.03 |
| P–450B cytochromes nM/mg of protein | 0.43 ± 0.03 | 0.76 ± 0.08* | 0.72 ± 0.02* |
| P–450B/p–450L | 1.10 | 1.02 | 2.32** |
| $B_5$ /P–450 | 0.82 | 0.81 | 0.97* |

* = $p < 0.05$; ** = $p < 0.01$ – significance of differences in relation to the control group.

EXAMPLE 34

CHANGE IN HORMONAL STATE OF ANIMALS AS EFFECTED BY THE COMPOUNDS OF THE PRESENT INVENTION

Studies were carried out on mongrel male guinea pigs with an initial body mass of 250-300 g. Each group included 12 animals. Sensibilization of animals was done as described in example 27. Blood (for hormonal level determination) was sampled in animals-before commencing the experiment and 18 hours after the last drug administration for 10 to 11 hours. The substance was orally administered three times to the sensitized and intact animals, at a dose of 50 μg/kg, 72, 48 and 18 hours before the second blood sampling. A hormonal state analysis was carried out by comparing individual changes in each animal in a group. Mean parametric values expressed in nM/l are presented in Table 21. The blood serum levels of the hormones progesterone, 17-oxyprogesterone, 11-desoxycortisol and cortisol were determined by radioimmunoassay using Beloris company kits. The hormone concentration in samples was determined from the relation graph between the activity of a precipitated bound labelled hormone and the hormone concentration in calibration samples.

The experimental results are presented in Table 21. The administration of the compound XLIV to intact animals resulted in a significant rise in the levels of free cortisol (F) and of its precursors oxyprogesterone (17-OH-P) and desoxycortisol, as well as a trend to increase the progesterone (P) level. In sensitized guinea pigs a significant decrease in blood serum F and 17-OH-P levels and a trend to decrease in P level were found. In sensitized animals which were administered the test compound, normalization of blood serum F and desoxycortisol levels and a significant increase in P and 17-OH-P levels were noted.

On administering compound III to both intact and sensitized experimental animals, the same hormonal state changes were observed.

Table 21

Change in blood serum hormonal levels as effected by the test compounds

| Group of animals / Parameters | Sensibilization | Sensibilization + compound 50 μg/kg | Compound 50 μg/kg |
|---|---|---|---|
| Control Cortisol Experiment | 3079.1<br>2157.7* | 1910.3<br>1997.0 | 1474.5<br>1891.5* |
| Control Progesterone Experiment | 3.91<br>3.20 | 1.73<br>5.08** | 2.53<br>2.87 |
| Control Oxyprogesterone Experiment | 2.95<br>1.93* | 1.79<br>2.31* | 2.83<br>3.88* |
| Control Desoxycortisol Experiment | 21.58<br>20.91 | 28.9<br>33.5 | 20.8<br>30.95* |

Blood levels of hormones was calculated in nM/l.

* = significance of differences was calculated in relation to the individual control. * = $p<0.05$; ** = $p<0.001$

EXAMPLE 35

EFFECT OF THE COMPOUNDS OF THE PRESENT INVENTION ON METABOLISM OF [$C^{14}$]-ARACHIDONIC ACID IN PULMONARY TISSUE HOMOGENATE

Studies were carried out on CBA male mice fed a standard vivarium ration. The animals were administered the compound at a dose of 50 μg/kg, and phenobarbital at a dose of 80 μg/kg, for three days. Then the animals were sacrified, their lungs were taken out, frozen in liquid nitrogen, homogenized in a glass homogenizer manufactured by the Wheaton company (U.S.A.) at +4°C in 10 volumes of 0.05 M Tris-HCl buffer. Aliquots (0.5 ml) of supernatant were incubated in 0.5 μC [$C^{14}$]-arachidonic acid ([$C^{14}$]-AA, Amersham, Great Britain; specific activity 50-60 μCi/mM) at +37°C for 30 minutes. The non-metabolized [$C^{14}$]-AA and its metabolism products were extracted in 20 volumes of a chloroform and methanol (1:1) mixture at an extraction efficacy not less than 90%, assessed using [$C^{14}$]-$PGF_{2\alpha}$. The separation and identification of [$C^{14}$]-AA and its metabolites was performed by thin-layer chromatography (the Merck company Kieselgel 60 plates, Germany) using an organic phase of a system of solvents (ethyl acetate:isooctane:acetic acid:water - 110:50:20:100) and labelled standards. Densitometry of the autoradiochromatograms obtained on the X-ray films X-Omat AR (Kodak, U.S.A.) and HS 11 (ORWO, Germany) were performed on the KS 3 densiscan (Kipp and Zonnen, Holland). Quantative analysis of individual eucosanoids was carried out using radiometry of the fractions obtained with high performance liquid chromatography (the Gilson company HPLC-system, France; the Du Pont company ZORBAX C8 column, U.S.A.) and with elution of spots on TLC-plates.

The experimental results are represented in Table 22. The test compound was found to stimulate the production of cyclooxygenase AA metabolites, as specified, namely it increased prostaglandin $E_2$ ($PGE_2$) synthesis, and 6-keto-$PGF_{1\alpha}$ , $PGF_{2\alpha}$, and lipoxygenase AA metabolism way, i.e. it increases the production of 5-HETE and HHT. At the same time, a decrease in AA metabolism is observed in the P-450 cytochrome system - 12-HETE and 15-HETE. It should be

noted that the change in the profile of AA metabolites in animals pretreated with one of the compounds of the present invention was similar to that found in animals receiving Phenobarbital - a known cytochrome P-450 system inducer.

Table 22

| Effect of the compound III on arachidonic acid metabolism | | | | |
|---|---|---|---|---|
| Groups | Control 1 | Phenobarbital 2 | Control 3 | Compound 4 |
| Fractions | percent of AA conversion | | | |
| Phospholipids | 8.17 ± 0.33 | 8.18 ± 0.21 | 7.65 ± 1.65 | 7.41 ± 0.25 |
| 6-keto-$PGF_{1\alpha}$ | 4.83 ± 0.47 | 4.12 ± 0.12 | 6.80 ± 0.56 | 7.95 ± 0.28 |
| $PGF_{2\alpha}$ | 6.27 ± 0.18 | 7.91 ± 0.08 | 6.40 ± 0.50 | 8.05 ± 0.15 |
| $TXB_2$ | 4.10 ± 0.20 | 6.21 ± 0.18 | 5.10 ± 0.30 | 5.76 ± 0.24 |
| $PGE_2$ | 2.47 ± 0.20 | 3.82 ± 0.14 | 2.40 ± 0.11 | 3.07 ± 0.11 |
| 5-HETE+HHT | 10.07 ± 0.18 | 10.5 ± 0.26 | 10.80 ± 0.31 | 13.64 ± 0.26 |
| 12-, 15-HETE | 34.80 ± 0.35 | 27.1 ± 0.11 | 38.55 ± 0.78 | 23.82 ± 0.82 |
| Unmetabolized AA | 19.97 ± 0.26 | 14.5 ± 0.28 | 2.85 ± 0.15 | 15.32 ± 1.56 |

EXAMPLE 36

CHANGE IN ANTIGEN-DEPENDENT HISTAMINE SECRETION BY PERITONEAL MAST CELLS OF SENSITIZED RATS AS EFFECTED BY THE COMPOUNDS OF THE PRESENT INVENTION

Sensibilization of Wistar male rats with an initial body mass of 200-250 g was carried out according to the published method of Guschin et al [Guschin et al Guschin LS., Voitenko V.G., Sviridov B.D. et al., A polyfunctional molecule produced by the conjugation of synthetic polyionimmunostimulant with specific antigen and an inhibitor of mast cell activation. Effects on histamine release. Agents and Actions (1989), Vol. 27, pp. 75-78]. After 14 days of sensibilization according to the standard method of Fredholm et al [Fredholm B.B., Gyschin I.S., Elwin K. et al., Cyclic AMP-independent inhibition by papaverine of histamine release induced by compound 48/80, - Biochem. Pharmacol. (1976), Vol. 25, pp. 1583-1588], a cellular suspension was isolated from the peritoneal cavity, and the spontaneous and chicken ovalbumin (OVA)-stimulated histamine secretions by mast cells (MC) was determined. The histamine level in mast cells of the control and experimental animals was also determined 2 ml of a cellular suspension containing 0.1-0.2 x $10^6$ MC/ml was incubated in the presence of 200 μg/kg of OVA. Histamine secretion was expressed as a percentage of its total level. The amount of histamine in samples was determined using a spectrofluorimetric method [Short P.A., Burkhalter A., Cohn V.N., A method for fluorimetric assay of histamine in tissues, J. Pharmacol. Exper. Ther. (1959), Vol. 127, pp. 182-186].

The test compound was administered to animals intraperitoneally, according the following dosage regimen: for 3 days prior to examining MC, a test compound at a dose of 50 μg/kg (group 2) or Suprastine at a dose 1000 μg/kg (group 3) was administered. Animals of the control group were intraperitoneally injected normal saline (group 1). Each group included 10 animals.

The experimental results are presented in Table 23. The administration of compound XLV was shown to significantly lower antigen-dependent histamine secretion by MC. At the same time, a significant rise in spontaneous histamine secretion by MC was noted. These changes occur against the background of a decrease in the histamine level in the MC of sensitized animals administered one of the test compounds.

Table 23

| Change in histamine secretion by mast cells of sensitized rats as effected by compound XLV | | | |
|---|---|---|---|
| Parameters | Treatment | | |
| | Control 1 | Compound 2 | Compound 3 |
| Spontaneous secretion, percent | 5.24 | 9.63*[1,3] | 5.62 |
| Stimulated secretion, percent | 7.16 | 2.32*[1,3] | 7.02 |

(continued)

| Change in histamine secretion by mast cells of sensitized rats as effected by compound XLV | | | |
|---|---|---|---|
| Parameters | Treatment | | |
| | Control 1 | Compound 2 | Compound 3 |
| Histamine level, μg/$10^6$ MC | 25.62 | 16.90*1 | 16.24*1 |
| * = $p < 0.05$ nm (significance of differences). Statistical processing using $\chi^2$ test. | | | |

EXAMPLE 37

EFFECT OF THE COMPOUNDS OF THE PRESENT INVENTION ON THE PRODUCTION OF ACTIVE OXYGEN FORMS IN MODEL SYSTEMS

The effect of the compounds of the present invention on changes in chemiluminescence (CL) as determined by the hydroxyl radical ( OH) and the superoxide anion-radical ($O_2$-) was studied using in model chemical and enzymatic systems.

Active oxygen forms (AOF) of different nature were generated in the following systems:

A. Hydroxyl radical - in a mixture of $FeSO_4$ with $H_2O_2$ (the Fenton reagent) [Halliwell B. Superoxide-dependent formation of hydroxyl radicals in the presence of iron salts. FEBS Lett. (1978), Vol. 96, pp. 238-241].
The incubation medium comprised 5 mM $KH_2PO_4$ (pH 7.4) ; 5 mM $FeSO_4$ and 2 mM luminol. $H_2O_2$ at final concentration of 5 mM was introduced through a dispencer into a cuvette following a background luminescence recording of the mixture of reagents. 5 to 10 ml of the test compounds (dissolved in water at the necessary concentration) were introduced into a cuvette. The total volume of the sample was 0.5 ml.
B. Superoxide anion-radical in a mixture of xantine and xantine oxidase [Afanas'ev I., Suslova T., Cheremisina Z. et al., Study of antioxidant properties of metal aspartates, Analyst (1995), Vol. 120, pp. 859-862].

The incubation medium comprised 5 mM $KH_2PO_4$ and 0.2 IU/ml xantine oxidase. Xantine at concentration 1 mM was introduced into sample through a dispencer. Luceginine (0.2 mM) was used in the system as a luminiscence sensitizer. The test compounds were introduced in a way similar to how they were introduced in the radical study.

Preliminary studies showed that the compounds of the present invention have no effect on xantine oxidase activity.

The CL of the systems described above was measured at 25˚C under pulse stirring (at the moment of reagents' introduction through the dispencer). CL signal indication was performed by its integration every 10 seconds for 5 minutes. CL flash recording duration after mixing the ingredients depended on a particular process kinetics. For each system, CL light sum (mV) was determined in the control samples without preparations (I-) and in samples comprising respective concentrations of the preparations (I+). To estimate the degree of CL inhibition (activation) the I+/I- ratio (in relative units) was found in the studied systems.

AOF formation and the effect of the test compounds on this process were recorded on the Luminometer-1251 instrument (LKB, Sweden).

A. Effect of the compounds of the present invention on the formation of hydroxyl radicals in the Fenton reagent.

When $H_2O_2$ was introduced into phosphate buffer comprising iron sulfate, a CL flash occured, which was caused by the formation of (predominantly) hydroxyl radicals in the reaction mixture. The flash had a short-term character-its maximum intensity being achieved in the 10th second and during the following 10-20 seconds luminiscence became dim and CL parameters decreased, down to background values.

Table 24 summarizes the data showing the effect of the compounds on hydroxyl radical generation in the Fenton reagent. The results show that the test compounds inhibit hydroxyl radical formation in the studied system.

Table 24

| Effect of the compounds of the present invention on hydroxyl radical formation | | | | |
|---|---|---|---|---|
| Compound No | Chemiluminiscence intensity (mV) $+\sigma_{n-1}$ / concentration of the test compounds | | | |
| | 1 mM | 0.1 mM | 0.01 mM | 1 μM |
| control | 7839 ± 171 | | | |
| XLIV | 2197 ± 311 0.28 (*) | 4625 ± 393 0.59 | 6444 ± 267 0.83 | 7747 ± 259 1.0 |
| XLV | 2195 ± 18 0.16 | 5800 ± 247 0.74 | 7443 ± 267 0.95 | 7666 ± 101 1.0 |
| control | 1120 ± 163 | | | |
| III | 164 ± 14 0.15 | 719 ± 71 0.64 | 972 ± 79 0.87 | 1100 ±84 0.98 |
| XI | 557 ± 52 0.50 | - | 1124 ± 113 | 1116 ± 90 |
| VII | 165 ± 7 0.15 | 11.58 ± 102 | - | - |
| IX | 225 ± 9 0.20 | 664 ± 47 0.59 | 1069 ± 72 0.95 | - |
| II | 175 ± 21 0.16 | 693 ± 42 0.62 | 772 ± 44 0.69 | 961 ± 42 0.86 |
| control | 4974 ±283 | | | |
| XII | 189 ± 13 0.04 | 575 ± 69 0.12 | 2829 ± 184 0.58 | 4438± 295 0.89 |
| VI | 257 ± 74 0.05 | 3110 ± 210 0.63 | 5014 ± 186 | - |
| XXI | 228 ± 72 0.05 | 3265 ± 184 0.66 | 4036 ± 186 0.81 | - |
| XIX | 626 ± 56 0.13 | 3377 ± 222 0.68 | 3846 ± 184 0.77 | 49089 ± 269 |
| XX | 536 ± 124 0.11 | 4018 ± 201 0.81 | 4205 ± 234 0.85 | - |
| control | 1332 ± 172 | | | |
| XII[a] | 615 ± 81 0.46 | 1246 ± 79 0.94 | - | - |
| (*) - relative effect: I+/I-, where I+ = CL intensity 9mV) in the presence of substance and I-= CVL intensity in the same sample without the test substance. | | | | |

B. Effect of the compounds of the present invention on superoxide anion-radical formation in the xantine-xantine oxidase system

The introduction of xantine into the medium containing xantine oxidase and lucegenine resulted in the appearance of a CL flash, which flash was at a maximum for 3-5 minutes and then a very slow reduction in chemiluminescence intensity began. The addition of the test compounds into the xantine-xantine oxidase mixture did not principally change

the CL response curve shape and only the maximum CL intensity values varied. Because of the "distension" of the kinetic curve, the chemiluminescence light sum was recorded during the first 3-5 minutes. This reflected the total amount of light quantums produced in the system till the maximum CL intensity values was achieved. As seen from the presented data (Table 25) the test compounds possess the ability to significantly inhibit the formation of the superoxide anion-radical.

Table 25

| Effect of the test compounds on formation of superoxide anion-radical | | | | |
|---|---|---|---|---|
| Compound No | **Chemiluminiscence intensity (mV) +$\sigma_{n-1}$ / concentration of the test compounds** | | | |
| | 1mM | 0.1 mM | 0.01 nM | 1 μM |
| control | 5616 ± 173 | | | |
| III | 4027 ± 683 0.72 | 5670 ± 379 | - | - |
| XI | 2263 ± 278 0.40 | 4621 ± 224 0.82 | - | - |
| VII | 1114 ± 51 0.20 | 4050 ± 291 0.72 | 5082 ± 278 0.91 | - |
| control | 5391 ± 195 | | | |
| II | 3143 ± 156 0.58 | 4753 ± 89 0.88 | 5229 ± 140 0.97 | - |
| control | 7290 ±128 | | | |
| XII | 1926 ± 33 0.26 | 4730 ± 139 0.65 | 6856 ± 209 0.94 | - |
| VI | 902 ± 15 0.12 | 5518 ± 131 0.76 | 7295 ± 106 | - |
| XXI | 4232 ± 146 0.58 | 6460 ± 166 0.89 | - | - |
| control | 6776: ± 150 | | | |
| XII[a] | 1955 ± 155 0.29 | 2188 ± 172 0.32 | 5471 ± 421 0.81 | 6168 ± 202 0.91 |
| (*) - relative effect: I+/I-, where I+ = CL intensity 9mV in the presence of substance and I- = CVL intensity in the same sample without the test substance. | | | | |

The results presented in examples 24-37 show that the compounds of the present invention possess a pronounced antihypoxic, antiallergic and antiinflammatory activity.

EXAMPLE 38

HEPATOPROTECTIVE ACTIVITY STUDY OF THE COMPOUNDS OF THE PRESENT INVENTION

Hepatoprotective properties of the compounds were studied using a model of subchronic liver lesion (hepatitis) with carbon tetrachloride.

70 mongrel female rats with an initial body mass of 190-200 g and fed a standard vivarium ration were used in experiment. Animals were divided into 7 groups (10 rats each):

Group 1 - control one; during the first four days of the experiment the animals were subcutaneously injected 0.2 ml

vaseline oil;
Group 2 - during the first four days the animals were subcutaneously injected 50% $CCl_4$ solution in vaseline oil at a rate of 0.1 ml per 100 g body mass;
Group 3 - Legalon in a starch gel was administered orally for 8 days, at a dose of 30 mg/kg against the background of $CCl_4$ administration;
Groups 4 and 5 - compound III, at doses of 50 and 500 μg/kg, resepectively, was administered in a way similar to group 3;
Groups 6 and 7 - compound XLIV, at doses 50 and 500 μg/kg, resepectively, was administered in a way similar to group 3.

The test compounds and Legalon were administered to animals 1 hour prior to $CCl_4$ administration. 24 hours after the last administration of the drugs, the animals were decapitated.
The hepatoprotective activity of the test compounds was estimated based on the folowing parameters:

1) in blood serum:

- activity of alanine aminotransferase (ALT) and aspartate aminotransferase (AST);
- total cholesterol (total CS);
- high density lipoprotein cholesterol (HDL-CS);
- low density lipoprotein cholesterol and very low density lipoprotein cholesterol (LDL-CS and VLDL-CS, respectively);
- triglycerides (TG);
- malonic dialdehyde (MDA);

2) in the liver:

- lipid composition: phospholipids (PL), free cholesterol (FCS), - triglycerides (TG);
- MDA.

The activity of the blood serum transaminases ALT and AST was assessed using the conventional Frenkel-Rightman method [Laboratory examination methods in the clinic, Ed. by Menshikov V.V. Moscow, Meditsina publishers (1969), 302 pp.].
The blood serum total-CS level was determined according to the Ilk method [Biochemical examination in the clinic. Ed. by Pokrovsky A.A. Moscow, Meditsina publishers (1969), pp. 300-302]. The HDL-CS level was measured in supernatant following heparine-manganese precipitation [Titov V.N., Brener E.D., Khaltaiev N.G., Zadoya A.A., Tvorogova M.G., A method and diagnostic significance of cholesterol level study in α-lipoproteins, Laboratornoye delo (Laboratory Matter) (1979), No 1, pp. 36-41] using the Ilk method [Biochemical examination in the clinic. Ed. by Pokrovsky A.A. Moscow, Meditsina publishers (1969), pp. 300-302]. LDL-CS and VLDL-CS levels were calculated according to the formula [Friedwald W.T., Levy R.I.,Fredrickson D.S., Estimation of the concentration of low-density lipoprotein cholesterol in plasma, without use of preparative ultracentrifuge, Clin. Chem. (1972), Vol. 18, pp. 499-502]:

$$\text{LDL–CS} = \text{total–CS} - (\text{HDL–CS} + \text{TG}/5),$$

where TG/5 corresponds to blood serum VLDL-CS level.
The method published by Rodionova et al [Rodionova L.P. Modification of blood serum triglyceride level determination method. Laboratornoye delo (Laboratory Matter) (1980), No 5, pp. 297-299] was used to determine the blood serum TG level.
The blood serum LP final product MDA was determined using the method published by Korobeinikova [Korobeinikova E.N., Modification of lipid peroxydation product determination in reaction with thiobarbituric acid, Laboratornoye delo (Laboratory Matter) (1989), No 7, pp. 8-10]. The concentration of TBA-active products was calculated using this regression equation:

$$C = 0.21 + 0.26D,$$

where C = concentration of TBA-active products (in nM MDA per 1 ml of serum) and D = index $D_{535}$ -$D_{580}$ (in optic density units).

Total hepatic lipids were extracted using the modified Folch method [Cates M. Technology of Lipidology, Moscow, Mir publishers (1975), pp. 74-76]. The quantative content of lipid fractions was assessed by a thin layer chromatography method (TLC) using the system of solvents hexane:diethyl ether:acetic acid in the ratio 80:20:2. Zones of individual lipid fractions were determined using a 10% alcohol solution of phosphoromolybdenic acid, and following elution they were spectrophotometrically analysed at 600 nm.

The hepatic MDA level was determined using the method published by Stalnaya et al [Stalnaya I.D., Garishvili T.G., Malonic dialdehyde determination method using thiobarbituric acid. In: Modern methods in biochemistry, Moscow, Meditsina publishers (1977), pp. 66-69]. MDA quantity was calculated using the molar extinction coefficient value of a stained trimethyl complex formed by MDA with two TBA molecules:

$$E = 1.56 \times 10^5\ cm^{-1} \times M^{-1}$$

The experimental results (Table 26) give evidence of the fact that in animals treated with $CCl_4$ a significant hyperenzymemia was noted. Administration of the compounds of the present invention was accompanied by normalization of blood serum ALT and AST activity, the maximum hepatoprotective effect being pronounced in administering the second substance.

Table 26

| Effect of the compounds of the present invention on the change in blood serum transaminase activity in animals with experimental hepatitis | | | |
|---|---|---|---|
| Groups of animals | Daily dose, mg/kg | ALT, nM/dl | AST, nM/dl |
| Control, intact animals | - | 674 ± 35 | 554 ± 27 |
| $CCl_4$ - 4 days | - | 789 ± 30* | 620 ±11* |
| Legalon + $CCl_4$ | 30.0 | 622 ± 36** | 531 ± 22** |
| Compound III+$CCl_4$ | 0.50 | 609 ± 42** | 522 ± 33** |
| Compound III + $CCl_4$ | 0.05 | 624 ± 31** | 478 ± 26*** |
| Compound XLIV + $CCl_4$ | 0.50 | 551 ± 28** | 475 ± 19*** |
| Compound XLIV + $CCl_4$ | 0.05 | 599 ± 18** | 470 ± 17** |
| *- significance of differences in relation to the control group data,<br>*=p<0.05<br>**, *** = significance of differences the data of the group of animals with experimental hepatitis ($CCl_4$): ** = p<0.01; *** = p<0.001. | | | |

The data obtained from analysis of the blood serum lipid composition in experimental animals showed (Table 27) that toxic liver lesion is accompanied by hypertriglyceridemia. Some peculiarities of cholesterol distribution among lipoprotein fractions under the effect of $CCl_4$ were noted, namely (along with a rise in the total cholesterol and VLDL-CS levels) the HDL-CS level rase and the LDL-CS level dropped. Against the background of administering Legalon and the test compounds, trends of a lowering of the total cholesterol level, and normalization of LDL-CS, VLDL-CS and HDL-CS levels were observed.

Table 27

| Effect of the compounds of the present invention on the change in blood serum levels of cholesterol and triglycerides in animals with experimental hepatitis | | | | | |
|---|---|---|---|---|---|
| Group of animals | Cholesterol level, mg/100 ml | | | | TG level, mg/100 ml |
| | total-CS | HDL-CS | LDL-CS | VLDL-CS | |
| Control, intact animals | 77.6 ± 2.9 | 47.3 ± 2.1 | 18.4 ± 0.7 | 11.7 ± 0:4 | 59.5 ± 2.5 |

(continued)

| Effect of the compounds of the present invention on the change in blood serum levels of cholesterol and triglycerides in animals with experimental hepatitis | | | | | |
|---|---|---|---|---|---|
| Group of animals | Cholesterol level, mg/100 ml | | | | TG level, mg/100 ml |
| | total-CS | HDL-CS | LDL-CS | VLDL-CS | |
| $CCl_4$ - 4 days | 93.5 ± 6.0* | 60.8 ± 4.6* | 12.1 ± 1.1 | 20.4 ± 1.6* | 102.9 ± 8.1 * |
| Legalon + $CCl_4$ | 90.9 ± 3.4 | 54.8 ± 2.5 | 20.1 ± 1.1** $p<0.01$ | 15.7 ± 1.1** $p<0.05$ | 78.0 ± 6.2** $p<0.05$ |
| Compound III, 0.5 μg/kg+ $CCl_4$ | 87.0 ± 2.6 | 59.3 ± 2.6 | 12.0 ± 0.6 | 15.8 ± 1.0** $p<0.05$ | 78.6 ± 5.4** $p<0.05$ |
| Compound III, 0.05 μg/kg + $CCl_4$ | 89.5 ± 3.8 | 52.3 ± 2.9 | 19.6 ± 1.4** $p<0.01$ | 17.7 ± 2.0 | 88.0 ± 10.2 |
| Compound XLIV, 0.5 μg/kg +$CCl_4$ | 87.2 ± 6.5 | 57.5 ± 5.1 | 17.1± 1.0** $p<0.01$ | 12.4 ± 0.7** $p<0.001$ | 62.4 ± 3.9** $p<0.001$ |
| Compound XLIV, 0.05 μg/kg+$CCl_4$ | 85.3 ±3.9 | 49.6 ± 2.5 | 21.9 ± 1.1** $p<0.001$ | 13.9 ± 1.1** $p<0.01$ | 68.3 ± 5.7 $p<0.001$ |
| * = significance of differences compared with the control group data,<br>** = significance of differences compared with the data of the group of animals with experimental hepatitis. | | | | | |

The results presented in table 28 show that $CCl_4$ administration resulted in a lowering of the cholesterol level. The test compounds normalized the FCS level and lowered the TG level. At the same time, a rise in phospholipid fraction was observed when the test substances were administered.

Table 28

| Effect of the compounds of the present invention on the change in hepatic lipid composition in rats with experimental hepatitis | | | |
|---|---|---|---|
| Group of animals | Lipid composition (in percent of total lipids) | | |
| | Phospholipids | FCS | Triglycerides |
| $CCl_4$ - 4 days | 21.3 ± 0.9 | 23.6 ± 1.2 | 23.9 ± 1.2 |
| Legalon + $CCl_4$ | 19.7 ± 1.1 | 15.0 ± 0.4** $p<0.01$ | 44.1 ± 1.9 |
| Compound III, 0.5 μg/kg+$CCl_4$ | 23.8 ± 1.0** $p<0.05$ | 18.9 ±0.7 | 35.3 ±0.7 |
| Compound III, 0.05 μg/kg+$CCl_4$ | 24.4 ± 0.7** $p<0.05$ | 20.3 ± 0.8** $p<0.05$ | 35.5 ± 1.1 |
| Compound XLIV, 0.5 μg/kg+$CCl_4$ | 26.1 ± 0.6** $p<0.001$ | 20.6 ± 0.8** $p<0.05$ | 33.9 ± 1.0** $p<0.05$’ |

(continued)

| Effect of the compounds of the present invention on the change in hepatic lipid composition in rats with experimental hepatitis | | | |
|---|---|---|---|
| Group of animals | Lipid composition (in percent of total lipids) | | |
| | Phospholipids | FCS | Triglycerides |
| Compound XLIV, 0.05 μg/kg + $CCl_4$ | 22.8 ± 0.9 | 18.0 ± 0.8 | 39.9 ± 1.7 |
| * = significance of differences in relation to the control group;<br>** = significance of differences in relation to the group of animals with experimental hepatitis ($CCl_4$). | | | |

Table 29 summarizes the data on changes in blood serum and hepatic final LP product MDA level in injury, caused by $CCl_4$ and the effect of administering the test compounds. Toxic liver injury was accompanied by a rise in MDA levels in both serum and liver. The treatment of animals with Legalon and the compounds of the present invention resulted in normalization of the MDA level in the tissues examined.

Table 29

| Effect of the compounds of the present invention on change in blood serum and hepatic final lipid peroxidation product MDA level in rats with experimental hepatitis | | | |
|---|---|---|---|
| Groups of animals | Dose of compounds, mg/kg | Serum MDA, nM/l | Hepatic MDA, nM/1 |
| Control, intact animals | - | 5.60 ± 0.60 | 3.49 ± 0.16 |
| $CCl_4$ - 4 days | - | 7.03 ± 0.38<br>p<0.05* | 5.98 ± 0.29*<br>p<0.001 |
| Legalon + $CCl_4$ | 30.0 | 5.58 ± 0.15** | 4.17 ± 0.31**<br>p<0.001 |
| Compound III + $CCl_4$ | 0.50 | 5.77 ± 0.15**<br>p<0.05 | 4.60 ± 0.52**<br>p<0.05 |
| Compound III + $CCl_4$ | 0.05 | 5.86 ± 0.70 | 5.00 ± 0.15**<br>p<0.05 |
| Compound XLIV + $CCl_4$ | 0.50 | 4.72 ± 0.57**<br>p<0.01 | 3.51 ± 0.30**<br>p<0.001 |
| Compound XLIV + $CCl_4$ | 0.05 | 5.82 ± 0.38**<br>p<0.05 | 4.47 ± 0.45**<br>p<0.05 |
| * = significance of differences with the control group results;<br>** = significance of differences with the results obtained in the group of animals with experimental hepatitis ($CCl_4$). | | | |

Thus, the test compounds of the present invention possess a pronounced antioxidant and lipid-regulating effect, which is comparable with the effect of the reference drug Legalon, and by a number of parameters the test compounds exceeded Legalon. At the same time it should be noted that the test compounds were administered at doses two to three orders lower than the Legalon doses.

EXAMPLE 39

HYPOLIPIDEMIC ACTIVITY OF THE COMPOUNDS OF THE PRESENT INVENTION

The hypolipidemic activity of the compounds of the present invention was studied using the model of experimental hyperlipidemia described by Arichi et al, [Arichi H., Kumura H.O., Effects of stibens compounds of roots of polygonum cuspidatium on the lipid metabolism, Chem Pharm. Bull. (1982), Vol. 30, No 5, pp. 1766-1767], with mongrel male rats with an initial body mass of 220-250 g. Following a standard ration for 10 days the rats, were intragastrally

administered an oil suspension comprising 10% cholesterol and 1% cholic acid (at a rate 1 ml suspension per 100 g body mass). Each group of animals included 10 rats. The test compounds III, V, VI, VII, VIII, IX, X, XIII, XXVIII, XLIV, XLVII, L and LI were orally administered to animals at doses 50 and 500 μg/kg during the last four days of the experiment. As a reference drug, nicotinic acid was used, administered at a dose of 10 μg/kg to animals for 10 days at the background of atherogenic load. Blood samples were taken for analysis 18 hours after the last administration of the test substances, during which period food was taken away from the rats.

The following parameters were determined: total cholesterol (total CS); high density lipoprotein cholesterol (HDL-CS); low density lipoprotein cholesterol and very low density lipoprotein cholesterol (LDL-CS and VLDL-CS); and triglycerides (TG). Blood serum CS level was determined according to the Ilk method [Biochemical examination in the clinic. Ed. by Pokrovsky A.A. Moscow, Meditsina publishers (1969), pp. 300-302]. HDL-CS level was measured in supernatant following heparine-manganese precipitation of LDL+VLDL [Titov V.N., Brener E.D., Khaltaiev N.G., Zadoya A.A., Tvorogova M.G., A method and diagnostic significance of cholesterol level study in α-lipoproteins. Laboratomoye delo (Laboratory Matter) (1979), No 1, pp. 36-41]. LDL-CS level determination was carried out by calculation according to the formula described by Friedwald et al [Friedwald W.T., Levy K.J., Leus R. Fat transport in lipoproteins: an integrated approach to mechanism and disorders, New Engl. J. Med. (1967), Vol. 276, p. 32].

To assess the effect of the test compounds on the ratio of atherogenic and antiatherogenic blood serum lipoproteins, cholesterol index ($K_{cs}$) was calculated according to the formula described by Klimov et al [Klimov A.N., Nikulcheva N.G., Lipoproteins, dylipoproteinemias and atherosclerosis. Moscow, Meditsina publishers (1984), 165 pp.].

Blood serum TG level was determined using a conventional method [Friedwald W.T., Levy R.I., Fredrickson D.S. Estimation of concentration of low-density lipoprotein cholesterol in plasma, without use of preparative ultracentrifuge. Clin. Chem. (1972), Vol. 18, pp. 499-502].

The administration of fat a suspension to animals was accompanied by a significant rise in the blood serum CS level, at the expense of the atherogenic fractions of lipoproteins (LDL and VLDL) with a background of a decrease in the level of the antiatherogenic fraction (HDL). A three-fold rise in atherogeneity index was observed. A significant rise in blood serum TG was also noted (Table 30). When the test compounds were administered to animals (Table 31), at both doses a drop of 10-26 percent in the total cholesterol level was noted as compared to the animals with atherogenic load, at the expense of LDL-CS and VLDL-CS lowering, and a rise of 20-100 percent in HDL-CS was typically noted. The drop in cholesterol atherogeneity index $K_{cs}$ was practically down to the control values, and was observed with administration of all the test compounds. Blood TG level in animals dropped to the control values upon administration of the test compounds.

Table 30

| Effect of nicotinic acid on the level of cholesterol and triglycerides in blood serum of experimental animals | | | |
|---|---|---|---|
| Groups of animals Parameters | Control 1 | Atherogenic diet 2 | Atherogenic diet + nicotinic acid 3 |
| Total-CS | 68.8 ± 2.3 | 96.9 ± 10.8*[1] | 65.7 ± 3.5 |
| HDL-CS | 37.5 ± 1.0 | 27.1 ± 1.3 | 42.1 ± 4.2***[1,2] |
| LDL-CS + VLDL-CS | 30.9 ± 0.9 | 68.7 ± 4.8***[1] | 25.7 ± 2.8***[2] |
| Triglycerids | 84.9 ± 3.2 | 146.5 ± 12.9***[1] | 89.4 ± 6.2***[2] |
| $K_{cs}$ | 0.835 | 2.58***[1] | 0.56***[2] |
| * = significance of differences, * = p<0.05; ** = p<0.01; *** =p<0.001. The superscript numbers signify the groups in relation to which differences are significant. | | | |

As an example of the effect of the test compounds on change in blood lipid composition, Table 31 summarizes the effect results of some of the compounds of the present invention on blood lipid composition in animals which were given an atherogenic load. The administration of these compounds was shown to normalize all the examined parameters, practically to the control values.

Table 31

| Effect of the compounds of general formula (I) on blood levels of cholesterol and triglycerids in experimental animals | | | | | |
|---|---|---|---|---|---|
| Parameters<br>Groups | Total CS | HDL-CS | LDL-CS+ VLDL-CS | Triglycerides | $K_{cs}$ |
| Control-1 | 72,2±2,9 | 29,6±2,6 | 42,6±2,7 | 104,7±8,0 | 1,44±0,1 |
| Atherogenic diet | 93,3±3,7*** | 21,3±1,5* | 72,0±4,0*** | 261,7±11,6*** | 3,38±0,2*** |
| Compound VII, 50 μg/kg | 72,3±4,9**A | 26,0±1,9 | 46,3±3,2***A | 250,6±12,0 | 1,78±0,12***A |
| Compound VII, 500 μg/kg | 78,1±3,3* | 22,3±2,2 | 55,8±3,9**A | 251,4±12,71 | 2,5±0,17**A |
| Compound IX, 50 μg/kg | 78,4±4,4*A | 29,7±3,6*A | 48,7±4,4**A | 24,5±20,2 | 1,64±0,16***A |
| Compound IX, 500 μg/kg | 72,1±3,9**A | 20,1±1,6 | 52,0±3,4**A | 201,8±8,1***A | 2,59±0,17**A |
| | | | | | |
| Compound XIII, 50 μg/kg | 76,3±5,5*A | 23,9±1,9 | 52,4±3,9**A | 201,2±8,7***A | 2,19±0,16***A |
| Compound XIII, 500 μg/kg | 69,3±3,7***A | 27,8±2,3*A | 41,5±2,9***A | 190,0±11,7***A | 1,49±0,1***A . |
| Compound XXVIII, 50 μg/kg | 85,4±4,0 | 34,6±4,6*A | 50,8±4,6**A | 157,5±12,4***A | 1,47±0,13***A |
| Compound XXVIII, 500 μg/kg | 80,7±4,8 | 47,0±6,9*A | 33,7±3,4***A | 204,1±22,1*A | 0,72±0,07***A |
| Compound X, 50 μg/kg | 87,9±7,0 | 31,8±3,3*A | 56,1±5,0*A | 218,7±17,9 | 1,76±0,16***A |
| Compound X, 500 μg/kg | 71,3±2,3***A | 38,4±4,5**A | 32,9±2,5***A | 179,2±18,3**A | 0,86±0,06***A |
| Compound VI, 50 μg/kg | 86,0±5,2 | 35,7±5,6*A | 50,3±5,5**A | 141,2±16,1***A | 1,41t0,16***A |
| Compound VI, 500 μg/kg | 70,8±3,7***A | 45,8±3,5***A | 25,0±1,6***A | 127,2±7,1***A | 0,55±0,04***A |
| Control-2 | 82,9±5,1 | 49,3±3,7 | 33,9±2,9 | 84,3±5,7 | 0,68±0,05 |
| Atherogenic diet | 100,6±4,3* | 33,8±2,5** | 66,8±3,3*** | 157,9±9,0*** | 1,98±0,1*** |
| Compound XLVII50 μg/kg | 86,5±3,9*A | 44,1±1,9**A | 42,4±1,7***A | 90,0±7,7***A | 0,96±0,04***A |
| Compound XLVII 500 μg/kg | 90,0±2,4 | 40,8±4,8 | 50,8±3,6**A | 116,7±10,8*A | 1,27±0,09 |
| Compound III, 50 μg/kg | 87,8±2,8*A | 46,1±2,8**A | 41,7±3,1***A | 136,4±9,7 | 0,9±0,04***A |
| Compound III, 500 μg/kg | 86,2±5,6 | 53,1±6,2*A | 33,1±2,9***A | 90,0±6,7***A | 0,62±0,05***A |
| Compound XLIV, 50μg/kg | 84,7±3,0**A | 47,3±1,1***A | 37,4±1,1***A | 85,0±6,3***A | 0,79±0,02***A |
| Control-3 | 94,1±1,2 | 45,6±2,5 | 48,5±2,4 | 112,1±10,6 | 1,06±0,03 |

(continued)

| Effect of the compounds of general formula (I) on blood levels of cholesterol and triglycerids in experimental animals | | | | | |
|---|---|---|---|---|---|
| Parameters / Groups | Total CS | HDL-CS | LDL-CS+ VLDL-CS | Triglycerides | $K_{cs}$ |
| Atherogenic diet | 107,7±6,1* | 27,6±1,5*** | 80,1±3,1*** | 207,1±16,0 | 2,90±0,16***A |
| Compound V, 50 μg/kg | 103,4±2,8 | 40,0±3,1**A | 63,3±1,9*A | 181,2±12,2 | 1,58±0,09***A |
| Compound LI, 50 μg/kg | 99,1±4,5 | 39,8±1,6**A | 59,3±3,5*A | 132,1±17,4**A | 1,49±0,07***A |
| Compound VIII, 50 μg/kg | 98,8±7,2 | 31,7±2,4 | 67,1±3,1*A | 136,8±18,2*A | 2,12±0,16**A |
| Compound L, 50 μg/kg | 94,1±2,1 | 31,0±2,1 | 63,1±2,4*A | 141,6±12,6**A | 2,03±0,09***A |
| * = differences are significant in relation to the control groups,<br>*A = differences are significant in relation to the groups of animals which were given atherogenic ration,<br>* = p<0.05; ** = p<0.01; *** = p<0.001. | | | | | |

Thus, the changes in blood serum lipid composition upon administering of the test compounds to experimental animals are comparable with the hypolipidemic effect of the reference drug nicotinic acid (table 30).

EXAMPLE 40

EXPERIMENTAL STUDY OF HYPOGLYCEMIC ACTIVITY OF THE COMPOUNDS OF THE PRESENT INVENTION

The experiments were carried out on Wistar male rats weighing 250-300 g. Experimental diabetes was induced with a single intravenous injection of streptozotocine (the Synthez cooperative enterprise at the IMBG of the Academy of Sciences of Ukraine), at a dose of 42 mg/kg, to rats which were preliminarily starved for 24 hours, with access to food provided immediately following the injection. Rats were selected for experiments 2 weeks after diabetes induction with glycemia 120-180 mg/dl. Each group included 12 animals.

The compounds were intragastrally administered to intact animals and to rats with streptozotocine diabetes for four days at daily doses of 50 μg/kg and 500 μg/kg, in a water solution at a rate of 1 ml per 200 g of body mass. Intact animals were given compounds III and XLIV, and rats with induced diabetes were given compound XLIV. Blood glucose level determination was done at the day of experiment, then animals were administered the test compounds and were deprived of food. The control animals received a corresponding volume of water. The effect was estimated based on the change in blood glucose level at 2 and 5 hours. Then animals received forage and 24 hours after the last drug administration blood was again sampled for examination -0.1 ml of blood was collected from the tail vein. The glucose level was determined using the o-toluidine method.

The glucose level was calculated in mg/dl, using standard glucose solutions. Then the degree of change in glucose level in relation to the initial level was determined for each animal, with its expression in percent from the initial level. A final calculation was performed for each group of animals. Each group included 10 animals.

**Table 32**

**Effect of two test compounds on blood glucose level in intact rats**

| Conditions of experiment / Group | Blood glucose level (mg/dl) | | | |
|---|---|---|---|---|
| | 2 hours before | 2 hours after | 5 hours after | 24 hours after |
| Control – 1 | 82.6 ± 4.1 | 58.6 ± 3.2 | 60.4 ± 2.3 | 83.8 ± 5.2 |
| Compound XLIV, 50 μg/kg | 81.2 ± 3.8 | 60.8 ± 4.0 | 64.0 ± 3.1 | 79.9 ± 5.2 |
| Compound XLIV, 500 μg/kg | 80.8 ± 2.7 | 64.8 ± 4.2 | 65.9 ± 3.9 | 83.7 ± 4.0 |
| Control – 2 | 72.6 ± 4.6 | 56.8 ± 3.8 | 65.4 ± 3.3 | 75.0 ± 3.2 |
| Compound III, 50 μg/kg | 84.2 ± 6.1 | 58.5 ± 5.9 | 70.8 ± 5.5 | 66.5 ± 3.3 |
| Compound III, 500 μg/kg | 71.3 ± 4.0 | 69.9 ± 2.8*[2] | 72.6 ± 1.5*[2] | 73.1 ± 3.4 |

* = significance of differences in relation to the control – 2

* = $p<0.05$

The results presented in Table 32 give evidence of the fact that compound XLIV has no effect on the curve of blood glucose level changes in intact animals following a short-term fasting period, while compound III administered at a dose of 500 μg/kg prevented a drop in blood glucose level in intact rats two hours after food deprivation.

Table 33

Effect of compound III of the present invention on blood glucose level in animals with experimental streptozotocine diabetes

| Group of animals / Blood glucose level | Control 1 | Compound III 50 μg/kg 2 | Compound III 500 μg/kg 3 |
|---|---|---|---|
| Initial level, mg/dl | 142.1 ± 9.9 | 146.9 ± 8.2 | 145.2 ± 10.6 |
| mg/dl in 2 hours | 114.9 ± 9.3 | 94.4 ± 5.4 | 98.8 ± 5.6 |
| percent from the initial level | 80.9 ± 5.1 | 65.4 ± 4.3* | 67.6 ± 3.6* |
| mg/dl in 5 hours | 80.5 ± 6.1 | 64.1 ± 5.0 | 65.1 ± 1.1 |
| percent from the initial level | 56.7 ± 5.0 | 43.9 ± 4.9 | 44.9 ± 4.5 |
| mg/dl in 24 hours | 153.9 ± 3.8 | 122.2 ± 9.2 | 143.4 ± 10.9 |
| percent from the initial level | 108.4 ± 7.1 | 83.7 ± 6.6 | 98.9 ± 10.2 |

* = a significant difference from the control group data, $p<0.05$.

The data presented in Table 33 shows that intragastral administration of compound III for four days at daily

doses 50 and 500 μg/kg results in a significant drop in blood glucose level two hours after the last drug administration to animals. A hypoglycemic antidiabetic effect was maintained 5 hours post administration, however, this effect was less pronounced.

Thus, the compounds of the present invention either have no effect or stabilize the blood glucose level in intact animals, and they possess a glucose-lowering activity in rats with streptozotocine diabetes, which is equally pronounced with administration at doses of both 50 μg/kg and 500 μg/kg.

The results presented in examples 38-40 give evidence of the fact that the compounds of the present invention possess a pronounced hepatoprotective effect. This effect compounds is explained by the fact that when they are administered into the body there occurs a functional rearrangement of many central body systems which participate in maintaining homeostasis.

EXAMPLE 41

EFFECT OF THE COMPOUNDS OF THE PRESENT INVENTION ON LEWIS PULMONARY CARCINOMA GROWTH

Experiments were carried out on male mice with an initial body mass of 18-20 g. Each group included 10 animals. Tumor transplantation was performed according to the standard technique [Experimental estrimation of antitumor drugs in the U.S.S.R. and the U.S.A. edited by E.P.Sof'ina, A.B.Syrkin, A.Goldin, A.Kline. Moscow, Meditsina publishers (1980), 296, pp.]. The administration of compounds II, III**,** IX, X, XH, XIV, XV, XVI, XVII (comparative example) XIX, XX, XXI, XXII and XLVII in drinking water was commenced 24 hours following transplantation and continued till the end of the experiment. The effect of the compounds on Lewis rat pulmonary carcinoma (LLC) was estimated on day 12 of the experiment based on the change in tumor volume expressed in $mm^3$.

The data presented in Table 34 show that all the tested compounds inhibit LLC growth, though to a different extent.

Table 34

| Effect of the compounds of the present invention on Lewis pulmonary carcinoma growth | | |
|---|---|---|
| Administered compound | Tumor volume ($mm^3$) | Growth inhibition percent |
| control, intact animals | 1492 ± 230 | - |
| XXII | 985 ± 179 | 34 |
| XLVII | 826±119* | 45 |
| Control, intact animals | 2633 ± 275 | - |
| **III** | 1249± 168** | 53 |
| II | 1025±150** | 61 |
| IX | 2372 ±323 | 10 |
| X | 1221 ± 209** | 54 |
| XII | 1299 ± 145** | 51 |
| XVII | 1367 ± 233** | 48 |
| Control, intact animals | 1373 ± 114 | - |
| XXI | 1023 ± 107 | 25 |
| XIX | 994 ± 173 | 28 |
| XX | 1148 ± 112 | 16 |
| Control, intact animals | 2436 ± 260 | - |
| XVI | 1076 ± 69** | 56 |
| XIV | 779 ± 94** | 68 |
| XV | 889 ± 67** | 63 |
| *= significance of differences in relation to the respective control, * = p<0.02; ** = p<0.01 | | |

EXAMPLE 42

EFFECT OF THE COMPOUNDS OF THE PRESENT INVENTION ON LEWIS PULMONARY CARCINOMA METASTASIZATION

A. Antimetastatic activity study of the compounds of the present invention.

The study was carried out on $BDF_1$ female mice with an initial body mass of 25-30 g. Each group included 15 animals. 0.3 ml of LLC tumor tissue suspension was intramuscularly injected, to the mice. Henks solution was used as a solvent. The test compounds II, III, VI, IX, X, XII, XXIII and XLIV were dissolved in water and orally administered to animals for 24 days, at dose of 50 and 500 μg/kg,in 0.2 ml of solvent, beginning from day two following tumor transplantation. At day 25 of the experiment the animals were sacrified and their lungs were resected and fixed in Bouen solution using the MBS-9 microscope at a magnification of 8 x 2. 24 hours following fixation, pulmonary metastatic colonies were calculated. Metastasizing index calculation was carried out in accordance with the methodological recommendations [Methodological recommendations on preclinical study of agents possessing ability to inhibit metastasizing process and to enhance efficacy of cytostatic therapy of malignant tumors. Moscow (1992) 13 pp.].

The test compounds were found to inhibit the spontaneous metastasizing process of the transplantable LLC tumor in the presence of a primary tumor node of different degree (by from 20 to 70 percent). Table 35 summarizes the results demonstrating the high antimetastatic activity of the test compounds.

Activity criterion for antimetastatic drugs is their ability to inhibit metastasizing by 35 percent.

Table 35

**Antimetastatic properties of the compounds of the present invention in oral administration to $BDF_1$ female mice with LLC**

| Parameters / Group | Mean number of metastases | Metastasizing inhibition percent |
|---|---|---|
| Control – 1 | 52.2 ± 5.1 | – |
| Compound III, 500 µg/kg | 30.1 ± 2.7**[1] | 42.0 |
| Compound III, 50 µg/kg | 38.5 ± 6.3**[1] | 26.0 |
| Control – 2 | 46.9 ± 8.5 | – |
| Compound XLIV, 50 µg/kg | 42.4 ± 4.5 | 10.0 |
| Compound XLIV, 500 µg/kg | 31.9 ± 3.8*[2] | 32.0 |
| Compound II, 50 µg/kg | 44.3 ± 8.0 | 5.5 |
| Compound II, 500 µg/kg | 32.9 ± 4.8*[2] | 30.0 |
| Compound VI, 50 µg/kg | 35.5 ± 4.5*[2] | 24.0 |
| Compound VI, 500 µg/kg | 39.0 ± 14.0 | 17.0 |
| Control – 3 | 50.8 ± 6.9 | – |
| Compound IX, 500 µg/kg | 38.5 ± 5.7*[3] | 24.0 |
| Compound X, 500 µg/kg | 34.6 ± 7.2*[3] | 30.8 |
| Compound XII, 500 µg/kg | 35.5 ± 4.6*[3] | 30.1 |
| Compound XXIII, 500µg/kg | 31.5 ± 5.8*[3] | 37.0 |

* = significance of differences in relation to the respective control; * = $p<0.05$; ** = $p<0.01$

**B. Compound antimetastatic activity estimation under the conditions of surgical resection of the primary tumor node**

An important , property of the compounds of the present invention is their ability to manifest pharmacological activity on the model with resection of the primary tumor node and not to lower the therapeutic efficacy of respective antitumor drugs. This property was demonstrated by experiments using a model wherein a spontaneously metastasizing LLC tumor is transplanted into the paw pads of C57BL/6 female mice weighing 20-25 g. Surgical resection of the primary tumor, node was performed 13 days following tumor transplantation. The test anitumor compounds were administered according to two dosage regimens:

Dosage regimen 1 - the animals were administered compound III from day 1 till day 13 of the experiment, at a dose of 500 µg/kg (group 1); cyclophosphane was administered intraperitoneally twice before operation, with a 96 hour interval, at a dose of 100 µg/kg (group 2); a combined administration of compound III and cyclophosphane was administered in a similar manner to the substances in groups 1 and 2 (group 3). The number of metastases was calculated at day 29 of the experiment.

Dosage regimen 2 - the animals were administered compound III from day 14 of the experiment (commencement of administration 24 hours post operation) till day 28 (group 4); cyclophosphane was administered twice post operation, with a 96 hour interval, at a dose of 100 µg/kg (group 5); a combined administration of compound III and Cyclophosphane was administered in a similar manner to the substances of groups 4 and 5 (group 6). The antimetastic efficacy of the drugs was considered at day 29 of the experiment.

The experimental results are presented in Table 36. As seen from these results, compound III manifested antimetastatic activity in the model, with surgical resection of the primary tumor node, and in combined administration with cyclophosphane following tumor resection, potentiation of the pharmacological effect was observed as well as summation of the tested phenomenon in administering both drugs prior to surgical resection of tumor.

Table 36

| Antimetastatic activity parameters of compound III under the conditions of surgical resection of the primary tumor node and in combined administration with cyclophosphane | | | | |
|---|---|---|---|---|
| Experimental group | Drugs | Metastasizing rate (%) | Mean number of metastases | Metastasizing inhibition percent |
| Administration of drugs prior to operation according to dosage regimen 1 | | | | |
| Control | Normal saline | 100 | 34.8 ± 4.9 | |
| Group 1 | compound III | 100 | 32.0 ±11.8 | 8.0 |
| Group 2 | Cyclophosphane | 100 | 19.5 ± 5.1* | 43.9 |
| Group 3 | Compound III + | 100 | 16 ± 2.4*** | 52.8 |
| Administration of drugs post to operation according to dosage regimen 2 | | | | |
| Control | Normal saline | 100 | 37.1 ± 13.2 | |
| Group 4 | compound III | 100 | 28.6 ± 8.5 | 22.9 |
| Group 5 | Cyclophosphane | 100 | 18.0 ± 4.3* | 51.5 |
| Group 6 | Compound III + Cyclophosphane | 100 | 6.7 ± 0.9* | 81.9 |

B.A comparative estimation of antimetastatic sactivity of the compound III substance and its tabletted dosage form

To estimate the therapeutic efficacy of compound III in a tabletted dosage form (0.2 g tablets 6 mm in diameter comprising 17 mg substance) studies were carried out on the model of spontaneous LLC carcinoma metastasizing.

Experiments were carried out on C57BL/6 female mice weighing 20-25 g. Experimental groups included 8 animals and the control groups had 11 mice. The substance form and tabletted dosage form of compound III were administered, orally at a dose of 500 μg/kg (where 500 μg is the amount of the active agent), daily from day 1 till day 10 of the experiment. The experiment lasted for 24 days.

The experimental results are presented in Table 37. They give evidence of the fact that the parameters of antimetastatic activity of the substance and tabletted dosage forms are practically similar.

Table 37

| Comparative therapeutic efficacy study of compound III substance and dosage form | | | | |
|---|---|---|---|---|
| Groups | Antimetastatic activity parameters | | | |
| | Metastasizing rate in percent | Tumor mass (g) | Mean number of metastases | Metastasizing inhibition percent |
| Control | 100 | 13.5 ± 0.9 | 42.0 ± 7.5 | |
| Substance | 100 | 12.9 ± 0.9 | 22.5 ± 0.9* | 46.4 |
| Dosage form | 100 | 12.9 ± 0.6 | 18.8 ±2.7** | 55.2 |
| *,*** - significance of differences in relation to the control,<br>* - p<0.05; * * *- p<0.001. | | | | |

EXAMPLE 43

STUDY OF THE EFFECT OF THE COMPOUNDS OF THE PRESENT INVENTION ON RESISTANCE OF ANIMALS TO MICROBIAL INFECTIONS

The effect of the compounds of the present invention on the change in the average lifetime of experimental animals infected with Salmonella spp.

The experiments were carried out on mongrel white mice of both sexes weighing 18-22 g. Compounds III, XLIV and the reference drug sodium nucleinate were administered orally for 3 days prior and post infection (i.e. a total of 6 times with a dosage regimen -3, -2, -1, 0, 1, 2, 3, where 0 is infection day). A 24-hour Salmonella spp. culture grown on Hottinger agar was used for infecting. Normal saline solution was used to prepare a cellular suspension. The suspension of Salmonella spp. cells was administered subcutaneously at a dose of 5 x $10^8$ cells per mouse in 0.5 ml.

The test compounds were administered at a dose of 500 μg/kg and the reference drug sodium nucleinate at a dose of 50 μg/kg, in 0.5 ml of normal saline solution.

At a dose 500 μg/kg the test compounds significantly increased the average lifetime of mice infected with Salmonella spp. (Table 38), exerting a protective effect under strict experimental conditions (a hundred percent lethality in the control group). A significant rise in the survival rate of mice administered compound III was observed day 2 of the experiment as compared with the control group, and by day 1 of the experiment for animals which were administered compound XLIV. At the same time it was established that the distribution character of lethal outcomes in animals of the control group was abnormal, while as affected by compounds III and XLIV the distribution became normal. This confirms the efficacy of the tested compounds.

The protective effect of the test compounds in relation to bacterial infection was comparable to the effect of sodium nucleinate, which is a known promoter of immune responses, including phagocytosis [Lazareva D.L., Alekhin E.K. Immunity stimulants (1985), 286 pp.]. An efficient dose of the test compounds was two orders lower than the corresponding dose for the reference drug.

Thus, the compounds of the present invention possess a protective effect against microbial infection.

**Table 38**

**Effect of the test compounds on resistance of mice to Salmonella spp.**

| Group of animals / Days following infection | Number of dead mice | | | | |
|---|---|---|---|---|---|
| | Control 1 | Compound III | Sodium nucleinate | Control 2 | Compound XLIV |
| Day 1 | 1 | 1 | – | 6 | 3*2 |
| Day 2 | 4 | 1*1 | – | – | – |
| Day 3 | 1 | 1 | 3 | – | 1 |
| Day 4 | 1 | 2 | 3 | 2 | – |
| Day 5 | 1 | 1 | 1 | 1 | 5 |
| Day 6 | 2 | 2 | 3 | – | 2 |
| Day 7 | – | 2 | – | 1 | 1 |
| Day 8 | – | – | 1 | – | – |
| MLT (days) | 3.3 ± 0.58 | 4.5 ± 0.65* | 4.7 ± 0.48* | 2.6 ± 0.71 | 4.0 ± 0.73* |

MLT = mean lifetime.

* = significance of differneces in relatrion to the control group of animals; * = p<0.05.

The superscript numbers signify the control groups in relation to which the differences are significant.

EXAMPLE 44

ANTIVIRAL ACTIVITY OF THE COMPOUNDS OF THE PRESENT INVENTION

A. Protective effect of compound XLIV in infection caused by encephalomyocarditis virus

Sudies were carried out on mongrel mice of both sexes with an initial body mass of 10-11 g. The control and experimental groups included 30 animals each. Compound XLIV and the reference drug interferon inducer Ridostine were administered once intraperitoneally 24 hours after the mice were infected with the encephalomyocarditis virus at doses of 30 μg/kg and 5.0 mg/kg, respectively. The encephalomyocarditis virus was administered at a dose of 100 $LD_{50}$ Antiviral activity was determined by change in mean lifetime (MLT) of mice and the degree of protection provided against lethal viral infection.

The results obtained are presented in Table 39. It is shown that compound XLIV possesses pronounced antiviral activity at a dose which, compared to the reference drug dose, is two orders lower. Table 39

| Antiviral activity of the test compound XLIV | | | |
|---|---|---|---|
| Group of animals | MLT, days | Survival rate, percent | Protection degree, percent |
| Control | 7.2 | 13.3 | 0 |
| Compound XLIV | 12.5* | 30.3* | 17.0 |
| Ridostine | 13.3* | 82.0* | 668.7 |
| * = significance of differences in relation to the control group;<br>* = p<0.05; ** = p<0.01 | | | |

B. Effect of the compounds of the present invention on intensity of influenzal infection in mice

Studies were carried out on white mongrel mice of both sexes weighing 8-10 g. Strain Aichi (allantois fluid) of type A human influenza virus was intranasally administered to the mice at a dose $LD_{50}$. The test compounds III, VI, VIII, XXXIII, XLVIII and XLVIII were orally administered to animals for three days before infection and for ten days following infection. Arbidol (a specific drug) was used as a reference drug, at a dose of 100 mg/kg, and was administered 24 and 2 hours prior to and 3 days post infection, and Tymogen (non-specific drug) was administered to animals at a dose of 10 μg/kg according to the same dosage regimen as the test compound. Antiviral activity was determined based on the MLT of the mice and the protection provided against lethal viral infection.

The study results are presented in Table 40. It was shown that the largest increase in MLT, survival rate and the degree of protection were observed when compound VII was administered at a dose of 500 μg/kg. The administration of the other tested compounds at doses of 50 and 500 μg/kg, and of Arbidol, was accompanied by an equal change in the parameters. The effect of Tymogen as an antiviral drug was less expressed.

Thus, the presented data give evidence of a pronounced antiviral effect for compounds of the present invention in experimental viral infection in mice caused by type A human influenza virus.

Table 40

| Antiviral effect of the compounds of the present invention on experimental influenzal infection in mice | | | |
|---|---|---|---|
| Group of animals, order Nos | MLT, days | Survival rate in percent | Protection degree in percent |
| 1. control-1 | 5.76 | 16.7 | - |
| 2. Arbidol | 9.22* | 43.4* | 26.7 |
| 3. Thymogene | 8.02 | 30.0 | 13.3 |
| 4. Compound VIII, 50 μg/kg | 9.88*1 | 40.0*1 | 23.3 |
| 4. Compound VIII, 500 μg/kg | 14.37**1 | 56.7*1 | 40.0 |
| 5. Compound XLVIII, 50 μg/kg | 9.58*1 | 34.5*1 | 17.8 |

(continued)

| Antiviral effect of the compounds of the present invention on experimental influenzal infection in mice | | | |
|---|---|---|---|
| Group of animals, order Nos | MLT, days | Survival rate in percent | Protection degree in percent |
| 6. Control - 2 | 9.1 | 34.5 | - |
| 7. Arbidol | 13:88*2 | 94.7*2. | 60.2 |
| 8. Compound VI, 50 μg/kg | 11.24*2 | 73.3*2 | 38.8 |
| 8. Compound VI, 500 μg/kg | 11.62*2 | 79.3*2 | 44.8 |
| 9. Compound III, 50 μg/kg | 10.1 | 86.6*2 | 52.1 |
| 9. Compound III, 500 μg/kg | 11.5*2 | 76.6*2 | 42.2 |
| 10. Compound XLVII, 50 μg/kg | 13.0*2 | 83.3*2 | 48.8 |
| 10. Compound XLVII, 500 μg/kg | 10.0 | 79.3*2 | 44.8 |
| 11. Compound XXXIII, 50 μg/kg , | 9.09 | 63.3 | 28.8 |
| 11. Compound XXXIII, 500 μg/kg | 11.9 | 76.6*2 | 76.6 |
| * = significance of differences in relation to the control group;<br>* = p<0.05; **= p<0.01. | | | |

B. Effect of the compounds of the present invention on human immunodeficiency virus reproduction in acute infection of lymphoblastoid cells

The study was carried out using a culture of human lymphoblastoid cells, namely MT-4 cells HIV-1/IV17 of type 1 human immunodeficiency virus isolated from the collection of the D.I.Ivanovsky Institute for Virology was used in the experiment. The test compounds III, VI and VII were introduced into the cellular culture at concentrations of 1.0, 0.1 and 0.01 μg/ml. The reference drug Azidothymidine was introduced at a dose of 0.05 μM/ml. The compounds were introduced into the culture of MT-4 cells at a dose of 1000 $TCD_{50}$ one hour prior to introduction of the virus. Viability of MT-4 cells was estimated at day 7 of the experiment.

The viability of the cells was the main efficacy parameters of the test compounds.

Preliminary experiments showed that the test compounds, are non-toxic for lymphoblastoid cells at concentrations of 1.0 μg/ml and less.

Table 41

| Effect of the test compounds on viability of MT-4 cells infected with HIV-1 | | |
|---|---|---|
| Group | Compound dose | Viability of cells in percent |
| Non-infected cells - 1 | - | 90 |
| Infected cells - 1 | - | 10-17 |
| Azidothymidine | 0.05 μM/ml | 75*1 |
| Compound III | 0.01 μg/ml | 18 |
| Compound III | 0.1 μg/ml | 39*1 |
| Compound III | 1.0 μg/ml | 68*1 |
| Non-infected cells - 2 | - | 88 |
| Infected cells - 2 | - | 24 |
| Azidothymidine | 0.01 μg/ml | 72*2 |
| Compound VI | 0.01 μg/ml | 45*2 |
| Compound VII | 0.1 μg/ml | 49*2 |
| Compound VI | 1.0 μg/ml | 54*2 |
| Compound VII | 0.01 μg/ml | 34 |

(continued)

| Effect of the test compounds on viability of MT-4 cells infected with HIV-1 | | |
|---|---|---|
| Group | Compound dose | Viability of cells in percent |
| Compound VII | 0.1 μg/ml | 49*2 |
| Compound VII | 1.0 μg/ml | 51*2 |
| * = significance of differences in relation to the respective group of infected cells, *= p<0.01. | | |

The results presented in Table 41 show that the test compounds, at concentrations of 1.0 and 0.1 μg/ml, exhibit a protective effect against the cytodestructive HIV-1 effect. The effect of the compounds is pronounced to a different extent for each compound, the effect of compound III being comparable with the effect of the reference drug Azidothymidine.

EXAMPLE 45

EFFECT OF THE COMPOUNDS OF THE PRESENT INVENTION ON THE ACTIVITY OF PERIPHERAL BLOOM NEUTROPHILS

Experiments were carried out on C57BL/6 female mice with intramuscularly transplanted LLC carcinoma. The activity of neutrophils was measured on days 11 and 14 of the experiment.

The experimental animals were divided into four groups:

control - animals with LLC tumor, 10 animals;
animals with LLC tumor + compound III at a dose 500.0 μg/kg for 10 days, 8 animals;
animals with LLC tumor + cyclophosphane 5 times at a dose 20.0 mg/kg (in 48 hours) for 10 days, 8 animals;
compound III and Cyclophosphane according to the above dosage regimen, 8 animals.

The duration of the experiment was 14 days. Table 42 summarizes the results of the study of neutrophil activity in the animals of the experimental groups.

Table 42

| Change in the activity of neutrophils in C57/BL/6 mice with LLC in combined and individual administration of compound III and Cyclophosphane | | | |
|---|---|---|---|
| Experimental Group | Percent of active neutrophils | | |
| | Background | day 11 | day 14 |
| Control | 22.4 ± 3.5 | 13.0 ± 3.0 | 16.9 ± 1.8 |
| Compound III | 20.1 ± 2.7 | 9.3 ± 1.7 | 15.3 ± 4.3 |
| Cyclophosphane | 21.5 ± 3.4 | 7.6 ± 2.0 | 20.6 ± 6.0 |
| Compound III + Cyclophosphane | 21.9 ± 4.2 | 20.8 ± 5.4 | 26.0 ± 3.4* |
| *= Significance of differences in relation to the control, p<0.05. | | | |

The development of LLC carcinomas was shown to be accompanied by a decrease in the number of active forms of neutrophils in the peripheral blood of experimental animals. The animals which were administered a combination of compound III and Cyclophosphane, were characterized by a significantly higher activity level of neutrophils as compared with the control animals.

EXAMPLE 46

EFFECT OF THE COMPOUNDS OF THE PRESENT INVENTION ON THE ACTIVITY OF PERITONEAL MACROPHAGES

Changes in the activity of peritoneal macrophages (PM), as effected by the test compounds, were studied.

The experiments were carried out on mongrel white male mice with an initial body mass of 20-22 g. Each group included 10 animals. Compounds III, VI, XLIV were dissolved in normal saline (NS). The test compounds were administered to animals orally for three days at doses of 50 and 500 μg/kg. The animals of the control group were given an equal volume of NS. The mice were sacrified 20 hours after the last compound administration and the peritoneal cavity was washed with 1.5 ml Henks solution comprising 5 U/ml heparine. 0.1 ml of the wash solution was incubated for 30 minutes at 37°C, with 0.1 ml 0.2% nitroblue tetrasolium (NBT) solution, then smears were prepared. Nuclei were additionaly stained with 0.1% neutral red solution. The results were expressed as a percentage of active cells [Klimov V.V., Kolovkina T.V. Nitroblue tetrasolium reduction test stimulated with pyrogenal. Laborotornoye delo (Laboratory matter) (1982), No 10, pp. 624-625]. PM activity degree was estimated based on the amount of reduced NBT inclusions (formazane) according to the following scores:

0 - no formazane inclusions, inactive PM;
1- single formazane inclusions;
2 - inclusions fill up to 1/3 of PM cytoplasm;
3 - inclusions fill up to 1/2 of PM cytoplasm;
4 - inclusions fill up all cytoplasm.

Table 43

| Change in the activity of peritoneal macrophages as effected by the compounds of the present invention | | | | |
|---|---|---|---|---|
| Group of Animals | Activity of macrophages, percent | | | |
| | 0 | 1 | 2 | 3 |
| Control | 25.3 ± 4.2 | 35.7 ± 3.6 | 32.6 ± 3.9 | 6.4 ± 1.4 |
| Compound XLIV, 50μg/kg | 23.9 ± 4.4 | 37.3 ± 3.4 | 34.9 ± 5.9 | 5.4 ± 2.0 |
| Compound XLIV, 500 μg/kg | 17.4 ± 2.5* | 37.3 ± 3.9 | 35.8 ± 4.4 | 9.4 ± 2.9* |
| Compound III, 50μg/kg | 11.6 ± 2.8* | 27.5 ± 2.5* | 44.6 ± 1.9* | 15.8 ± 3.3* |
| Compound III, 500μg/kg | 12.8 ± 1.3* | 31.3 ± 2.3 | 46.0 ± 2.6* | 10.9 ± 2.1* |
| Compound VI, 50μg/kg | 17.9 ± 1.4* | 30.9 ± 3.0 | 40.3 ± 2.1* | 10.4 ± 1.9* |
| Compound VI, 500 μg/kg | 20.3 ± 3.4 | 37.9 ± 3.1 | 34.0 ± 4.0 | 7.1 ± 2.0 |
| * = significance of differences in relation to the control group; * = p<0.01: | | | | |

The data presented in Table 43 show that the tested compounds increase the portion of active macrophages at the expense of a decrease in the amount of inactive forms, the effect of compound III in study being pronounced at both substance doses, while the effect of compound XLIV is more pronounced at a dose 500 μg/kg, and that of compound VI is more pronounced at a dose 50 μg/kg.

Thus, the tested compounds posses the ability to increase the portion of active forms of macrophages but at different doses.

EXAMPLE 47

EFFECT OF THE COMPOUNDS OF THE PRESENT INVENTION ON CHANGES IN PROSTACYCLINE TO THROMBOXANE RATIO IN MICE WITH PULMONARY CARCINOMA

Experiments were carried out on C57B1 male mice weighing 18-20 g. Lewis pulmonary carcinoma (generation 3) transplantation was performed according to a conventional technique [Experimental estrimation of antitumor drugs in the U.S.S.R. and the U.S.A. edited by E.P.Sof'ina, A.B.Syrkin, A.Goldin A.Kline. Moscow, Meditsina publishers (1980),

296 pp.]. Each group included 8 animals. The administration of compound III at a dose 500 μg/kg per day in drinking water was started 24 hours after tumor transplantation.

The ratio of thromboxane $A_2$ to prostacycline was determined according to the technique described in example 35. At day 24 of the experiment the animals were sacrified and the test parameters were determined.

Groups of animals:

Group 1-intact control.
Group 2 - mice with transplanted Lewis carcinoma.
Group 3 - mice with transplanted Lewis carcinoma + compound III.

Table 44

| Change in arachidonic acid metabolism in mice with transplanted Lewis carcinoma as effected by compound III | | | |
|---|---|---|---|
| Group | Prostacycline | Thromboxane $A_2$ | Ratio 6-keto-$PGF_{1\alpha}$ :$TXB_2$ |
| Group 1 | 8.9 ± 0.42 | 5.98 ± 0.30 | 1.5 ± 0.05 |
| Group 2 | 4.57 ± 0.07**C | 7.6 ± 0.31*C | 0.60 ± 0.03**C |
| Group 3 | 5.80 ± 0.75*2 | 7.23 ± 0.18*C | 0.80 ± 0.11*2 |
| * = significance of differences between groups: C = control group; 2 = group 2; * = p < 0.05;**=p<0.01. | | | |

The data presented in table 44 give evidence of the fact that in mice with transplanted Lewis carcinoma the thromboxane $A_2$ level was found to rise by 1.4 times and a twofold drop in prostacycline formation and prostacycline to thromboxane $A_2$ ratio were detected. The administration of compound III to animals results in a rise in prostacycline formation and in the prostacycline to thromboxane $A_2$ ratio, as well as a drop in thromboxane $A_2$ synthesis.

EXAMPLE 48

EFFECT OF THE COMPOUNDS OF THE PRESENT INVENTION ON THE DEVELOPMENT OF POSTSRESS CONDITIONS IN MICE

Experiments were carried out on inbred Balb male mice with an initial body mass of 16-18 g. Each group included 12 animals. The model of swimming in warm water for 20 minutes was used to develop emotional-locomotory stress in the animals. Stress development was estimated based on the activity of natural killer lymphocytes (NK cells) and the functioning of the interferon system [Sukhikh T.G., Nossik N.N., Parshina O.V., Van'ko L.V., Meerson F.Z., Yershov F.I., Relationship between the natural cellular cytotoxicity system and the interferon system in immobilization stress, Bulletin of Experimental Biology (1984), N , pp. 593-595].

The activity of NK-cells was determined using the test of $H^3$-uridine release from the labelled target cells YAC-1 (mouse cellular lymphoma maintained by passages in vitro in suspension in the RPMI-1640 medium with 10% fetal serum). The $H^3$-uridine (3-5 μC/cells/ml) was introduced into the cellular suspension at a concentration of 1 million cells/ml in the 60th minute, while shaking with a 15 minute interval and subsequent washing with the medium.

The source of the NK-cells was splenocytes of mice, obtained using the method published by Nossik et al [Nossik N.N., Bopegamage S.A., Yershov F.I., Properties of interferons produced by different cell populations. Acta microbiologica Hungarica (1988), Vol. 35, Iss. 4, pp. 397-403].

In studying the interferon reaction of mouse splenocytes, spleen cells were placed into an incubation medium (the RPMI-1640 medium + 10% fetal calf serum), their concentration was brought to 1-3 x $10^3$ cells/ml and they were incubated at 37°C for 24 hours. Newcastle disease virus (α-interferon induction) and FGA mitogen (γ-interferon induction) were used as intereferon inducers [Nossik N.N:, Bopegamage S.A., Yershov F.I. Properties of interferons produced by different cell populations. Acta microbiologica Hungarica (1988), Vol. 35, Iss. 4, pp. 397-403].

The animals were divided into four groups, each including 36 mice.

Group 1- intact animals not subjected to stress and given normal saline (NS).
Group 2 - animals which were subjected to stress effect and given NS.
Group 3 - animals which were subjected to stress effect and administered compound III.
Group 4 - animals which were subjected to stress effect and administered compound XLIV.

The test compounds (at a dose 50 μg/kg) and NS were orally administered to animals four times, according to the following dosage regimen: for two days before exposure to stress (first and second administrations), two hours prior to stress (third administration) and 24 hours after third administration (fourth administration).

The activity of NK-cells and interferon state were dynamically determined 4 hours following stress, 24 hours later and then on days 5, 7 and 10.

Table 45

| Change in activity of NK-cells as effected by the compounds of the present invention | | | | | |
|---|---|---|---|---|---|
| | Time after stress, days | | | | |
| Group of animals | 0.16 | 1 | 5 | 7 | 10 |
| 1. Intact control | 81 ± 0.82 | 79 ± 1.32 | 79 ± 0.99 | 76 ± 0.66 | 80 ± 0.99 |
| 2. Stress | 57.3 ± 0.82***1 | 39 ± 48***1,3 | 68.3 ± 1.65 | 76.5 ± 0.49 | 71.7 ± 2.46*1 |
| 3. Stress compound III | + 68.5 ± 0.99*1,2 | 69.3 ± 1.81***2 | 79 ± 1.46 | 69.3 ± 4.85 | 77 ± 1.98 |
| 3. Stress compound XLIV | +69.5 ± 1.32**2 | 61.5 ± 2.64*1,2 | 71 ± 0.82 | 67.7 ± 3.95 | 77.5 ± 1.65 |
| *, ** = significance of differences; the superscript numbers signify groups in relation to which differences are significant;<br>*= $p < 0.05$; ** = $p<0.01$ | | | | | |

The results presented in Table 45 show that administration of the test compounds prevents the drop in the activity of NK-cells caused by stress. In addition, the administration of the test compounds to animals results in normalization of α-interferon production and raises γ-interferon production (Table 46).

Table 46

| Effect of the test compounds of general formula (I) on α-interferon and γ-interferon production by splenocytes of mice under poststress conditions | | | | | | |
|---|---|---|---|---|---|---|
| Group of animals | Time after stress, days | | | | | |
| | 0 | 0.16 | 1 | 5 | 7 | 10 |
| α-interferon, U/ml | | | | | | |
| 1. Intact control | 160 | 160 | 80 | 160 | 160 | 160 |
| 2. Stress | 160 | 10 | 10 | 16 | 32 | 16 |
| 3. Stress + compound III | 160 | 10 | 10 | 160 | 160 | 160 |
| 4. Stress + compound XLIV | 160 | 10 | 80 | 80 | 100 | 140 |
| γ-interferon, U/ml | | | | | | |
| 1. Intact control | 40 | 40 | 32 | 40 | 40 | 40 |
| 2. Stress | 80 | 20 | 40 | 40 | 10 | 10 |
| 3. Stress + compound III | 80 | 80 | 32 | 32 | 320 | 80 |

(continued)

| γ-interferon, U/ml | | | | | | |
|---|---|---|---|---|---|---|
| 4. Stress + compound XLIV | 80 | 20 | 320 | 40 | 32 | 32 |
| Interferon production by splenocytes of 8 mice per measurement point. | | | | | | |

Thus, in animals which were administered the test compounds, the consequences of stress reaction were less pronounced than-in mice which were not administered them.

EXAMPLE 49

EFFECT OF THE COMPOUNDS OF THE PRESENT INVENTION ON MITOGEN-INDUCED BLASTTRANSFORMATION OF LYMPHOCYTES

Human lymphocytes were used for the blasttransformation reaction [Kiseleva E.P., Tsveibakh A.S., Goldman E.I., Pigareva N.V., Application of a micromethod to study blasttransformation of lymphocytes in humans and animals, Immunology (1985), No 1, pp. 76-78]. 10 ml of blood was collected in a sterile syringe containing heparine (to a final concentration 25 U/ml) and incubated to sediment red blood cells (60-90 minutes at 37˚C). The leukocytic suspension layer was selected and the number of nucleated cells was calculated. The obtained cells were incubated for 72 hours in a humid atmosphere with 5% $CO_2$ at 37˚C, in the cells of plates for immune reactions in RPMI-1640 medium comprising 15% fetal calf serum and 50 μg/kg Neomycine (incubation mixture volume 0.15 ml). 5 x $10^5$ cells were introduced into each plate cell. Incubation was carried out in the presence or absence of mitogen phytohemagglutinin (PHA) (Sigma) (13.3 μg/kg) or concanavalin A (ConA) (Sigma) (13.3μg/kg), with or without addition of the test compounds at different concentrations. 2 mCi $H^3$ -thymidine in RPMI-1640 medium was introduced into each cell 16 hours before termination of incubation. Following termination of the culturing period. 0.15 ml of cellular suspension from each plate cell was transferred onto FN-8 paper filters. The filters were dried, washed twice with normal saline for 5 minutes and then washed with TCA for 60 minutes. After drying the radioactivity of samples was calculated on a counter in a ZhS-8 scintillator. The stimulation index was calculated as a ratio of the sample radioactivity with mitogen to the sample radioactivity without mitogen.

The results presented in Tables 47-49, give evidence of a stimulating effect of compounds XLIV and III on the blasttranformation reaction of human lymphocytes. Enhancement of the reaction induced by both PHA and ConA was also observed.

Table 47

| Effect of compound XLIV on PHA- induced blasttranformation of human lymphocytes | | | | |
|---|---|---|---|---|
| No | Compound XLIV concentration, M/l | Mitogen | Radioactivity, number of pulses for 20 seconds | Stimulation index |
| 1 | Control | - | 139 ± 51 | |
| | | +PHA | 3085 ± 166 | 22.2 |
| 2 | $10^{-8}$ | - | 261 ± 100 | |
| | | +PHA | 3830 ± 286* | 14.7 |
| 3 | $10^{-7}$ | - | 300 ± 107 | |
| | | +PHA | 2960 ± 210 | 9.9 |
| 4 | $10^{-6}$ | - | 150 ± 33 | |
| | | +PHA | 4414 ± 570* | 29.6 |
| 5 | $10^{-5}$ | - | 132 ± 13 | |
| | | +PHA | 2999 ± 213 | 23.2 |
| 6 | $10^{-4}$ | - | 301 ± 103 | |

(continued)

| Effect of compound XLIV on PHA- induced blasttranformation of human lymphocytes | | | | |
|---|---|---|---|---|
| No | Compound XLIV concentration, M/l | Mitogen | Radioactivity, number of pulses for 20 seconds | Stimulation index |
| | | +PHA | 3276 ± 225 | 10.9 |

Table 48

| Effect of compound III on concanavalin A - induced blasttranformation of human lymphocytes | | | | |
|---|---|---|---|---|
| No | Compound III concentration, M/l | Mitogen | Radioactivity, number of pulses for 20 seconds | Stimulation index |
| 1 | Control | - | 317 ± 88 | |
| | | +ConA | 941 ± 113 | 2.97 |
| 2 | $10^{-8}$ | - | 234 ± 36 | |
| | | +ConA | 849 ± 133 | 3.63 |
| 3 | $10^{-7}$ | - | 478 ± 35 | |
| | | +ConA | 1483 ± 67** | 3.10 |
| 4 | $10^{-6}$ | - | 408 ± 75 | |
| | | +ConA | 1922 ± 248** | 4.71 |
| 5 | $10^{-5}$ | - | 467 ± 109 | |
| | | +ConA | 1562 ± 134** | 3.34 |
| 6 | $10^{-4}$ | - | 274 ± 28 | |
| | | +ConA | 1197 ± 97 | 4.37 |
| 7 | $10^{-3}$ | - | 330 ± 105 | |
| | | +ConA | 680 ± 82 | 2.06 |

Table 49

| Effect of compound XLIV on concanavalin A - induced blasttranformation of human lymphocytes | | | | |
|---|---|---|---|---|
| No | Compound XLIV concentration, M/l | Mitogen | Radioactivity, number of pulses for 20 seconds | Stimulation index |
| 1 | Control | - | 361 ± 43 | |
| | | +ConA | 1061 ± 243 | 2.94 |
| 2 | $10^{-8}$ | - | 233 ± 54 | |
| | | +ConA | 971 ± 44 | 4.17 |
| 3 | $10^{-7}$ | - | 242 ± 19 | |
| | | +ConA | 1786 ± 241* | 7.38 |
| 4 | $10^{-6}$ | - | 289 ± 52 | |
| | | +ConA | 1963 ± 205* | 6.96 |
| 5 | $10^{-5}$ | - | 339 ± 32 | |
| | | +ConA | 1695 ± 139* | 5.00 |
| 6 | $10^{-4}$ | - | 322 ± 38 | |

(continued)

| Effect of compound XLIV on concanavalin A - induced blasttranformation of human lymphocytes | | | | |
|---|---|---|---|---|
| No | Compound XLIV concentration, M/l | Mitogen | Radioactivity, number of pulses for 20 seconds | Stimulation index |
| | | +ConA | 1259 ± 90 | 3.91 |
| 7 | $10^{-3}$ | - | 356 ± 51 | |
| | | +ConA | 1072 ± 135 | 3.01 |

The data presented in examples 41-49 give evidence of the fact that the test compounds possess immunomodulating activity, significantly inhibit the growth of a transplantable tumor, the metastasizing process and increase the resistivity of animals to microbial and viral infections.

All the above mentioned studies also show that the compounds of the present invention possess the above mentioned biological efficacy at doses which are 2 to 3 orders lower than the doses of known drugs that are used for practically similar efficacy.

Further, examples of dosage forms are presented below, in which the compounds of the present invention can be used.

EXAMPLE 50

EXAMPLES OF DOSAGE FORMS

A. Gelatine capsules.

The composition of the powder introduced into a capsule is

| | |
|---|---|
| A compound of the present invention | 1-35 mg |
| Magnesium oxide | 50 mg |
| Starch | 100-200 mg. |

The above indicated ingredients are mixed and the mixture is introduced into solid gelatine capsules at an amount of 151-285 mg.

B. Tabletted dosage form.

The tabletted dosage form is prepeared using the above mentioned ingredients:

| | |
|---|---|
| Compound of the present invention | 1-35 mg |
| Potato starch | 100 mg |
| polyvinyl pyrrolidone | 10 mg |
| Magnesium stearate | 2 mg |
| Lactose | 48-82 mg |
| Aerosyl | 5 mg |

The ingredents are stirred and pressed to produce tablets weighing 200 mg each.

B. Aerosolic dosage form.

The composition of aerosolic mixture is calculated per 10 administrations:

| | |
|---|---|
| Compound of the present invention | 10-40 mg |
| Magnesium sulphate | 150 mg |
| Lactose | 110-140 mg |

The compound is mixed with excipients and placed into a special device for spraying.

D. uppositoria.

The following bases can be used as suppositorial bases water-insoluble bases-cocoa oil;
water-soluble bases or bases mixable with water - gelatine-glycerinic or polyethylene oxide bases;
combined bases - soapy-glycerinic.

Example of a suppositorium composition:

| | |
|---|---|
| Compound of the present invention | 1-35 mg |
| Cocoa oil - | amount needed to prepare a suppositorium. |

If needed, rectal, vaginal and urethral suppositoria with respective excipients can be produced.

E. Ointments.

The following compounds can be used as an ointment base:

carbohydrate ointment bases - white and yellow vaseline (Vaselinum album, Vaselinum flavum), vaseline oil (Oleum Vaselini) and white and yellow ointment (Unguentum album, Unguentum flavum), and as supplements to confer a firmer consistence, hard paraffine and wax can be used;
absorbtive ointment bases - hydrophylic vaseline (Vaselinum hydrophylicum), lanoline (Lanolinum) and coldcreme (Unguentum leniens);
ointment bases washable with water- hydrophylic ointment (Unguentum hydrophylum);
water-soluble ointment bases - polyethylene glycol ointment (Unguentum Glycolis Polyaethyleni), bentonite bases and others.

Example of an ointment composition:

| | |
|---|---|
| A compound of the present invention | 0.1-0.5 g |
| Vaseline | 10 g |

Ointments are produced according to the respective technology.

F. Solution for injections.

The following substances can be used as solvents to prepare a solution for injection 0.9% sodium chloride solution, distilled water, novocaine solution. Dosage forms: ampules, vials, syringes-tubes, inserts.

Composition of solution for injections:

| | |
|---|---|
| A compound of the present invention- | 1-5 mg |
| Distilled water- | 1-2 ml |

The production of different dosage forms for injections is possible: sterile solutions, sterile powders and tablets.

EXAMPLE 51

VERSIONS OF APPLYING THE COMPOUNDS OF THE PRESENT INVENTION AS COMPONENTS OF COSMETIC AGENTS

A. Lotions.

Composition of a therapeutic-cosmetic lotion:

| | |
|---|---|
| A compound of the present invention | 0.1-1 g |
| Flavor- | 0.5-1.5 g |

(continued)

| | |
|---|---|
| Ethyl alcohol 60°-70° - | 100-150 ml |

Lotions are produced according to conventional technology with pH parameters 5.5-6.0.

G. Creams.

Composition of a therapeutic-cosmetic cream:

| | |
|---|---|
| A compound of the present invention - | 0.1-1 g |
| Olive oil - | 8-10 g |
| Triethanol amine - | 1 g |
| Glycerine - | 3-5 g |
| Sodium benzoate - | 1-2 g |
| Flavor - | 1-1.5 g |
| Lanoline - | 20-25 g |
| Water - | up to 100 g |

Creams are produced according to the respective technology.

## Claims

**1.** A compound which is cyclohexylcarbonylhistamine or N-(imidazolyl-4-acetyl)-heptylamine, or a peptide of general formula I:

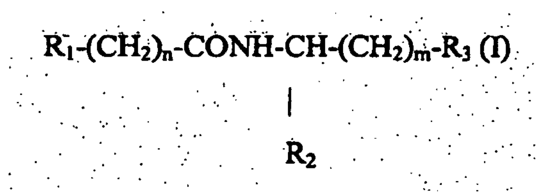

either of structure

| $R_1$ | n | $R_2$ | m | $R_3$ |
|---|---|---|---|---|
| 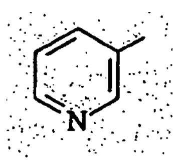 | 0 | H | 1 | 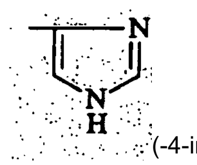 (-4-imidazolyl) |
| 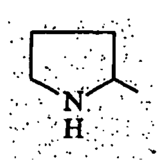 | 0 | H | 1 | 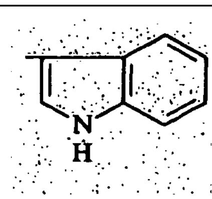 (-3-indolyl) |
| 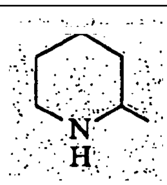 | 0 | H | 1 | -4-imidazolyl |

(continued)

| $R_1$ | n | $R_2$ | m | $R_3$ |
|---|---|---|---|---|
| 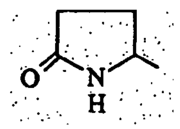 | 0 | H | 1 | -3-indolyl |

or wherein
$R_1$ is a $C_1$-$C_3$-hydrocarbon radical substituted by amino or carboxy, the carboxy group being optionally esterified and the amino group being optionally substituted by acyl or the ester of carbonic acid; or
$R_1$ is a $C_1$-$C_3$-hydrocarbon radical simultaneously substituted by amino and carboxy, the carboxy group being optionally esterified and the amino group being optionally substituted by acyl or the ester of carbonic acid; or
$R_1$ is a $C_1$-$C_3$ hydrocarbon radical substituted by an indole residue or a 5-6 membered unsaturated heterocyclic group, the hydrocarbon radical optionally simultaneously comprising an amino group which is unsubstituted or substituted by acyl ', or the ester of carbonic acid;
$R_2$ is hydrogen or carboxy which is optionally esterified; $R_3$ is an indole group, which group is unsubstituted or substituted by methyl or hydroxy, the hydroxy group in turn being unsubstituted or substituted by acyl, alkyl or aralkyl; or
$R_3$ is a 5-6 membered saturated or unsaturated heterocyclic group comprising oxygen, sulfur and/or 1-3 nitrogen atoms, or a 5-6-membered unsaturated carbocyclic group, or a methyl derivative thereof; or
$R_3$ is a $C_1$-$C_3$ hydrocarbon radical substituted by amino or carboxy, the carboxy group being optically esterified; or
$R_3$ is a $C_1$-$C_3$ hydrocarbon radical simultenaously substituted by amino and
n is from 0-4 and m is from 1-5;
or a pharmaceutically acceptable salt thereof,
for use an antibacterial, antitumour, antiviral or antimetastatic agent, or to alleviate stress conditions.

**2.** A compound according to claim 1, which is for use in combination with a cytostatic agent or radiotherapy.

**3.** A compound which is a peptide of general formula (I) of claim 1, wherein:

$R_1$ is $NH_2$-$CH_2$-, n is 1-4 or
$R_1$ is HOOC-$CH_2$-, n is 0-3; or
$R_1$ is Rz-OCO-$CH_2$-, n is 0-3; Rz is -H or a $C_1$-$C_3$ hydrocarbon radical; or
$R_1$ is HOOC-$CH_2$-($CH_3$)C(Rv)-, Rv is H or $CH_3$; or
$R_1$ is $C_6H_5CH_2$-OCO-NH-$CH_2$-, n is 1-2; or
$R_1$ is Rx-CONH-$CH_2$-, n is 1-4, Rx is a $C_1$-$C_3$-hydrocarbon radical; or
$R_1$ is $CH_3$CONH-CH(COOH)-, n is 1-2; or
$R_1$ is $CH_3$CONH-CH($[CH_2]_k$COOH)-, n is 0, k is 1-2; or
$R_1$ is $NH_2$-CH($[CH2]_k$COOH)-, n is 0, k is 1-2; or
$R_1$ is HOOC-CH($NH_2$)-, n is 0; or
$R_1$ is HOOC-CH($NH_2$), N is 1-2; or
$R_1$ is $CH_3$OOC-CH($NH_2$)-, n is 1-2; or
$R_1$ is $(CH_3)_3$C-OCONH-CH(COOC$H_2$-$C_6H_5$)-, n is1-2; or
$R_1$ is -$CH_2$-4-imidazolyl, -$CH_2$-3-indolyl, n is 0-1 ; or
$R_1$ is Rb-$CH_2$-CH(NHRy)-, Rb is -4-imidazolyl, -3-indolyl, Ry is tert-butyloxycarbonyl, benzyloxycarbonyl, H-, n is 0;
$R_2$ is -H, -COOH, -COORz wherein Rz is -H or a $C_1$-$C_3$-hydrocarbon radical; and

$R_3$= 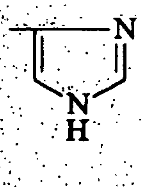, 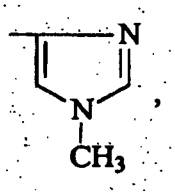, 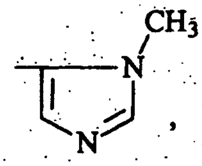, 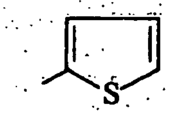

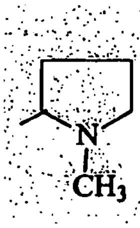, 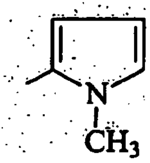, 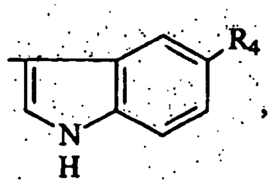, 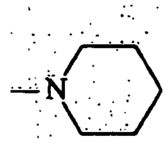

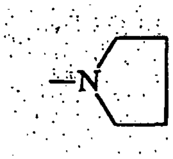, 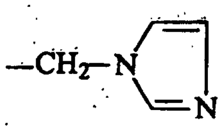, 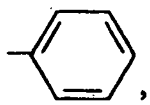, 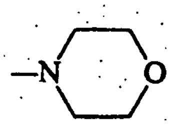, 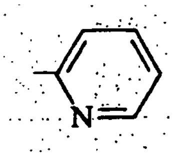

m= 1; or
$R_3$ is $-CH_2-NH_2$, m =1-2; or
$R_3$ is $-CH_2-COOH$, m =1-2; or
$R_3$ is $-CH(NH_2)-COOH$, m =1-2,
where $R_4$ = -H, -OH, $-OCH_3$ or $-OCH_2C_6H_5$,

for a use as defined in claim 1.

**4.** A compound which is a peptide of general formula (I) of claim 1 and is selected from the following compounds:

| N° comp. | $R_1$ | n | $R_2$ | m | $R_3$ |
|---|---|---|---|---|---|
| **II** | $HOOC-CH_2-$ | 1 | H | 1 | 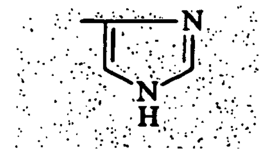 (-4-imidazolyl) |
| **III** | $HOOC-CH_2-$ | 2 | H | | -4-imidazolyl |
| **IV** | $HOOC-CH_2-$ | 3 | | 1 | -4-imidazolyl |
| **V** | $CH_3CO-NH-CH(COOH)-$ | 2 | H | 1 | -4-imidazolyl |
| **VI**[a] | $(CH_3)_3C-OCONH-CH-$<br>$COOCH_2C_6H_5$ | 2 | $-COOCH_3$ | 1 | -4-imidazolyl |
| **VI** | $NH_2-CH-$ COOH | 2 | $COOCH_3$ | 1 | -4-imidaizolyl; |
| **VII** | $HOOC-CH_2-$ | 2 | $-COOCH_3$ | 1 | -4-imidazolyl |
| **VIII** | $NH_2-CH_2-CH_2-$ | 1 | $-COOCH_3$ | 1 | -4-imidazolyl |
| **IX** | $HOOC-CH(NH_2)-$ | 2 | $-COOCH_3$ | 1 | -4-imidazolyl |
| **X**[a] | $C_6H_5CH_2OCONH-CH_2-$ | 2 | H | 1 | 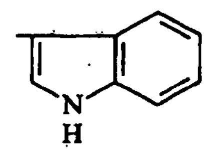 (-3-indolyl) |
| **X** | $NH_2-CH_2-$ | 2 | H | 1 | -3-indolyl |
| **XI** | $HOOC-CH_2-$ | 2 | H | 1 | 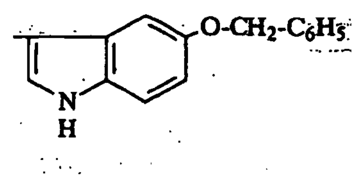 (-3-indolyl-$OCH_2C_6H_5$) |

(continued)

| N° comp. | $R_1$ | n | $R_2$ | m | $R_3$ |
|---|---|---|---|---|---|
| XII | $HOOC-CH_2-$ | 2 | H | 1 | 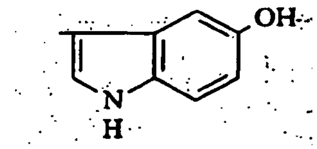 (-3-indolyl-OH) |
| XIII | $HOOC-CH_2-$ | 2 | H | 1 | 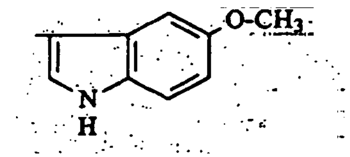 (-3-indolyl-OMe) |
| XIV | $HOOC-CH_2-$ | 2 | H | 1 | 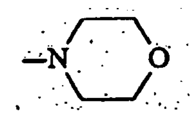 |
| XV | $HOOC-CH_2-$ | 2 | H | 1 | 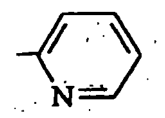 |
| XVI | $HOOC-CH_2-$ | 2 | H | 1 | 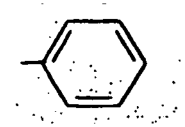 |
| XVII | $HOOC-CH_2-$ | 2 | H | 1 | 3-indolyl |
| XVIII | $HOOC-CH_2-$ | 2 | H | 1 | 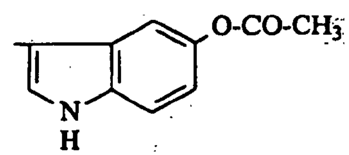 (-3-indolyl-$OCOCH_3$) |

(continued)

| N° comp. | $R_1$ | n | $R_2$ | m | $R_3$ |
|---|---|---|---|---|---|
| XXII | HOOC-$CH_2$- | 1 | H | 1 | 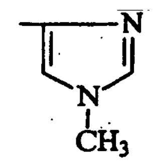 |
| XXIII | HOOC-$CH_2$- | 2 | -$COOCH_3$ | 1 | 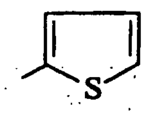 |
| XXIV | HOOC-$CH_2$- | 2 | H | 1 | 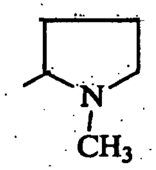 |
| XXV | HOOC-$CH_2$- | 2 | H | 1 | 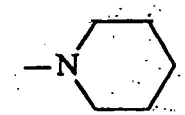 |
| XXVI | HOOC-$CH_2$- | 2 | H | 1 | 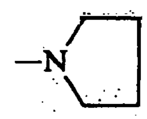 |
| XXVII | HOOC-$CH_2$- | 1 | H | 2 | 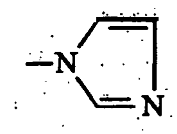 |
| XXVIII | $C_2H_5OCO$-$CH_2$- | 0 | H | 1 | -4-imidazolyl |
| XXIX | 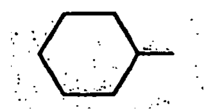 | 0 | H | 1 | -4-imidazolyl |

(continued)

| N comp. | R1 | n | R2 | m | R3 |
|---|---|---|---|---|---|
| **XXX** | 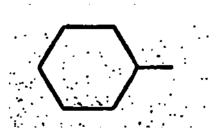 | 0 | H | 1 | -3-indolyl |
| **XXXIII** | $-CH_2$-4-imidazolyl | 1 | H | 4 | $CH_3-CH_2-$ |
| **XXXIV** | $-CH_2$-3indolyl | 0 | H | 2 | $-(NH_2)CH-COOH$ |
| **XXXV** | $-CH_2$-3-indolyl | 1 | H | 1 | $-CH_2-NH_2$ |
| **XXXVI** | $-CH(NH_2)-CH_2$4-imidazolyl | 0 | H | 1 | -COOH |
| **XXXVII** | $-CH_2-NHCO-CH_3$ | 2 | H | 1 | -4-imidazolyl |
| **XXXVIII** | $NH_2-CH(COOH)-$ | 1 | H | 1 | -4-imidazolyl |
| **XXXIX** | NH2-$CH(CH_2COOH)-$ | 0 | H | 1 | -4-imidazolyl |
| **XL**[a,b,c] | 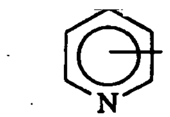 | 0 | H | 1 | -4-imidazolyl |
| **XLI** | 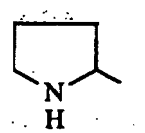 | 0 | H | 1 | 3-indolyl |
| **XLII** | 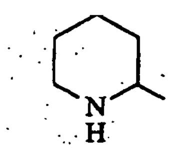 | 0 | H | 1 | -4-imidazolyl |
| **XLIII** | 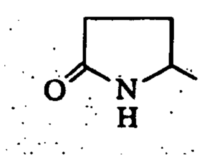 | 0 | H | 1 | -3-indolyl |

(continued)

| N□ comp. | R1 | n | R2 | m | R3 |
|---|---|---|---|---|---|
| Q | 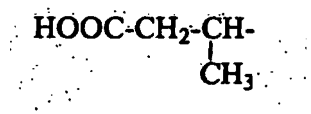 | 1 | H | 1 | -4-imidazolyl |

for a use as defined in claim 1.

**5.** A compound according to claim 1, which is glutarylhistamine structure

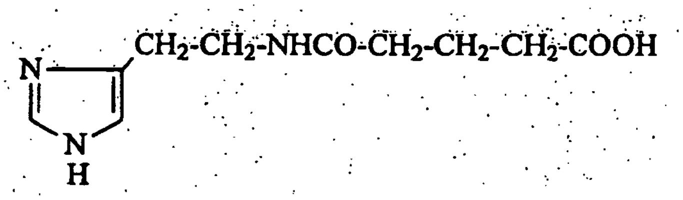

or a pharmaceutically acceptable salt thereof.

**6.** A compound according to any one of the preceding claims, wherein, when the compound is for antiviral use, it is for use in treating influenza or HIV.

**7.** A compound of structure (II) (III), (IV), (V), (VI[a]), (VI). (VII), (VIII), (IX). (X[a]). (XXV) (XXXI), (XXVII), (XXVIII), (VVIX), (XXXIII), (XXXIV). (XXXV), (XXXVII) (XXXIII), (XXXIX), (XL[a,b,c]), (XLI), (XLII), (XLIII) or (Q) or a pharmaceutically acceptable salt thereof

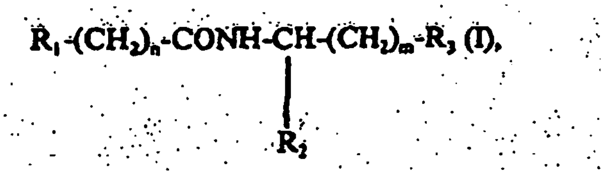

| | $R_1$ | n | $R_2$ | m | $R_3$ |
|---|---|---|---|---|---|
| **II** | HOOC-$CH_2$- | 1 | H | 1 | 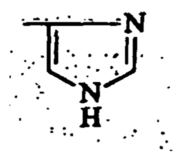 (-4-imidazolyl) |
| **III** | HOOC-$CH_2$- | 2 | H | 1 | -4-imidazol |
| **IV** | HOOC-$CH_2$- | 3 | H | | -4-imidazblyl |
| **V** | $CH_3CO$=NH-CH(COOH)- | 2 | H | 1 | -4-imidazolyl |
| **VI[a]** | 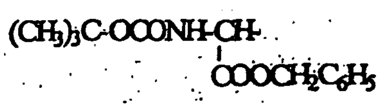 | 2 | -$COOCH_3$ | 1 | -4-imidazol |
| | 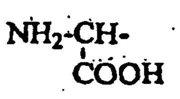 | 2 | -$COOCH_3$ | 1 | -4-imidazol |
| **VII** | HOOC-$CH_2$- | 2 | -$COOCH_3$ | 1 | -4-imidazolyl |
| **VIII** | $NR_2$-$CH_2$-$CH_2$ - | 1 | -$COOCH_3$ | 1 | -4-imidazolyl |
| **IX** | HOOC-CH($NH_2$)- | 2 | -$COOCH_3$ | 1 | -4-imidazolyl |

(continued)

| | $R_1$ | n | $R_2$ | m | $R_3$ |
|---|---|---|---|---|---|
| **X[a]** | $C_6H_5CH_2OCONH-CH_2-$ | 2 | H | 1 | 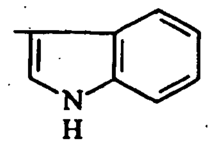 (-3-indolyl) |
| **X** | $NH_2-CH_2-$ | 2 | H | 1 | -3-indolyl |
| **XI** | $HOOC-CH_2-$ | 2 | H | 1 | 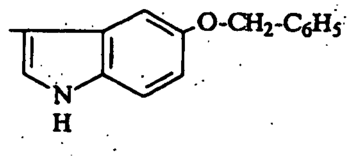 (-3-indolyl-$OCH_2C_6H_5$) |
| **XII** | $HOOC-CH_2-$ | 2 | H | 1 | 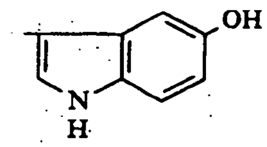 (-3-mdolyl-OH) |
| **XIII** | $HOOC-CH_2-$ | 2 | H | 1 | 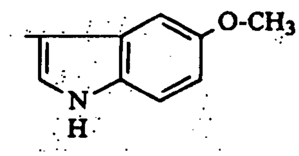 (-3-indolyl-OMe |
| **XIV** | $HOOC-CH_2-$ | 2 | H | 1 | 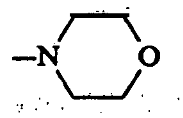 |
| **XV** | $HOOC-CH_2-$ | 2 | H | 1 | 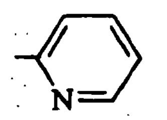 |
| **XVI** | $HOOC-CH_2-$ | 2 | H | 1 | 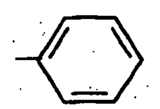 |
| **XVIII** | $HOOC-CH_2-$ | 2 | H | 1 | 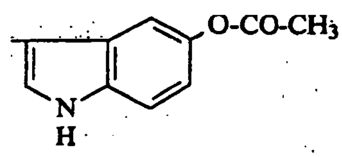 (-3-indolyl-$OCOCH_3$) |
| **XXII** | $HOOC-CH_2-$ | 1 | H | 1 | 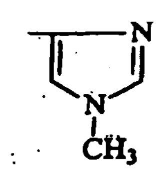 |

(continued)

| | $R_1$ | n | $R_2$ | m | $R_3$ |
|---|---|---|---|---|---|
| **XXIII** | HOOC-$CH_2$- | 2 | -$COOCH_3$ | 1 | 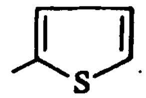 |
| **XXIV** | kOOC-$CH_2$- | 2. | H | 1 | 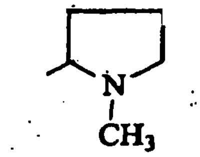 |
| **XXV** | HOOC-$CH_2$- | 2 | H | 1 | 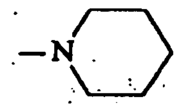 |
| **XXVI** | HOOC-$CH_2$ | 2 | H | 1 | 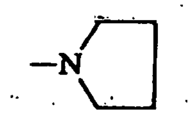 |
| **XXVII** | HOOC-$CH_2$- | 1 | H | 2 | 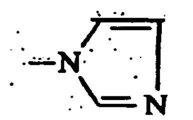 |
| **XXVIII** | $C_2H_5OCO$-$CH_2$- | 0 | H | 1 | -4-imidazolyl |
| **XXLX** | 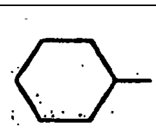 | 6 | H | 1 | -4-imidazol |
| **XXXIII** | -$CH_2$-4-imidazolyl | 0 | H | 4 | CH3-$CH_2$ |
| **XXXIV** | $CH_2$3-indolyl | 0 | H | 2 | -($NH_2$)CH-COOH |
| **XXXV** | $CH_2$3-indolyl | 1 | H | 1 | -$CH_2$-NH2 |
| **XXXVII** | $CH_2$3-indolyl $CH_2$-NHCO-$CH_3$. | 2 | H | 1 | 4-imidazol |
| **XXXVIII** | $CH_2$(COOH)- | 1 | H | 1 | -4-imidazol |
| **XXXIX** | NH2-CH($CH_2$COOH)- | 0 | H | 1 | -4-imidazol |
| **XL**[a,b,c] | 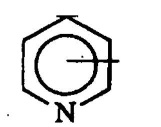 | 0 | H | 1 | -4-imidazolyl |
| **XLI** | 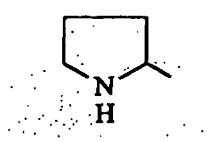 | 0 | H | 1 | 3-indolyl |
| **XLII** | 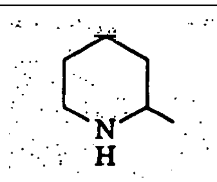 | 0 | H | 1 | -4-imidazolyl |

(continued)

| | $R_1$ | n | $R_2$ | m | $R_3$ |
|---|---|---|---|---|---|
| XLIII | 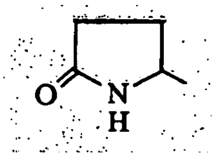 | 0 | H | 1 | -3-indolyl |
| Q | 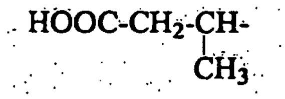 | 1 | H | 1 | -4-imidazolyl |

**8.** A compound according to claim 7, which is glutarylhistamine of formula

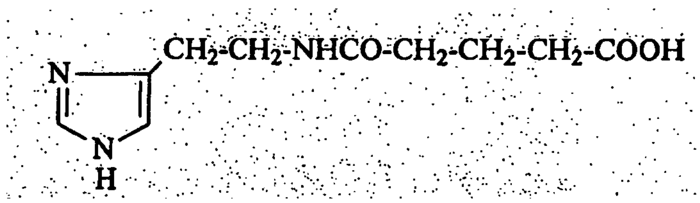

of a pharmaceutically acceptable salt thereof.

**9.** A process for producing a compound according to claim 7, or a pharmaceutically acceptable salt thereof, which process comprises the amino group acylation of an amino compound of general formula $NH_2$-CH($R_2$)-$(CH_2)_m$-$R_3$ wherein $R_2$,$R_3$ and m are as defined in claim 7, with as acylating agent an activated carboxy compound of general formula $R_1$-$(CH_2)_n$-COX,wherein X is an activating group.

**10.** A process according to claim 9 wherein when $R_1$ is HOOC-$CH_2$-, n is 0-3; or $R_1$ = HOOC-$CH_2$-($CH_3$)C(Rv)-, n =1 and Rv = H or $CH_3$; and $R_2$ is H or -$COOCH_3$, the activated carboxy compound is an anhydride of a dicarbonic acid and the process is carried out in an organic solvent.

**11.** A process according to claim 9 wherein, when $R_1$=-$CH_2$-$NH_2$, n = 1-2; $R_2$ = -H,

$R_3$= 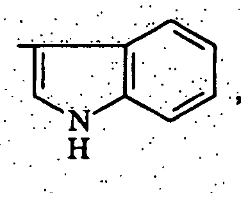 ,

m=1 or
when $R_1$= HOOC-CH($NH_2$)-, n=1-2; and
$R_2$=-$COOCH_3$ or H; and

$R_3$= 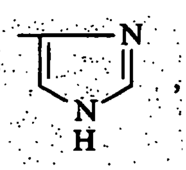 ,

m=1

the acylating agent is a pentafluorophenyl ester of N-benyloxycarbonyl-γ-aminobutyric acid or N-benzyloxycarbonyl-β-alanine or an α-benzl ester of N-tert-butyl oxycarbonyl-L-glutamic or aspartic acid and wherein the process is carried out an organic solvent with subsequent cleavage of the N-benzyloxycarbonyl protecting group and α-benzyl ester or pentafluoro-phenyl ester from the protected derivative of a dipeptide using catalytic hydrogenolysis, with subsequent cleaving of the N-tert-butyl oxycarbonyl group in an anhydrous acidic medium.

**12.** A process according to claim 9 wherein $R_1$=$CH_3$CONH-CH(COOH)-and n=1-2; or$R_1$=$CH_3$CONH-CH([CH2]$_k$COOH)-,n=0,K 1-2; or $R_1$ =.$CH_3$CONH= $CH_2$-,n=2-4 and $R_2$ = H,

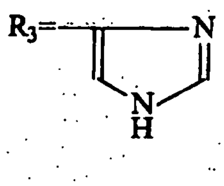
or
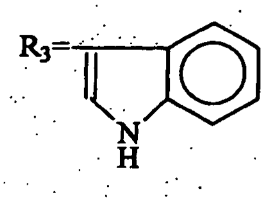

and m=1,
the acylating agent is p-nitrophenyl acetate.

**13.** A compound according to claim 7 or 8, for use in a method of treatment of the human or animal body by therapy.

**14.** A pharmaceutical composition comprising a pharmaceuticaly acceptable carrier or diluent and, as an active compound, a compound according to claim 7 or 8.

**15.** A compound according to claim 7 or 8, for use as an antibacterial antitumour, antiviral or antimetastatic agent, or to alleviate stress conditions.

**16.** A compound according to claim 7 or 8 as an antihypoxic, a lipid regulator, or a hypoglycemic agent, for controlling symptoms or preventing asthma or pulmonary emphysemia, for controlling symptoms of cutaneous lesions, psoriasis or eczema, for preventing abortion, dysfunctional uterine bleeding dr amenorrhea, for controlling the symptoms of ischemic disease or obesity, for use as a hepatoprotective agent, or for controlling or preventing the symptoms of gerontological disease.

**17.** A compound according to claim 16, which is for use in controlling hepatic lesions including toxic lesion hepatitis , cirrhosis or alcohol abuse.

**Patentansprüche**

**1.** Verbindung darstellend Cyclohexylcarbonylhistamin oder N-(Imidazolyl-4-Acetyl)-Heptylamin oder Peptid der allgemeinen Formel (I):

$$R_1\text{-}(CH_2)n\text{-}CONH\text{-}\underset{\displaystyle R_2}{\underset{|}{CH}}\text{-}(CH_2)m\text{-}R_3 \qquad \textbf{(I)}$$

oder der Struktur

| $R^1$ | n | $R_2$ | m | $R^3$ |
|---|---|---|---|---|
| 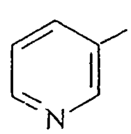 | 0 | H | 1 | 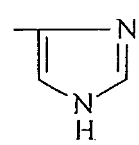 |

(fortgesetzt)

| $R^1$ | n | $R_2$ | m | $R^3$ |
|---|---|---|---|---|
| | | | | (-4-Imidazolyl) |
| 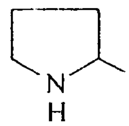 | 0 | H | 1 | 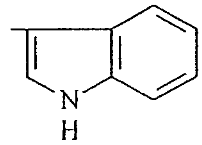 (-3-Indolyl) |
| 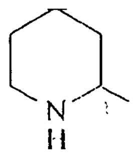 | 0 | H | 1 | -4-Imidazolyl |
| 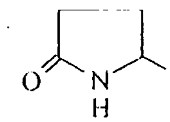 | 0 | H | 1 | -3-Indolyl |

oder worin $R_1$ fuer einen $C_1$-$C_3$-Kohlenwasserstoffrest steht, welcher durch eine Amino- oder Carboxylgruppe substituiert ist, wobei die Carboxylgruppe gegebenenfalls verestert ist, und die Aminogruppe durch einen Acylsubstituenten oder Kohlensaeurester gegebenenfalls substituiert ist; oder
$R_1$ fuer einen $C_1$-$C_3$-Kohlenwasserstoffrest steht, welcher gleichzeitig durch eine Amino- und Carboxylgruppe substituiert ist, wobei die Carboxylgruppe gegebenenfalls verestert ist, und die Aminogruppe durch einen Acylsubstituenten oder Kohlensaeurester gegebenenfalls substituiert ist; oder
$R_1$ fuer einen $C_1$-$C_3$-Kohlenwasserstoffrest steht, welcher durch einen Indolrest oder eine 5-6-gliedrige ungesaettigte heterozyklische Gruppe substituiert ist, wobei der Kohlenwasserstoffrest gegebenenfalls gleichzeitig eine Aminogruppe enthaelt, welche unsubstituiert ist oder durch einen Azylsubstituenten oder Kohlensaeurester substituiert ist;
$R_2$ fuer einen Wasserstoffatom oder eine Carboxylgruppe steht, welche gegebenenfalls verestert ist;
$R_3$ fuer eine Indolgruppe steht, welche unsubstituiert oder durch Methyl oder eine Hydroxylgruppe substituiert ist, wobei die Hydroxylgruppe unsubstituiert oder durch Azyl, Alkyl oder Aralkyl substituiert ist;
$R_3$ fuer eine 5 bis 6-gliedrige gesaettigte oder ungesaettigte heterozyklische Gruppe enthaltend Sauerstoff, Schwefel und/oder 1 bis 3 Stickstoffatome oder eine 5 bis 6-gliedrige ungesaettigte carbozyklische Gruppe oder deren Methylderivat steht; oder
$R_3$ fuer einen $C_1$-$C_3$-Kohlenwasserstoffrest steht, welcher durch eine Amino- oder Carboxylgruppe substituiert ist, wobei die Carboxylgruppe gegebenenfalls verestert ist, oder
$R_3$ fuer einen $C_1$-$C_3$-Kohlenwasserstoffrest steht, welcher gleichzeitig durch eine Amino- oder Carboxylgruppe substituiert ist;
n=0 bis 4, m=1 bis 5 oder deren pharmazeutisch vertraegliches Salz fuer die Verwendung als ein bakterienfeindliches, antivirales, Antitumor- oder Antimetastaseagens oder fuer die Ueberwindung von Stresszustaenden.

**2.** Verbindung gemaess Anspruch 1 fuer die Verwendung in Kombination mit einem cytostatischen Agens und Radiotherapie.

**3.** Verbindung, welche ein Peptid der allgemainen Formel (I) nach Anspruch 1 darstellt, worin $R_1$ fuer $NH_2$-$CH_2$- steht, n=1-4; oder
$R_1$ fuer HOOC-$CH_2$- steht, n=0-3; oder
$R_1$ fuer Rz-OCO-$CH_2$- steht, n=0-3; Rz fuer -H oder einen $C_1$-$C_3$-Kohlenwasserstoffrest steht; oder
$R_1$ fuer HOOC-$CH_2$-($CH_3$)C(Rv)- steht, Rv fuer H, -$CH_3$ steht; oder
$R_1$ fuer $C_6H_5CH_2OCO$-NH-$CH_2$- steht, n=1-2; oder
$R_1$ fuer Rx-CONH-$CH_2$- steht, n=1-4, Rx fuer einen $C_1$-$C_3$-Kohlenwasserstoffi-est steht; oder
$R_1$ fuer $CH_3CONH$-CH(COOH)- steht, n=1-2; oder

$R_1$ fuer $CH_3CONH-CH([CH_2]_kCOOH)$- steht, n=0, k=1-2; oder
$R_1$ fuer $NH_2-CH([CH_2]_kCOOH)$- steht, n=0, k=1-2 ; oder
$R_1$ fuer $HOOC-CH(NH_2)$- steht, n=0; oder
$R_1$ fuer $HOOC-CH(NH_2)$- steht, n=1-2; oder
$R_1$ fuer $CH_3OOC-CH(NH_2)$- steht, n=1-2; oder
$R_1$ fuer $(CH_3)_3C-OCONH-CH(COOCH_2-C_6H_5)$- steht, n=1-2; oder
$R_1$ fuer-$CH_2$-4-Imidazolyl, -$CH_2$-3-Indolyl steht, n=0-1; oder
$R_1$ fuer Rb-$CH_2$-CH(NHRy)- steht, Rb fuer -4-Imidazolyl, -3-Indolyl, $R_y$ fuer tret.Butyloxycarbonyl, Benzyloxycarbonyl, -H- steht, n=0 ;
$R_2$ fuer -H, -COOH, -COORz steht, worin Rz fuer H oder einen $C_1$-$C_3$-Kohlenwasserstoffrest steht; und

$R_3$= 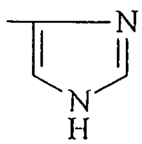 , 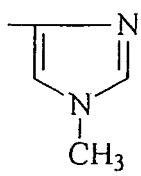 , 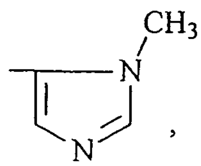 , 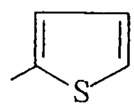

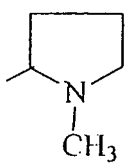 , 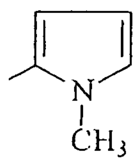 , 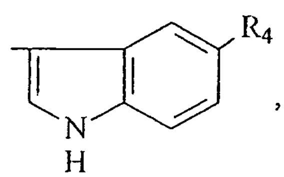 , 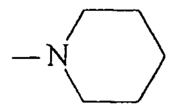

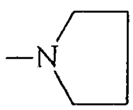 , 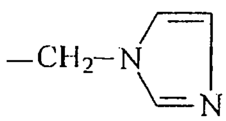 , 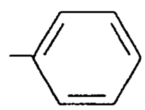 , 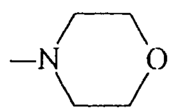 , 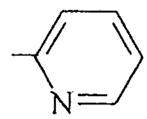

m=1; oder
$R_3$ fuer-$CH_2$-$NH_2$ steht, m=1-2; oder
$R_3$ fuer-$CH_2$-COOH steht, m=1-2; oder
$R_3$ fuer -$CH(NH_2)$-COOH steht, m=1-2, worin $R_4$= -H, -OH, -$OCH_3$, -$OCOCH_3$ oder -$OCH_2C_6H_5$ fuer die Verwendung, wie im Anspruch 1 bestimmt ist.

**4.** Verbindung, welche ein Peptid der allgemeinen Formel (I) gemaess Anspruch 1 darstellt und aus den folgenden Verbindungen ausgewaehlt ist:

| **N° Comp.** | **$R_1$** | **n** | **$R_2$** | **m** | **$R_3$** |
|---|---|---|---|---|---|
| **II** | HOOC-$CH_2$- | 1 | H | 1 | 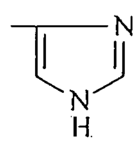 (-4-Imidazolyl) |
| **III** | HOOC-$CH_2$- | 2 | H | 1 | -4-Imidazolyl |
| **IV** | HOOC-$CH_2$- | 3 | H | 1 | -4-Imidazolyl |

(fortgesetzt)

| N° Comp. | $R_1$ | n | $R_2$ | m | $R_3$ |
|---|---|---|---|---|---|
| **V** | $CH_3CO$-NH-CH(COOH) | 2 | H | 1 | -4-Imidazolyl |
| **VI**[a] | $(CH_3)_3$-COCONH-CH-$COOCH_2C_6H_5$ | 2 | $-COOCH_3$ | 1 | -4-Imidazolyl |
| **VI** | 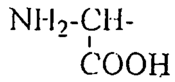 | 2 | $-COOCH_3$ | 1 | -4-Imidazolyl |
| **VII** | HOOC-$CH_2$- | 2 | $-COOCH_3$ | 1 | -4-Imidazolyl |
| **VIII** | $NH_2$-$CH_2$-$CH_2$- | 1 | $-COOCH_3$ | 1 | -4-Imidazolyl |
| **IX** | HOOC-CH($NH_2$)- | 2 | $-COOCH_3$ | 1 | -4-Imidazolyl |
| **X**[a] | $C_6H_5CH_2OCONH$-$CH_2$- | 2 | H | 1 | 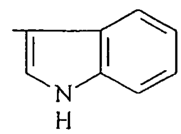 (-3-Indolyl) |
| **X** | $NH_2$-$CH_2$- | 2 | H | 1 | -3-Indolyl |
| **XI** | HOOC-$CH_2$- | 2 | H | 1 | 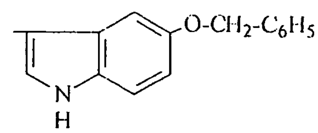 (-3-Indolyl-$OCH_2C_6H_5$ |
| **XII** | HOOC-$CH_2$- | 2 | H | 1 | 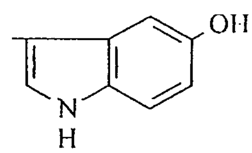 (-3-Indolyl-OH) |
| **XIII** | HOOC-$CH_2$- | 2 | H | 1 | 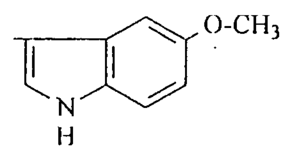 (-3-Indolyl-OMe) |
| **XIV** | HOOC-$CH_2$- | 2 | H | 1 | 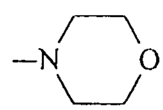 |
| **XV** | HOOC-$CH_2$- | 2 | H | 1 | 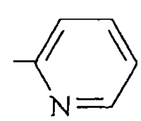 |
| **XVI** | HOOC-$CH_2$- | 2 | H | 1 | 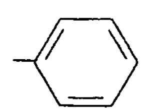 |

(fortgesetzt)

| N° Comp. | $R_1$ | n | $R_2$ | m | $R_3$ |
|---|---|---|---|---|---|
| **XVII** | $HOOC-CH_2-$ | 2 | H | 1 | 3-Indolyl |
| **XVIII** | $HOOC-CH_2-$ | 2 | H | 1 | 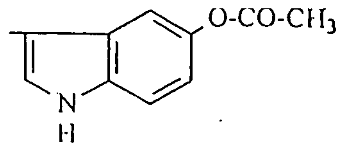 (-3-Indolyl-$OCOCH_3$) |
| **XXII** | $HOOC-CH_2-$ | 1 | H | 1 | 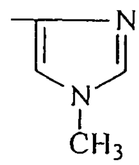 |
| **XXIII** | $HOOC-CH_2-$ | 2 | $-COOCH_3$ | 1 | 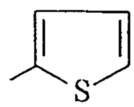 |
| **XXIV** | $HOOC-CH_2-$ | 2 | H | 1 | 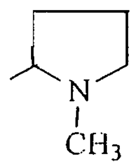 |
| **XXV** | $HOOC-CH_2-$ | 2 | H | 1 | 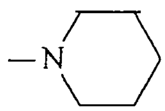 |
| **XXVI** | $HOOC-CH_2-$ | 2 | H | 1 | 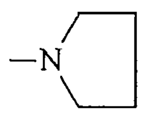 |
| **XXVII** | $HOOC-CH_2-$ | 1 | H | 2 | 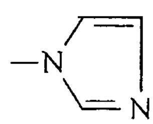 |
| **XXVIII** | $C_2H_5OCO-CH_2-$ | 0 | H | 1 | -4-Imidazolyl |
| **XXIX** | 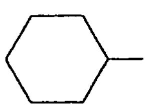 | 0 | H | 1 | -4-Imidazolyl |
| **XXX** | 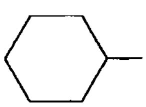 | 0 | H | 1 | -3-Indolyl |
| | | | | | |
| **XXXIII** | $-CH_2$-4-Imidazolyl | 0 | H | 4 | $CH_3-CH_2-$ |

(fortgesetzt)

| N° Comp. | $R_1$ | n | $R_2$ | m | $R_3$ |
|---|---|---|---|---|---|
| **XXXIV** | $-CH_2$-3-Indolyl | 0 | H | 2 | $-(NH_2)CH-COOH$ |
| **XXXV** | $-CH_2$-3-Indolyl | 1 | H | 1 | $-CH_2-NH_2$ |
| **XXXVI** | $-CH(NH_2)-CH_2$-4-Imidazolyl | 0 | H | 1 | -COOH |
| **XXXVII** | $-CH_2-NHCO-CH_3$ | 2 | H | 1 | -4-Imidazolyl |
| **XXXVIII** | $NH_2-CH(COOH)-$ | 1 | H | 1 | -4-Imidazolyl |
| **XXXIX** | $NH_2-CH(CH_2COOH)-$ | 0 | H | 1 | -4-Imidazolyl |
| **XL**[a,b,c] | 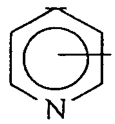 | 0 | H | 1 | -4-Imidazolyl |
| **XLI** | 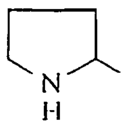 | 0 | H | 1 | 3-Indolyl |
| **XLII** | 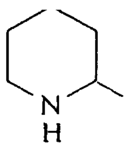 | 0 | H | 1 | -4-Imidazolyl |
| **XLIII** | 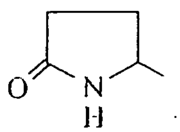 | 0 | H | 1 | -3-Indolyl |
| **Q** | 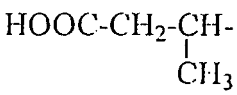 | 1 | H | 1 | -4-Imidazolyl |

fuer die Verwendung, wie im Anspruch 1 bestimmt ist.

**5.** Verbindung gemaess Anspruch 1, welche ein Glutarylhistamin der Struktur

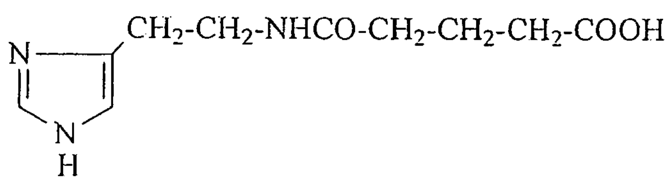

oder deren pharmazeutisch geeignetes Salz darstellt.

**6.** Verbindung gemaess einem der vorhergehenden Ansprueche, worin, wenn die Verbindung fuer eine antivirale Verwendung geeignet ist, diese fuer die Behandlung der Grippe oder HIV-Infektion geeignet ist.

**7.** Verbindung der Struktur (II), (III), (IV), (V), (VI[a]), (VI), (VII), (VIII), (IX), (X[a]), (X), (XI), (XII), (XIII), (XIV), (XV),(XVI),

(XVIII), (XXII), (XXIII), (XXIV), (XXV), (XXVI), (XXVII), (XXVIII), (XXIX), (XXXIII), (XXXIV), (XXXV), (XXXVII), (XXXVIII), (XXXIX), (XL[a,b,c]), (XLI), (XLII), (XLIII) oder (Q) oder deren pharmazeutisch geeignetes Salz

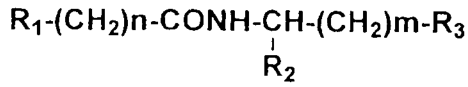

(I)

| N Comp. | $R_1$ | n | $R_2$ | m | $R_3$ |
|---|---|---|---|---|---|
| **II** | $HOOC-CH_2-$ | 1 | H | 1 | 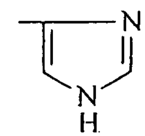 (-4-Imidazolyl) |
| **III** | $HOOC-CH_2-$ | 2 | H | 1 | -4-Imidazolyl |
| **IV** | $HOOC-CH_2-$ | 3 | H | 1 | -4-Imidazolyl |
| **V** | $CH_3CO-NH-CH(COOH)$ | 2 | H | 1 | -4-Imidazolyl |
| **VI[a]** | $(CH_3)_3-COCONH-CH-$ <br> $COOCH_2C_6H_5$ | 2 | $-COOCH_3$ | 1 | -4-Imidazolyl |
| **VI** | $NH_2-CH-$ <br> $COOH$ | 2 | $-COOCH_3$ | 1 | -4-Imidazolyl |
| **VII** | $HOOC-CH_2-$ | 2 | $-COOCH_3$ | 1 | -4-Imidazolyl |
| **VIII** | $NH_2-CH_2-CH_2-$ | 1 | $-COOCH_3$ | 1 | -4-Imidazolyl |
| **IX** | $HOOC-CH(NH_2)-$ | 2 | $-COOCH_3$ | 1 | -4-Imidazolyl |
| **X[a]** | $C_6H_5CH_2OCONH-CH_2-$ | 2 | H | 1 | 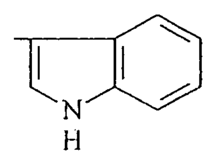 (-3-Indolyl) |
| **X** | $NH_2-CH_2-$ | 2 | H | 1 | -3-Indolyl |

(fortgesetzt)

| N° Comp. | $R_1$ | n | $R_2$ | m | $R_3$ |
|---|---|---|---|---|---|
| **XI** | HOOC-$CH_2$- | 2 | H | 1 | 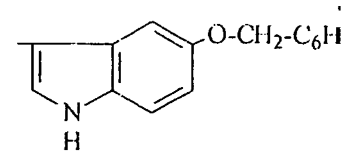 (-3-Indolyl-$OCH_2C_6H_5$) |
| **XII** | HOOC-$CH_2$- | 2 | H | 1 | 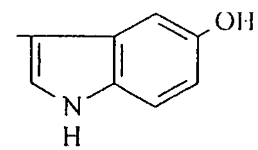 (-3-Indolyl-OH) |
| **XIII** | HOOC-$CH_2$- | 2 | H | 1 | 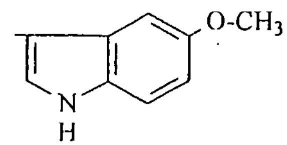 (-3-Indolyl-OMe) |
| **XIV** | HOOC-$CH_2$- | 2 | H | 1 | 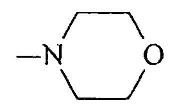 |
| **XV** | HOOC-$CH_2$- | 2 | H | 1 | 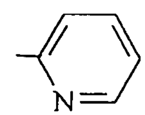 |
| **XVI** | HOOC-$CH_2$- | 2 | H | 1 | 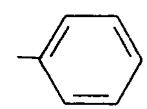 |

(fortgesetzt)

| N° Comp. | $R_1$ | n | $R_2$ | m | $R_3$ |
|---|---|---|---|---|---|
| | | | | | |
| **XVIII** | HOOC-$CH_2$- | 2 | H | 1 | 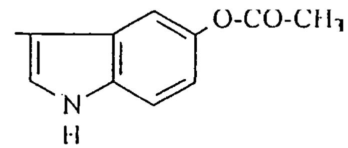 (-3-Indolyl-$OCOCH_3$) |
| **XXII** | HOOC-$CH_2$- | 1 | H | 1 | 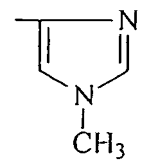 |
| **XXIII** | HOOC-$CH_2$- | 2 | -$COOCH_3$ | 1 | 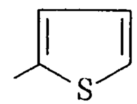 |
| **XXIV** | HOOC-$CH_2$- | 2 | H | 1 | 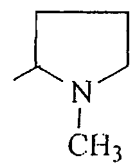 |
| **XXV** | HOOC-$CH_2$- | 2 | H | 1 | 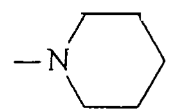 |
| **XXVI** | HOOC-$CH_2$- | 2 | H | 1 | 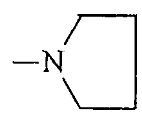 |

(fortgesetzt)

| N° Comp. | $R_1$ | n | $R_2$ | m | $R_3$ |
|---|---|---|---|---|---|
| **XXVII** | HOOC-$CH_2$- | 1 | H | 2 | 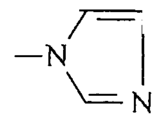 |
| **XXVIII** | $C_2H_5OCO$-$CH_2$- | 0 | H | 1 | -4-Imidazolyl |
| **XXIX** | 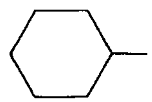 | 0 | H | 1 | -4-Imidazolyl |
| **XXXIII** | -$CH_2$-4-Imidazolyl | 0 | H | 4 | $CH_3$-$CH_2$ |
| **XXXIV** | -$CH_2$-3-Indolyl | 0 | H | 2 | -($NH_2$)CH-COOH |
| **XXXV** | -$CH_2$-3-Indolyl | 1 | H | 1 | -$CH_2$-$NH_2$ |
| **XXXVII** | -$CH_2$-NHCO-$CH_3$ | 2 | H | 1 | -4-Imidazolyl |
| **XXXVIII** | $NH_2$-CH(COOH)- | 1 | H | 1 | -4-Imidazolyl |
| **XXXIX** | $NH_2$-CH($CH_2$COOH)- | 0 | H | 1 | -4-Imidazolyl |
| **XL**[a,b,c] | 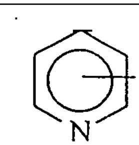 | 0 | H | 1 | -4-Imidazolyl |
| **XLI** | 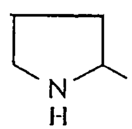 | 0 | H | 1 | 3-Indolyl |
| **XLII** | 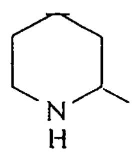 | 0 | H | 1 | -4-Imidazolyl |

(fortgesetzt)

| N Comp. | $R_1$ | n | $R_2$ | m | $R_3$ |
|---|---|---|---|---|---|
| XLIII | 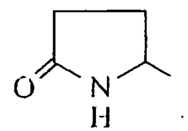 | 0 | H | 1 | -3-Indolyl |
| Q | 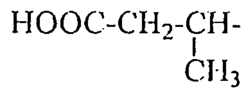 | 1 | H | 1 | -4-Imidazolyl |

**8.** Verbindung gemaess Anspruch 7, welche Glutarylhistamin der Struktur

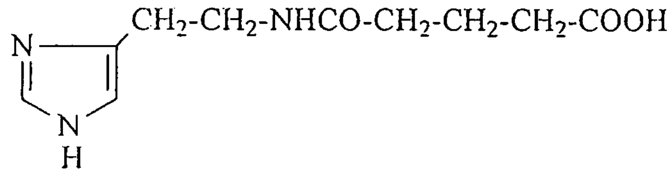

oder deren pharmazeutisch geeignetes Salz darstellt.

**9.** Verfahren zur Herstellung einer Verbindung gemaess Anspruch 7 oder ihres pharmazeutisch geeigneten Salzes umfassend das Azylieren der Aminogruppe der Aminoverbindung der allgemeinen Formel $NH_2$-$CH(R_2)$-$(CH_2)_m$-$R_3$, worin $R_2$, $R_3$ und m die Bedeutungen haben, welche im Anspruch 7 bestimmt sind, durch eine nach der Carboxylgruppe aktivierte Verbindung der allgemeinen Formel $R_1$-$(CH_2)$n-COX, worin die X fuer eine aktivierende Gruppe steht als ein Azylierungsagens.

**10.** Verfahren gemaess Anspruch 9, worin, wenn $R_1$ fuer HOOC-$CH_2$-, n=0-3; oder $R_1$ fuer HOOC-$CH_2$-$(CH_3)C(R_v)$-, n=1 und $R_v$ fuer H oder $CH_3$; und $R_2$ fuer -H oder -$COOCH_3$ steht, als eine aktivierte Carboxyverbindung Dikarbonsaeureanhydride verwendet werden und der Prozess in einem organischen Loesungsmittel durchgefuehrt wird.

**11.** Verfahren zur Herstellung gemaess Anspruch 9, worin, wenn $R_1$ fuer -$CH_2$-$NH_2$ steht, n=1-2, $R_2$=H

$R_3$= 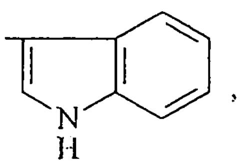 ,

m=1
oder wenn $R_1$ fuer HOOC-CH($NH_2$)- steht, n=1-2, $R_2$ fuer-$COOCH_3$ oder H steht,

$R_3$= 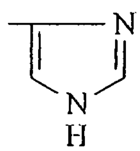 ,

m=1
als Azylierungsagens Pentafluorphenylaester der N-Benzyloxycarbonyl-γ-Aminooelsaeure oder N-Benzyloxycarbonyl-β-Alanin oder α-Benzylaesters der N-tret.-Butyloxycarbonyl-L-Glutamin-oder Asparaginsaeure eingesetzt werden und der Prozess in einem organischen Loesungsmittel mit darauffolgender Abspaltung von geschuetzten Dipeptidderivaten der N-Benzyloxycarbonylschutzgruppe und des α-Benzylaesters oder Pentafluorphenylaesters durch eine katalytische Hydrogenolyse mit darauffolgender Abspaltung der N-tret.-Butyloxycarbonylgruppe in einem wasserfreien sauren Medium durchgefuehrt wird.

**12.** Verfahren gemaess Anspruch 9, worin, wenn $R_1$ fuer $CH_3CONH$-CH(COOH)- steht, n=1-2; oder $R_1$ fuer $CH_3CONH$-CH($[CH_2]_k$C0OH)- steht, n=0, k=1-2; oder $R_1$ fuer $CH_3CONH$-$CH_2$- steht, n=2-4; und $R_2$ fuer H steht,

$R_3=$ 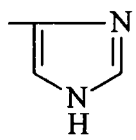 oder $R_3=$ 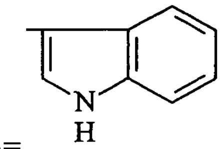 ,

m=1,
als Azylierungsagens n-Nitrophenylazetat eingesetzt wird.

**13.** Verbindungen gemaess Anspruch 7 oder 8 fuer die Verwendung im Verfahren zur Behandlung eines Menschen oder Tieres.

**14.** Pharmazeutische Zusammensetzung enthaltend einen pharmazeutisch annehmbaren Traeger oder ein Verduennungsmittel und als Wirkstoff eine im Anspruch 7 oder 8 bestimmte Verbindung.

**15.** Verbindung gemaess Anspruch 7 oder 8 fuer die Verwendung als ein bakterienfeindliches, Antitumor-, antivirales oder Antimetastaseagens oder fuer die Ueberwindung von Stresszustaenden.

**16.** Verbindung gemaess Anspruch 7 oder 8 fuer die Verwendung als antihypoxisches, lipidenregulierendes oder hypoglykemisches Agens, fuer die Beseitigung von Symptomen und der Vorbeugung von Asthma oder Lungenblaehung, fuer die Beseitigung von Symptomen der Hautbeschaedigung und Hauterkrankungen, Psoriasis oder Ekzem, fuer die Vorbeugung der Fehlgeburtbedrohung, von disfunktionellen Mutterblutungen oder Amenorrhea oder fuer die Beseitigung von Symptomen der ischaemischen Erkrankung oder Verfettung, fuer die Verwendung als Hepatoprotektoragens oder fuer die Beseitigung oder Vorbeugung von Symptomen von gerontologischen Erkrankungen.

**17.** Verbindung gemaess Anspruch 16, welche geeignet ist fuer die Verwendung zur Vorbeugung von Leberbeschaedigungen einschliesslich toxischer Beschaedigungen, Hepatitis, Zirrhose oder der mit dem Alkoholismus verbundenen Leberbeschaedigungen.

## Revendications

**1.** Composé qui représente un cyclohexylcarbonyle histamine soit un N-(imidazolyl-4-acétyl)héptylamine ou un péptide de formule générale (I) :

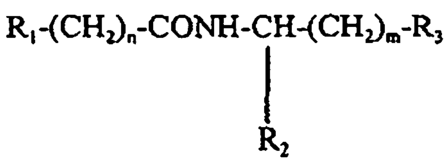 (I),

soit des structures

| $R_1$ | n | $R_2$ | m | $R_3$ |
|---|---|---|---|---|
| 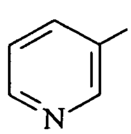 | 0 | H | 1 | 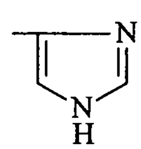 (-4-imidazolyle) |

(suite)

| $R_1$ | n | $R_2$ | m | $R_3$ |
|---|---|---|---|---|
| 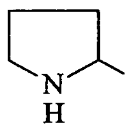 | 0 | H | 1 | 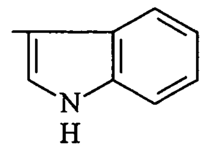 (-3-indolyle) |
| 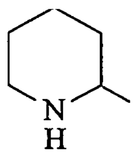 | 0 | H | 1 | -4- imidazolyle |
| 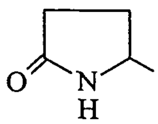 | 0 | H | 1 | -3-indolyle |

soit où **$R_1$** représente un radical hydrocarboné en $C_1$-$C_3$ substitué par un groupe aminé ou carboxylique, le groupe carboxylique étant éventuellement estérifié et le groupe aminé étant éventuellement substitué par un substituant acylique ou un ester d'acide carbonique ; soit **$R_1$** représente un radical hydrocarboné en $C_1$-$C_3$ substitué à la fois par un groupe aminé et un groupe carboxylique, le groupe carboxylique étant éventuellement estérifié et le groupe aminé étant éventuellement substitué par un substituant acylique ou un ester d'acide carbonique ; soit
$R_1$ représente un radical hydrocarboné en $C_1$-$C_3$ substitué par un reste indolylique ou un groupe hétérocyclique insaturé comprenant de 5 à 6 chaînons, le radical hydrocarboné comprenant éventuellement à la fois un groupe aminé non-substitué ou substitué par un substituant acylique ou un ester d'acide carbonique ;
$R_2$ représente un atome d'hydrogène ou un groupe carboxylique éventuellement estérifié ;
$R_3$ représente un groupe indolique non-substitué ou substitué par un méthyle ou un groupe hydroxyle, le groupe hydroxyle étant non-substitué ou substitué par un acyle, un alkyle ou un aralkyle ;
$R_3$ représente un groupe hétérocyclique saturé ou insaturé comprenant de 5 à 6 chaînons, comportant l'oxygène, le soufre et/ou de 1 à 3 atomes d'azote, ou bien un groupe carbocyclique insaturé comprenant de 5 à 6 chaînons ou leur dérivé méthyle ; soit
$R_3$ représente un radical hydrocarboné en $C_1$-$C_3$ substitué par un groupe aminé ou carboxylique, le groupe carboxylique étant éventuellement estérifié ; soit
$R_3$ représente un radical hydrocarboné en $C_1$-$C_3$ substitué à la fois par un groupe aminé et un groupe carboxylique ;
n = de 0 à 4, m = de 1 à 5 ou son sel pharmaceutiquement acceptable pour une utilisation à titre d'un agent antibactérien, antiviral, antitumoral ou antimétastatique ou bien pour surmonter des états de stress.

**2.** Composé selon la revendication 1 pour une utilisation en association avec un agent cytostatique et la radiothérapie.

**3.** Composé qui représente un peptide de formule générale (I) selon la revendication 1, où $R_1$ représente $NH_2$-$CH_2$-, n représente 1 à 4 ; soit
$R_1$ représente HOOC-$CH_2$, n représente 0 à 3 ; soit
$R_1$ représente Rz-OCO-$CH_2$, n représente 0 à 3 ; Rz représente -H ou un radical hydrocarboné en $C_1$-$C_3$ ; soit
$R_1$ représente HOOC-$CH_2$-($CH_3$)C(Rv)-, Rv représente H, -$CH_3$ ; soit
$R_1$ représente $C_6H_5CH_2$-OCO-NH-$CH_2$, n représente 1-2 ; soit
$R_1$ représente Rx-CONH-$CH_2$, n représente 1 à 4, Rx représente un radical hydrocarboné en $C_1$-$C_3$ ; soit
$R_1$ représente $CH_3$CONH-CH(COOH)-, n représente 1-2 ; soit
$R_1$ représente $CH_3$CONH-CH($[CH_2]_k$COOH)-, n représente 0, k représente 1-2 ; soit
$R_1$ représente $NH_2$-CH($[CH_2]_k$COOH)-, n représente 0, k représente 1-2 ; soit
$R_1$ représente HOOC-CH($NH_2$)-, n représente 0 ; soit
$R_1$ représente HOOC-CH($NH_2$)-, n représente 1-2 ; soit

$R_1$ représente $CH_3OOC-CH(NH_2)-$, n représente 1-2 ; soit
$R_1$ représente $(CH_3)_3C-OCONH-CH(COOCH_2-C_6H_5)-$, n représente 1-2 ; soit
$R_1$ représente $-CH_2$-4-imidazolyle, $-CH_2$-3-indolyle, n représente 0-1 ; soit
$R_1$ représente Rb-$CH_2$-CH(NHRy)-, Rb représente -4-imidazolyle, -3-indolyle, $R_y$ représente un tretbutyloxycarbonyle, benzyloxycarbonyle, H-, n représente 0 ;
$R_2$ représente -H, -COOH, -COORz, où Rz représente H ou un radical hydrocarboné en $C_1$-$C_3$ ; et

$R_3$= 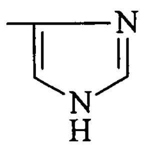 , 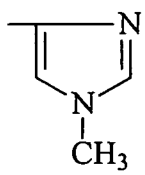 , 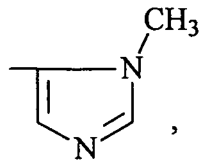 , 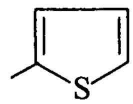

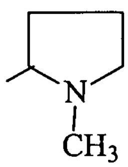 , 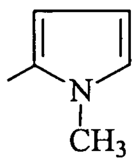 , 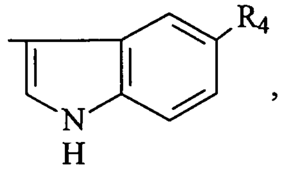 , 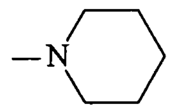

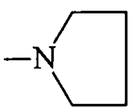 , 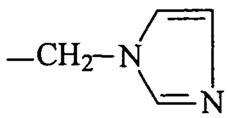 , 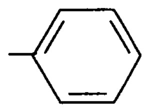 , 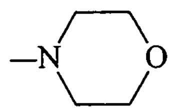 , 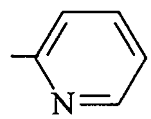

m=1; soit
$R_3$ représente $-CH_2-NH_2$, m = 1-2 ; soit
$R_3$ représente $-CH_2-COOH$, m = 1-2 ; soit
$R_3$ représente $-CH(NH_2)-COOH$, m = 1-2 ; où $R_4$ =-H, -OH, $-OCH_3$, $-OCOCH_3$ ou bien $-OCH_2C_6H_5$ pour une utilisation telle que définié selon la revendication 1.

4. Composé qui représente un péptide de formule générale (I) selon le revendication 1 et qui est choisi parmi des composés suivants :

| N° comp. | $R_1$ | n | $R_2$ | m | $R_3$ |
|---|---|---|---|---|---|
| **II** | HOOC-$CH_2$- | 1 | H | 1 | 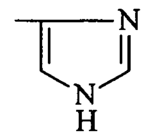<br>(-4-imidazolyle) |
| **III** | HOOC-$CH_2$- | 2 | H | 1 | -4-imidazolyle |
| **IV** | HOOC-$CH_2$- | 3 | H | 1 | -4-imidazolyle |
| **V** | $CH_3$CO-NH-CH(COOH)- | 2 | H | 1 | -4-imidazolyle |
| **VI[a]** | $(CH_3)_3$-COCONH-CH-<br>$COOCH_2C_6H_5$ | 2 | -$COOCH_3$ | 1 | -4-imidazolyle |
| **VI** | $NH_2$-CH-<br>COOH | 2 | -$COOCH_3$ | 1 | -4-imidazolyle |
| **VII** | HOOC-$CH_2$- | 2 | -$COOCH_3$ | 1 | -4-imidazolyle |
| **VIII** | $NH_2$-$CH_2$-$CH_2$- | 1 | -$COOCH_3$ | 1 | -4-imidazolyle |
| **IX** | HOOC-CH($NH_2$)- | 2 | -$COOCH_3$ | 1 | -4-imidazolyle |
| **X[a]** | $C_6H_5CH_2$OCONH-$CH_2$- | 2 | H | 1 | 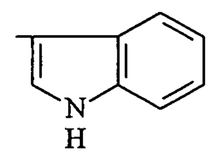<br>(-3-indolyle) |
| **X** | $NH_2$-$CH_2$- | 2 | H | 1 | -3-indolyle |

(suite)

| N° comp. | $R_1$ | n | $R_2$ | m | $R_3$ |
|---|---|---|---|---|---|
| **XI** | HOOC-$CH_2$- | 2 | H | 1 | 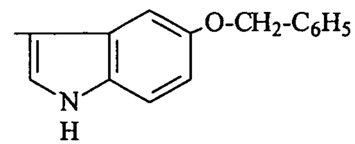 (-3-indolyle-$OCH_2C_6H_5$) |
| **XII** | HOOC-$CH_2$- | 2 | H | 1 | 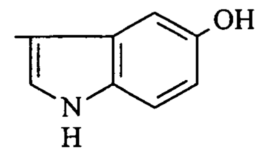 (-3-indolyle-OH) |
| **XIII** | HOOC-$CH_2$- | 2 | H | 1 | 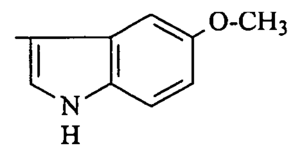 (-3-indolyle-OMe) |
| **XIV** | HOOC-$CH_2$- | 2 | H | 1 | 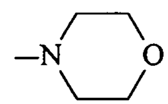 |
| **XV** | HOOC-$CH_2$- | 2 | H | 1 | 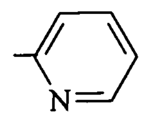 |
| **XVI** | HOOC-$CH_2$- | 2 | H | 1 | 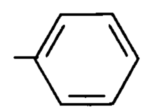 |

(suite)

| N° comp. | $R_1$ | n | $R_2$ | m | $R_3$ |
|---|---|---|---|---|---|
| XVII | HOOC-$CH_2$- | 2 | H | 1 | 3-indolyle |
| XVIII | HOOC-$CH_2$- | 2 | H | 1 | 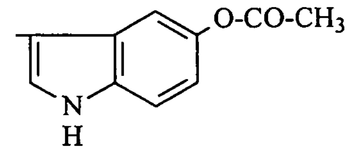 (-3-indolyle-$OCOCH_3$) |
| XXII | HOOC-$CH_2$- | 1 | H | 1 | 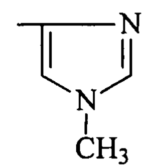 |
| XXIII | HOOC-$CH_2$- | 2 | -$COOCH_3$ | 1 | 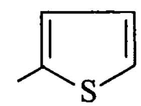 |
| XXIV | HOOC-$CH_2$- | 2 | H | 1 | 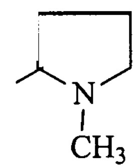 |
| XXV | HOOC-$CH_2$- | 2 | H | 1 | 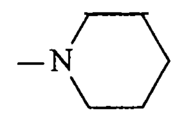 |
| XXVI | HOOC-$CH_2$- | 2 | H | 1 | 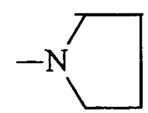 |

(suite)

| N° comp. | $R_1$ | n | $R_2$ | m | $R_3$ |
|---|---|---|---|---|---|
| **XXVII** | $HOOC-CH_2-$ | 1 | H | 2 | 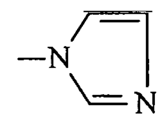 |
| **XXVIII** | $C_2H_5OCO-CH_2-$ | | H | | -4-imidazolyle |
| **XXIX** | 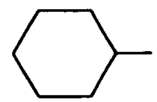 | 0 | H | 1 | -4-imidazolyle |
| **XXX** | 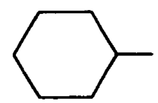 | 0 | H | 1 | -3-indolyle |
| | | | | | |
| **XXXIII** | $-CH_2$-4-imidazolyl | 0 | H | 4 | $CH_3-CH_2-$ |
| **XXXIV** | $-CH_2$-3-indolyl | 0 | H | 2 | $-(NH_2)CH-COOH$ |
| **XXXV** | $-CH_2$-3-indolyl | 1 | H | 1 | $-CH_2-NH_2$ |
| **XXXVI** | $-CH(NH_2)-CH_2$-4-imidazolyl | 0 | H | 1 | -COOH |
| **XXXVII** | $-CH_2-NHCO-CH_3$ | 2 | H | 1 | -4-imidazolyle |
| **XXXVIII** | $NH_2-CH(COOH)-$ | 1 | H | 1 | -4-imidazolyle |
| **XXXIX** | NH2-CH($CH_2$COOH)- | 0 | H | 1 | -4-imidazolyle |
| **XL** [a,b,c] | 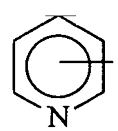 | 0 | H | 1 | -4-imidazolyle |

(suite)

| N° comp. | $R_1$ | n | $R_2$ | m | $R_3$ |
|---|---|---|---|---|---|
| XLI | 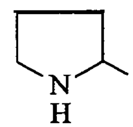 | 0 | H | 1 | 3-indolyle |
| XLII | 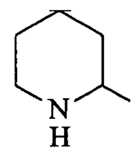 | 0 | H | 1 | -4-imidazolyle |
| XLIII | 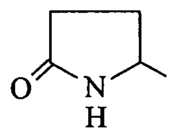 | 0 | H | 1 | -3-indolyle |
| Q | 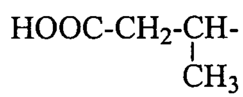 | 1 | H | 1 | -4-imidazolyle |

pour une utilisation telle que définie selon la revendication 1.

**5.** Composé selon la revendication 1 qui représente un glutaryl histamine de structure

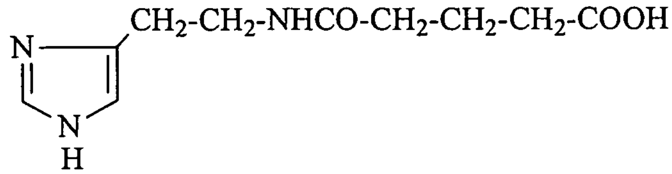

ou son sel pharmaceutiquement acceptable.

**6.** Composé selon l'une quelconque des revendications précédentes où lorsque le composé est destiné pour une utilisation antivirale, il est destiné pour le tratement de la grippe ou de l'infection à virus de l'immunodéficience humaine.

**7.** Composé de structure (II), (III),(IV), (V), ($VI^a$), (VI), (VII), (VIII), (IX), ($X^a$), (X), (XI), (XII), (XIII), (XIV), (XV), (XVI), (XVIII), (XXII), (XXIII), (XXIV), (XXV), (XXVI), (XXVII), (XXVIII), (XXIX), (XXXIII), (XXXIV), (XXXV), (XXXVII), (XXXVIII), (XXXIX), ($XL^{a,b,c}$), (XLI), (XLII), (XLIII) ou bien (Q) ou son sel pharmaceutiquement acceptable

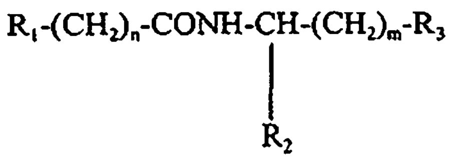 (I),

| N° comp. | $R_1$ | n | $R_2$ | m | $R_3$ |
|---|---|---|---|---|---|
| II | HOOC-$CH_2$- | 1 | H | 1 | 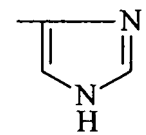<br>(-4-imidazolyle) |
| III | HOOC-$CH_2$- | 2 | H | 1 | -4-imidazolyle |
| | HOOC-$CH_2$- | 3 | H | 1 | -4-imidazolyle |
| V | $CH_3$CO-NH-CH(COOH)- | 2 | H | 1 | -4-imidazolyle |
| VI[a] | $(CH_3)_3$-COCONH-CH-<br>$COOCH_2C_6H_5$ | 2 | -$COOCH_3$ | 1 | -4-imidazolyle |
| VI | $NH_2$-CH-<br>COOH | 2 | -$COOCH_3$ | 1 | -4-imidazolyle |
| VII | HOOC-$CH_2$- | 2 | -$COOCH_3$ | 1 | -4-imidazolyle |
| VIII | $NH_2$-$CH_2$-$CH_2$- | 1 | -$COOCH_3$ | 1 | -4-imidazolyle |
| IX | HOOC-CH($NH_2$)- | 2 | -$COOCH_3$ | 1 | -4-imidazolyle |
| X[a] | $C_6H_5CH_2OCONH$-$CH_2$- | 2 | H | 1 | 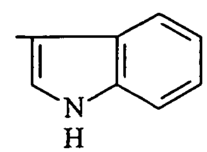<br>(-3-indolyle) |
| X | $NH_2$-$CH_2$- | 2 | H | 1 | -3-indolyle |

(suite)

| N° comp. | R1 | n | R2 | m | R3 |
|---|---|---|---|---|---|
| **XI** | HOOC-$CH_2$- | 2 | H | 1 | 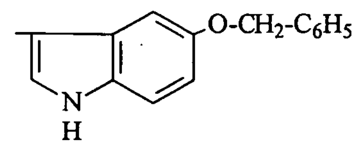 (-3-indolyle-$OCH_2C_6H_5$) |
| **XII** | HOOC-$CH_2$- | 2 | H | 1 | 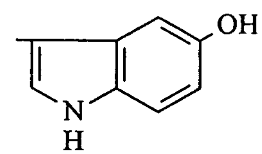 (-3-indolyle-OH) |
| **XIII** | HOOC-$CH_2$- | 2 | H | 1 | 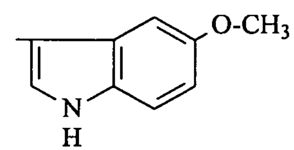 (-3-indolyle-OMe) |
| **XIV** | HOOC-$CH_2$- | 2 | H | 1 | 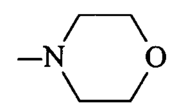 |
| **XV** | HOOC-$CH_2$- | 2 | H | 1 | 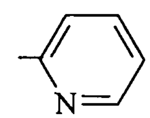 |
| **XVI** | HOOC-$CH_2$- | 2 | H | 1 | 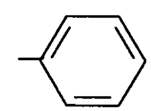 |
| **XVII** | HOOC-$CH_2$- | 2 | H | 1 | 3-indolyle |

(suite)

| N° comp. | $R_1$ | n | $R_2$ | m | $R_3$ |
|---|---|---|---|---|---|
| **XVIII** | HOOC-$CH_2$- | 2 | H | 1 | 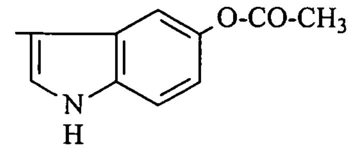 (-3-indolyle-$OCOCH_3$) |
| **XXII** | HOOC-$CH_2$- | 1 | H | 1 | 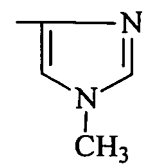 |
| **XXIII** | HOOC-$CH_2$- | 2 | -$COOCH_3$ | 1 | 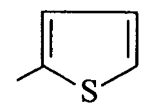 |
| **XXIV** | HOOC-$CH_2$- | 2 | H | 1 | 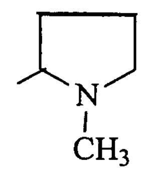 |
| **XXV** | HOOC-$CH_2$- | 2 | H | 1 | 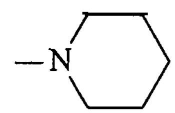 |
| **XXVI** | HOOC-$CH_2$- | 2 | H | 1 | 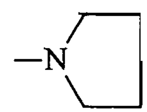 |

(suite)

| N° comp. | $R_1$ | n | $R_2$ | m | $R_3$ |
|---|---|---|---|---|---|
| **XXVII** | $HOOC-CH_2-$ | 1 | H | 2 | 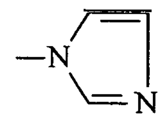 |
| **XXVIII** | $C_2H_5OCO-CH_2-$ | | H | | -4-imidazolyle |
| **XXIX** | 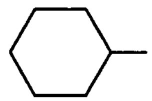 | 0 | H | 1 | -4-imidazolyle |
| **XXX** | 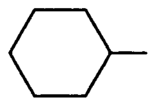 | 0 | H | 1 | -3-indolyle |
| **XXXIII** | $-CH_2$-4-imidazolyl | 0 | H | 4 | $CH_3-CH_2-$ |
| **XXXIV** | $-CH_2$-3-indolyl | 0 | H | 2 | $-(NH_2)CH-COOH$ |
| **XXXV** | $-CH_2$-3-indolyl | 1 | H | 1 | $-CH_2-NH_2$ |
| **XXXVII** | $-CH_2-NHCO-CH_3$ | 2 | H | 1 | -4-imidazolyle |
| **XXXVIII** | $NH_2-CH(COOH)-$ | 1 | H | 1 | -4-imidazolyle |
| **XXXIX** | $NH2-CH(CH_2COOH)-$ | 0 | H | 1 | -4-imidazolyle |
| **XL** [a,b,c] | 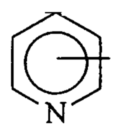 | 0 | H | 1 | -4-imidazolyle |
| **XLI** | 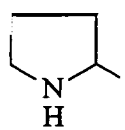 | 0 | H | 1 | 3-indolyle |

(suite)

| N° comp. | $R_1$ | n | $R_2$ | m | $R_3$ |
|---|---|---|---|---|---|
| **XLII** | 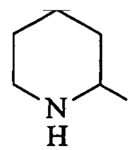 | 0 | H | 1 | -4-imidazolyle |
| **XLIII** | 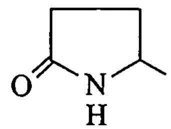 | 0 | H | 1 | -3-indolyle |
| **Q** | 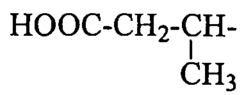 | 1 | H | 1 | -4-imidazolyle |

**8.** Composé selon la revendication 7 qui représente un glutaryl histamine de structure

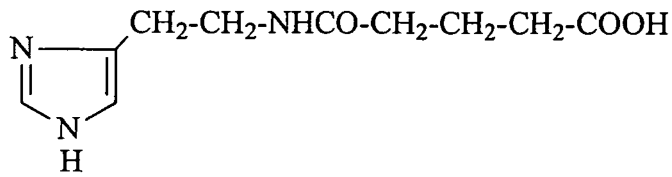

ou son sel pharmaceutiquement acceptable.

**9.** Procédé de préparation d'un composé selon la revendication 7 ou de son sel pharmaceutiquement acceptable comprenant une acylation d'un groupe aminé d'un composé aminé de formule générale $NH_2$-$CH(R_2)$-$(CH_2)_m$-$R_3$, où $R_2$, $R_3$ et m sont tels que définis selon la revendication 7, avec un composé dont le groupe carboxylique est activé de formule $R_1$-$(CH_2)_n$-COX, où X est un groupe activant, à titre d'un agent d'acylation.

**10.** Procédé selon la revendication 9 où lorsque $R_1$=HOOC-$CH_2$-, n=0-3 ; ou bien $R_1$=HOOC-$CH_2$-$(CH_3)C(R_v)$-, n=1, et $R_v$=H ou $CH_3$ ; et $R_2$=-H ou -$COOCH_3$, des anhydrides des acides dicarboxyliques sont utilisés à titre d'un composé carboxy activé et le procédé est mené dans un solvant organique.

**11.** Procédé de préparation selon la revendication 9 dans lequel lorsque $R_1$=-$CH_2$-$NH_2$, n=1-2, $R_2$=H,

$R_3$= 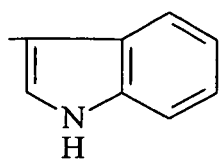 ,

m=1
ou lorsque $R_1$=HOOC-$CH(NH_2)$-, n=1-2, $R_2$=-$COOCH_3$ ou H,

$R_3$= 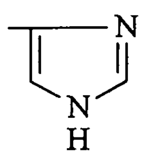 ,

m=1
des esters pentafluorophényliques de l'acide N-benzyloxycarbonyl-γ-aminobutyrique ou bien de N- benzyloxycarbonyl-β-alanine ou bien de l'ester α-benzylique de l'acide N-tret-butyloxycarbonyl-L-glutamique ou aspartique sont utilisés à titre d'un agent d'acylation et le procédé est mené dans un solvant organique suivi de l'élimination des dérivés protégés des dipeptides d'un groupe protecteur N-benzyloxycarbonyle et de l'ester α-benzylique ou de l'ester pentafluorophénylique par une hydrogénolyse catalytique suivie par l'élimination d'un groupe N-tret-butyloxycarbonyle dans un milieu acide anhydre.

**12.** Procédé selon la revendication 9, dans lequel lorsque $R_1$=$CH_3CONH$-CH(COOH)-et n=1-2 ou bien $R_1$= $CH_3CONH$-$CH([CH_2]_kCOOH)$-, n=0, k=1-2 ; ou bien $R_1$= $CH_3CONH$-$CH_2$-, n=2-4 ; et $R_2$=H,

$R_3$= 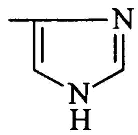 soit $R_3$= 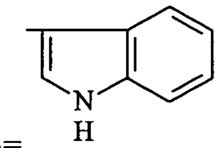 ,

m=1,
un n-nitrophénylacétate est utilisé à titre d'un agent d'acylation.

**13.** Composé selon la revendication 7 ou 8 pour une utilisation dans une méthode de traitement de l'homme ou de l'animal.

**14.** Composition pharmaceutique comprenant un excipient ou un diluant pharmaceutiquement acceptable et un composé tel que défini selon la revendication 7 ou 8 à titre d'un agent actif.

**15.** Composé selon la revendication 7 ou 8 pour une utilisation à titre d'un agent antibactérien, antitumoral, antiviral ou antimétastatique ou bien pour surmonter des états de stress.

**16.** Composé selon la revendication 7 ou 8 pour une utilisation à titre d'un agent antihypoxique, régulateur lipidique ou hypoglycémique, pour supprimer des signes et prévenir l'asthme ou l'emphysème pulmonaire, pour supprimer des signes des affections de la peau et des maladies dermiques, du psoriasis ou de l'eczéma, pour prévenir la menace d'avortement, des hémorragies utérines dysfonctionnelles ou de l'aménorrhée, ou pour supprimer des signes de l'ishémie ou de l'obésité, pour une utilisatrion à titre d'un agent hépatoprotecteur ou pour supprimer ou prévenir des signes des maladies gérontologiques.

**17.** Composé selon la revendication 16 destiné à une utilisation pour supprimer les affections du foie y compris celles toxiques, l'hépatite, la cirrhose ou liées à l'alcoolisme.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2701947 A
- US 4568489 A
- WO 9512581 A
- EP 95107298 A, Koyama, Masayoshi, Takahashi, Mikiko, Yanagawa, Masayoshi
- JP 6115161 A
- US 1488738 B1


### Non-patent literature cited in the description


- **Marone G. ; Columbo N. ; Soppeisa L. et al.** *J. Immunol.,* 1984, vol. 133 (3), 1542-1546
- **Wise J. ; Wojtecka-Lukasik E. ; Maslinski S.** *Agents Actions,* 1986, vol. 18 (1-2), 262-265
- **Duchateau J. ; Bolla K.** *Med. Oncol, and Tumor Pharmacoter.,* 1989, vol. 6 (6), 19-23
- **Muller E. ; Sonnenschein B.** *Naturamed.,* 1989, vol. 4 (4), 34-38
- **Proulx M. ; Dusouich P.** *J. Pharm. Pharmacol.,* 1995, vol. 47 (5), 392-397
- **Barakat M. ; du Souich P.** *J. Pharm. Pharmacol.,* 1996, vol. 48, 906-910
- **Sokolov E.I.** Diabetes mellitus and atherosclerosis. Nauka publishers, 1996, 405
- **Bestervelt L.L. ; Vaz A.D.N. ; Coon M.J.** *Proc. Natl. Acad. Sci. U.S.A.,* 1995, vol. 92 (9), 3764-3768
- **Krzhechkovskaya V.V. ; Meltsev G.Yu. ; Marokko I.N.** *The 4th All-Union Symposium on Medical Enzymology,* 1983, 137
- **Marokko I.N. ; Krzhechkovskaya V.V. ; Malikova N.A. ; Izotov M.V. ; Benediktova S.A. ; Spiridonova S.M.** *Bulletin of Experimental Biology and Medicine,* 1991, 200-202
- **Fornhem C. ; Kumlin M. ; Lundberg J.M. ; Alving K.** *Eur. Resp. J.,* 1995, vol. 8 (7), 1100-1109
- **Fornhem C. ; Lundberg J.M. ; Alving K.** *Eur. Resp. J.,* 1995, vol. 8 (6), 928-937
- **Marokko I.N. ; Krzhechkovskaya V.V. ; Malikova N.A.** P-450 Cytochrome and Modification of Macromolecules. *Proceedings of the All-Union Conference,* 1989, 339
- **Macharadze D.Sh ; Marokko I.N. ; Balabolkin I.I. ; Yukhtina N.V. ; Malikova N.A.** *Pediatry,* 1994, 9-12
- **Fomhem C. ; Lundberg J.M. ; Alving K.** *Eur. Resp. J.,* 1995, vol. 8 (6), 928-937
- **Knickle L.C. ; Bend J.R.** *J. Moll. Pharmacol,* 1994, vol. 45 (6), 1273-1280
- **Quiroga J. ; Prieto J.** *Phamacol. Therap.,* 1993, vol. 58 (1), 67-91
- **Ma Y.H. ; Gebremedich D. ; Schwartzman M.L. et al.** *Circulation Research,* 1993, vol. 72 (1), 126-136
- **Quiroga J. ; Prieto J.** *Pharmacol. Therap.,* 1993, vol. 58 (1), 67-91
- **Caroll M.A. ; Balazy M. ; Margiotta P. ; Falck J.R. ; Mcgiff J.C.** *J. Biol. Chem.,* 1993, vol. 268 (17), 12260-12266
- **Sakairi Y. ; Jacobson H.R. ; Noland T.D. ; Capdevilla J.H. ; Falck J.R. ; Breyer M.D.** *Amer. J. Physiol-Renal. F1. Elect.,* 1995, vol. 37 (5), F931-F939
- **Oyekan A.O.** *Eur. J. Pharmacol.,* 1995, vol. 277 (2-3), 123-132
- **Sakairi Y. ; Jacobson H.R. ; Noland T.D. ; Capdevila J.H. ; Falck J.R. ; Breyer M.D.** *Amer. J. Physiol-Renal. F1. Elect.,* 1995, vol. 37 (5), F931-F939
- Mediators: Release and Functions. **Parker Ch.V.** Immunology. Mir publishers, 1989, vol. 3, 170-247
- **Chakravarty N.K.** The mast cell: Its role in health and disease. 1979, 38-46
- **Fesus L. ; Szucs E. ; Barrett K. et al.** *J. Biol. C. Chem.,* 1995, 13771-13778
- **Imaoka S. ; Sugiyama T. ; Taniguchi N. ; Funae Y.** *Carcinogenesis,* 1993, vol. 14 (1), 117-121
- **Debinski H.S. ; Lee C.S. ; Danks J.A. ; Mackenzie P.I. ; Desmond P.V.** *Gastroenterology,* 1995, vol. 108 (5), 1464-1469
- **Kapil A. ; Koul I.B. ; Suri O.P.** *Phytother. Res.,* 1995, vol. 9 (3), 189-1993
- **Wolfgang GHI ; Robertson DG ; Welty DF ; Metz AL.** *Fund. Appl. Toxicol.,* 1995, vol. 26 (2), 272-281
- **Remaley A.T. ; Schumacher U.K. ; Amouzadeh H.R. ; Brever H.B. ; Hoeg J.M.** *J. Lipid Res.,* 1995, vol. 36 (2), 308-314
- **Stegeman J.J. ; Hahn M.E. ; Weisbrod R. ; Woodin B.R. ; Joy J.S. ; Najibi S. ; Cohen R.A.** *Mol. Pharmacol.,* vol. 47 (2), 296-306

- **Wade A. ; Harred W.** *Feder. Proct.,* 1976, vol. 55, 2475-2479
- **Buters J.T.M. ; Zysset T. ; Reichen J.** *Biochem. Pharmacol.,* 1993, vol. 46 (6), 983-991
- **Reichen J. ; Buters J.T.M. ; Sojcic Z. ; Roos F.J.** *Experentia,* 1992, vol. 48 (5), 482-486
- **Tatony Ya.N.** Obesety. Pathophysiology, diagnostics, therapy. the Polish Medical publishers, 1981, 364
- **Shimojo N. ; Ishizaki T. ; Imaoka S. ; Funae Y. ; Fujii S. ; Okuda K.** *Biochem. Pharmacol.,* 1993, vol. 46 (4), 621-627
- **Sokolov E.I.** Diabetes mellitus and atherosclerosis. Nauka publishers, 1996, 450
- Immunology. Mir publishers, 1989, vol. 3, 202-228
- **Paltsev M.A. ; Ivanov A.A.** Intercellular ineractions. Meditsina publishes, 1995, 224
- **Mayansky A.N. ; Pikuza O.I.** Clinical aspects of phagocytosis. MAGARIF publishers, 1993, 192
- **Henderson W.R ; Jorg A. ; Klebanoff S.J.** *J. Immunol.,* 1982, vol. 128 (6), 2609-2613
- **Burov Yu.V. ; Syrkin A.B. ; Kinzirsky A.S. ; Koroleva A.M.** Chemical-pharmaceutical production. *Review information,* 1992, 42
- **Evstigneeva R.P. ; Zheltukhina G.A. ; Ogrel' S.A. ; Nebol'sin V.E.** Synthesis of pseudopeptides based on biogenic amines. *Reports of the Russian Sciences Academy,* 1995, vol. 345 (4), 493-495
- **Mc Caman M.W. ; Stetzler J. ; Clark B.** Synthesis of γ-Glutamyl dopamine and Other Peptidoamines in the Nervous System of Aplysia californica. *J. Neurochem.,* 1985, vol. 45 (6), 1828-1835
- **Evstigneeva R.P. ; Zheltukhina G.A. ; Ageeva E.A. ; Babizhaev M.A.** Lipoperoxidase activity of carnozine and carcinine. *Reports of the Academy of Sciences of the USSR,* 1993, vol. 333 (1), 104-106
- **Schreder E. ; Lubke K.** Peptides. Mir publishers, 1967, 249
- **Evstigneeva R.P. ; Zheltukhina G.A. ; Ogrel' S.A. ; Nebol'sin V.E.** Synthesis of pseudopeptides based on biogenic amines. *Reports of the Academy of Sciences of the USSR,* 1995, vol. 345 (4), 493-495
- **Konishi H. ; Kakimoto Y.** Formation of γ-Glutamylhistamine from histamine in rat brain. *J.Neurochem.,* 1976, vol. 27, 1461-1463
- **Mc Caman M.W. ; Stetzler J. ; Clark B.** Synthesis of γ-Glutamyldopamine and Other Peptidoamines in the Nervous System of Aplysia californica. *J. Neurochem.,* 1985, vol. 45 (6), 1828-1835
- **Parker K.L. ; Schimmer B.P.** The Role of Nuclear Receptors in Steroid-Hormones Production. *Seminars in Cancer Biology,* 1994, vol. 5 (5), 317-325
- **Emanuel N.M. ; Obukhova L.K. ; Hajdich V.I. ; Murza L.I. ; Bunto T.V.** Aging Inhibition through Activation of the System of Microsomal Oxidases with Phenobarbital. *Reports of the USSR Academy of Sciences,* 1977, vol. 235 (4), 957-960
- **Honn K.V. ; Tang D.G. ; Gao X. ; Butovich I.A. ; Liu B. ; Timar J. ; Hagmann W.** *Cancer and Metastasis Reviews,* 1994, vol. 13 (3-4), 265-396
- **Sernov L.N. ; Gatsura V.V.** A differential indicator method for determinig ischemic and necrotic zone in experimental myocardial infarction in rats. *Bulletin of Experimental Biology and Medicine,* 1989, 534-535
- **Weigle W. ; Cochrane C. ; Dixon F.** Anaphylactogenic properties of soluble antigen-antibody complexes in the guinea pigs and rabbits. *J. Immunology,* 1969, vol. 85, 469-477
- **Gubler E.V.** Computer methods of analysis and recognition of pathological processes. Nauka publishers, 1978, 365
- **Andersson P.** Antigen induced bronchial anaphylaxis in actively sensitized guinea pig. *Allergy,* 1980, vol. 35, 65-71
- **Yu-Hong L. ; Barnes P. ; Rogers D.** Inhibition of neurogenic plasma exudation and bronchoconstriction by a K+ channel activator, BRL 38227, in guinea pig airways in vivo. *Europ. J. Pharmacol.,* vol. 239, 257-259
- **Tarayre J.P. ; Barbara M. ; Aliaga M.** Tisne-Versilles, Comparative actions of immunosuppressants, glucocorticoids and non-steroidal antiinflammatory drugs on various models of delayed hypersensitivity and on a non-immune inflammation in mice. *Arzneim.-Forsch. Drug Res.,* 1990, vol. 40, 1125-1131
- **Ezamuzie Ch. ; Umezurike C.C.** Effect of histamine H2-receptor antagonists on acute inflammatory of the rat paw oedema. *J. Pharmacol.,* 1989, vol. 41, 261-265
- **Winter, De Rosa M. ; Giroud J.P. ; Willoughby D.A.** Studies of the mediators of the acute inflammatory reponse induced in rats in different sites by carrageenan , and turpentine. *J. Pharmacol.,* 1971, vol. 104, 15-29
- **Makarova O.V. ; Kovaleva V.L. ; Sladkopevtsev A.S. ; Mikhailova L.P. ; Noseikina E.M.** *Experimental model of non-infectious pulmonary granulomatosis,* 1996, 76-79
- **Vengerovsky A.I. ; Chuchalin V.S. ; Pauls O.V. ; Saratikov A.S.** Effect of hepatoprotectors on lipid metabolism in hepatitis induced by CCl. *Bulletin of Experimental Biology,* 1987, 430-432
- **Volchegorsky I.A. ; Nalimov A.G. ; Yarovinsky B.G. ; Lifshits R.I.** Comparison of different approaches to determining lipid peroxidation products in heptane-isopropanolic blood extracts, Voprosy Meditsinskoy Chimii. *Problems of Medical Chemistry,* 1989, 127-131

- **Korobeinikova E.N.** Modification of lipid peroxidation products in the reaction with thiobarbituric acid. *Laboratornoye delo,* 1989, 8-10
- The method of determining malonic dialdehyde using thiobarbituric acid. **Stalnaya I.D. ; Garishvili T.G.** Modern methods in biochemistry. Meditsina publishers, 1977, 66-69
- **Cates M.** Technology of Lipidology. Mir publishers, 1975, 74-76
- **Omura T. ; Scato R.** The monooxide binding pigment of liver microsomes., 11. Solubilization, purification and properties. *J. Chem.,* 1964, vol. 239, 2379-2385
- **Ahokas J. ; Pelkonen O. ; Karkin N.** Characterisation of BP-hydroxylase of Trout-liver. *Cancer Res.,* 1977, vol. 37, 3737-3743
- **Hartree E.** Determination of protein: a modification of the Lowry method, that gives a linear photometric responses. *Ann. Biochem.,* 1972, vol. 48, 422-427
- **Guschin LS. ; Voitenko V.G. ; Sviridov B.D. et al.** A polyfunctional molecule produced by the conjugation of synthetic polyionimmunostimulant with specific antigen and an inhibitor of mast cell activation. Effects on histamine release. *Agents and Actions,* 1989, vol. 27, 75-78
- **Fredholm B.B. ; Gyschin I.S. ; Elwin K. et al.** Cyclic AMP-independent inhibition by papaverine of histamine release induced by compound 48/80. *Biochem. Pharmacol.,* 1976, vol. 25, 1583-1588
- **Short P.A. ; Burkhalter A. ; Cohn V.N.** A method for fluorimetric assay of histamine in tissues. *J. Pharmacol. Exper. Ther.,* 1959, vol. 127, 182-186
- **Halliwell B.** Superoxide-dependent formation of hydroxyl radicals in the presence of iron salts. *FEBS Lett.,* 1978, vol. 96, 238-241
- **Afanas'ev I. ; Suslova T. ; Cheremisina Z. et al.** Study of antioxidant properties of metal aspartates. *Analyst,* 1995, vol. 120, 859-862
- Laboratory examination methods in the clinic. Meditsina publishers, 1969, 302
- Biochemical examination in the clinic. Meditsina publishers, 1969, 300-302
- **Titov V.N. ; Brener E.D. ; Khaltaiev N.G. ; Zadoya A.A. ; Tvorogova M.G.** A method and diagnostic significance of cholesterol level study in $\alpha$-lipoproteins. *Laboratornoye delo,* 1979, 36-41
- **Friedwald W.T. ; Levy R.I. ; Fredrickson D.S.** Estimation of the concentration of low-density lipoprotein cholesterol in plasma, without use of preparative ultracentrifuge. *Clin. Chem.,* 1972, vol. 18, 499-502
- **Rodionova L.P.** Modification of blood serum triglyceride level determination method. *Laboratornoye delo,* 1980, 297-299
- **Korobeinikova E.N.** Modification of lipid peroxydation product determination in reaction with thiobarbituric acid. *Laboratornoye delo,* 1989, 8-10
- Malonic dialdehyde determination method using thiobarbituric acid. **Stalnaya I.D. ; Garishvili T.G.** Modern methods in biochemistry. Meditsina publishers, 1977, 66-69
- **Arichi H. ; Kumura H.O.** Effects of stibens compounds of roots of polygonum cuspidatium on the lipid metabolism. *Chem Pharm. Bull.,* 1982, vol. 30 (5), 1766-1767
- **Titov V.N. ; Brener E.D. ; Khaltaiev N.G. ; Zadoya A.A. ; Tvorogova M.G.** A method and diagnostic significance of cholesterol level study in $\alpha$-lipoproteins. *Laboratomoye delo,* 1979, 36-41
- **Friedwald W.T. ; Levy K.J. ; Leus R.** Fat transport in lipoproteins: an integrated approach to mechanism and disorders. *New Engl. J. Med.,* 1967, vol. 276, 32
- **Klimov A.N. ; Nikulcheva N.G.** Lipoproteins, dylipoproteinemias and atherosclerosis. Meditsina publishers, 1984, 165
- **Friedwald W.T. ; Levy R.I. ; Fredrickson D.S.** Estimation of concentration of low-density lipoprotein cholesterol in plasma, without use of preparative ultracentrifuge. *Clin. Chem.,* 1972, vol. 18, 499-502
- **Lazareva D.L. ; Alekhin E.K.** *Immunity stimulants,* 1985, 286
- **Klimov V.V. ; Kolovkina T.V.** Nitroblue tetrasolium reduction test stimulated with pyrogenal. *Laborotornoye delo,* 1982, 624-625
- **Sukhikh T.G. ; Nossik N.N. ; Parshina O.V. ; Van'ko L.V. ; Meerson F.Z. ; Yershov F.I.** Relationship between the natural cellular cytotoxicity system and the interferon system in immobilization stress. *Bulletin of Experimental Biology,* 1984, 593-595
- **Nossik N.N. ; Bopegamage S.A. ; Yershov F.I.** Properties of interferons produced by different cell populations. *Acta microbiologica Hungarica,* 1988, vol. 35 (4), 397-403
- **Nossik N.N ; Bopegamage S.A. ; Yershov F.I.** Properties of interferons produced by different cell populations. *Acta microbiologica Hungarica,* 1988, vol. 35 (4), 397-403
- **Kiseleva E.P. ; Tsveibakh A.S. ; Goldman E.I. ; Pigareva N.V.** Application of a micromethod to study blasttransformation of lymphocytes in humans and animals. *Immunology,* 1985, 76-78